# EUROPEAN PATENT SPECIFICATION

(11) **EP 3 528 830 B1**
(45) Date of publication and mention of the grant of the patent: **05.10.2022**
(21) Application number: 17791037.9
(22) Date of filing: 20.10.2017
(51) Int. Cl.: A61K 38/12, A61K 47/36, A61P 31/00

(54) **POLYMYXIN-ALGINATE OLIGOMER CONJUGATES**
POLYMYXIN-ALGINAT-OLIGOMER-KONJUGATE
CONJUGUÉS OLIGOMÈRE D'ALGINATE-POLYMYXINE

(30) Priority: 21.10.2016 GB 201617860; 13.09.2017 GB 201714710
(43) Date of publication of application: 28.08.2019
(73) Proprietor: Algipharma AS, 1337 Sandvika (NO)
(72) Inventor: FERGUSON, Elaine, Cardiff South Wales CF14 4XY (GB); THOMAS, David William, Cardiff South Wales CF14 4XY (GB); DESSEN, Arne, 3440 Røyken (NO); RYE, Philip, 1359 Eiksmarka (NO)
(74) Representative: Dehns
(86) International application number: PCT/EP2017/076927
(87) International publication number: WO 2018/073449

(56) References cited:
- EP-A1- 0 428 486
- EP-A1- 0 428 486
- GB-A- 976 301
- GB-A- 2 270 920
- US-A- 5 177 059
- US-A- 5 177 059
- US-A- 6 011 008
- SEVERINO ET AL.: "Sodium alginate-cross-linked polymyxin B sulphate- loaded solid lipid nanoparticles: Antibiotic resistance tests and HaCat and NIH/3T3 cell viability studies", COLLOIDS AND SURFACES B: BIOINTERFACES, vol. 129, 2015, pages 191-197, XP29158163,
- SEVERINO PATRÍCIA ET AL: "Sodium alginate-cross-linked polymyxin B sulphate-loaded solid lipid nanoparticles: Antibiotic resistance tests and HaCat and NIH/3T3 cell viability studies", COLLOIDS AND SURFACES B: BIOINTERFACES, ELSEVIER AMSTERDAM, NL, vol. 129, 28 March 2015 (2015-03-28), pages 191-197, XP029158163, ISSN: 0927-7765, DOI: 10.1016/J.COLSURFB.2015.03.049

## Description

The present invention provides a novel modified form of polymyxin-class antibiotics having advantageous properties. More specially, it has been recognised that covalently conjugating alginate oligomers to polymyxin-class antibiotics reduces the host toxicity of said antibiotics without significantly reducing their antibacterial efficacy and, in some embodiments, even prolonging the antibacterial effects of the antibiotics. In other words polymyxin-alginate oligomer conjugates are a class of novel chemical entities having antibacterial efficacy (which may be essentially the same or similar antibacterial efficacy as non-conjugated polymyxin-class antibiotics) but less host toxicity and even a longer duration of antibacterial effects. The polymyxin-alginate oligomer conjugates of the invention may also be more effective in combating biofilm than the non-conjugated form of the same polymyxin-class antibiotic. The polymyxin-alginate oligomer conjugates of the invention therefore represent improved treatments for bacterial infections in animal subjects. The invention therefore provides medical uses and methods of treatment reflecting these properties of the polymyxin-alginate oligomer conjugates of the invention, namely the use of the polymyxin-alginate oligomer conjugates of the invention in the treatment or prevention of bacterial infections. The invention further provides methods for preparing the conjugates of the invention.

Polymyxin-class antibiotics are a well-known and well-characterised class of cyclic polypeptide antibiotics that bind to and damage the cell membrane bacteria, in particular Gram-negative bacteria. It is understood that polymyxins may also bind to the lipid A portion of bacterial endotoxin and neutralise the biological effects of this endotoxin. Polymyxins are generally described in Hoeprich, P.D., The Polymyxins, Medical Clinics of North America, p. 1257, 54 (5), Sept. 1970, which is herein incorporated by reference in its entirety. These antibiotics were initially described as secondary metabolites from strains of Gram positive bacteria, namely Bacillus sp., e.g. *Bacillus polymyxa varcolistinus* (Minor, R.W., ed.: Antibiotics derived from Bacillus polymyxin. An. N. Y Acad. Sci, 51:853, 1949, which is herein incorporated by reference) and include substances known as circulins and colistins, e.g. polymyxins A1, A2, B1, B1-I, B2, B3, B4, B5, B6, C (circulin A), D1, D2, E1 (colistin A), E2 (colistin B), F, K1, K2, M, P1, P2, S and T (Velkov et al, J, Med. Chem., 2010, 53, 1898-1916 and the accompanying supporting information, Storm et al, Ann. Rev. Biochem. 46:723-63, 1977 and Srinivasa and Ramachandran, Ind. J. Biophys., 17: 112-118, 1979. Polymyxin structures are disclosed in The Merck Index, 15th Ed. (2013); Principles of Medicinal Chemistry, 2nd Ed. 1981, p. 779.

The polymyxins may be described generally as cationic branched cyclic decapeptides, more specifically heptacyclic peptides having a tripeptide side chain, which demonstrate antibacterial efficacy, believed to be by virtue of a surfactant activity. A hydrophobic fatty acid tail linked to the α-amino group of the N-terminal amino acid residue of the tripeptide is typically present. Derivatives thereof, including truncated nonapeptide forms in which the N-terminal amino acid residue of the tripeptide has been removed, have since been synthesised with varying degrees of success.

Functionally, the class is very effective against Gram negative bacteria, especially Gram-negative bacilli such as *Pseudomonas aeruginosa, Escherichia coli, Klebsiella sp., Enterobacter sp., Salmonella sp., Shigella sp., Vibrio sp., Pasteurella sp., Hemophilus sp.,* and *Bordetella sp.* Effects against Gram-positive bacteria vary and are limited in most cases. The class is however beset with toxicity issues (that is toxicity to the subject or "host" to whom the polymixin antibiotic is administered, namely "host toxicity"), especially nephrotoxicity and neurotoxicity. Polymyxin derivatives have been prepared which overcome this issue to varying degrees while retaining antibiotic properties to varying degrees.

The serious host toxicity problems of the polymyxin-class antibiotics meant that their use was curtailed for many years. However, the recent need for "new" antibiotics which can be employed in the combat of multidrug resistant bacteria (so called antibiotics of last resort) has led to the increased use of polymyxin-class antibiotics despite their host toxicity problems. Today the most commonly used polymyxin-class antibiotics are colistin (a mixture of polymyxin E1 and E2) and polymyxin B (a mixture of polymyxins B1, B1-I, B2, B3, and B6, with B1 and B2 predominating). If systemic administration is required, colistin is typically used in sulfomethylated form (colistin methanesulfonate) as it has been found that colistin carrying sulfomethylated amine groups, when administered systemically, is less toxic than unmodified colistin. It is however less potent as an antibacterial agent. Upon administration CMS is hydrolysed to partially sulfonmethylated derivatives and so some antibacterial activity is restored. The nonapeptide truncated forms of polymyxins also have reduced toxicity.

In other approaches small dextrin molecules have been attached to colistin molecules via a succinyl linker and such conjugates have been shown to have reduced host toxicity (WO 2012/035310). These conjugates do however have drastically reduced antibacterial effects which may be partially reinstated with amylase catalysed digestion of the dextrin groups to (Table 3; Example 1, page 17). It is notable that full antibiotic activity is not recovered in any of the experiments reported. It has further been observed that in a two compartment model of biological membrane diffusion these conjugates do not display the early pharmacokinetic/pharmacodynamic (PK/PD) profile of non-conjugated colistin (Azzopardi E. et al., Antimicrob. Agents Chemother., 2015, vol. 59(4), 1837-1843). It was suggested that non-conjugated colistin should be administered alongside the conjugated form to mitigate the reduced effectiveness of the dextrin:colistin conjugates.

There is therefore a continuing need for antibiotics having the antibacterial effects of the polymyxin-class antibiotics without the host toxicity problems of the polymyxin-class antibiotics. Put differently, there is a need to identify a means to reduce the host toxicity problems of the polymyxin-class antibiotics without compromising the antibacterial effectiveness of those antibiotics.

Alginate oligomers have been described in the literature at length. Briefly, alginates are linear polymers of (1-4) linked β-D-mannuronic acid (M) and/or its C-5 epimer α-L-guluronic acid (G). The primary structure of alginates can vary greatly. The M and G residues can be organised as homopolymeric blocks of contiguous M or G residues, as blocks of alternating M and G residues and single M or G residues can be found interspacing these block structures. An alginate molecule can comprise some or all of these structures and such structures might not be uniformly distributed throughout the polymer. In the extreme, there exists a homopolymer of guluronic acid (polyguluronate) or a homopolymer of mannuronic acid (polymannuronate). Alginate oligomers may be obtained from alginate polymers which are typically isolated from natural sources as large high molecular weight polymers (e.g. an average molecular weight in the range 300,000 to 500,000 Daltons). Such large alginate polymers may be degraded, or broken down, e.g. by chemical or enzymatic hydrolysis to produce alginate structures of lower molecular weight (i.e. alginate oligomers).

As shown in the Examples, it has now been found that covalent conjugation of polymyxin-class antibiotics to alginate oligomers creates a novel chemical entity with antibacterial efficacy (which may be essentially the same as or similar to, or at least not significantly reduced as compared to, the antibacterial efficacy of the polymyxin-class antibiotic) but with reduced host toxicity as compared to the non-conjugated form of the polymyxin-class antibiotic. It has been further found that the antibacterial effects of the polymyxin-alginate oligomer conjugate can be prolonged as compared to the non-conjugated form of the polymyxin-class antibiotic. It has also been found that certain polymyxin-alginate oligomer conjugates may be prepared which, in certain physiological models, have an early PK/PD profile which is similar to the non-conjugated form of the polymyxin-class antibiotic.

Accordingly, in a first aspect the invention provides a polymyxin-alginate oligomer conjugate comprising a polymyxin-class antibiotic connected covalently to at least one alginate oligomer via a direct covalent bond or a covalent molecular linker, wherein said alginate oligomer has 2 to 75 monomer residues.

The polymyxin-alginate oligomer conjugates may also be described by Formula I:

P-(L-A)ₙ (I)

wherein P- is a polymyxin-class antibiotic, L is a direct covalent bond or a covalent molecular linker, -A is an alginate oligomer and n is an integer of 1 to 10, e.g. 1 to 9, 1 to 8, 1 to 7, 1 to 6, 1 to 5, 1 to 4, 1 to 3, or 2 or 1.

In accordance with the invention a polymyxin-class antibiotic is broadly defined as a cationic cyclic deca- or nona-peptide which demonstrates antibacterial efficacy wherein said peptide consists of a heptacyclic peptide with a tri- or di-peptide side chain. In certain embodiments, a hydrophobic fatty acid tail is linked to the α-amino group of the N-terminal amino acid residue of the tri- or di-peptide, and which may, in some embodiments be saturated or unsaturated (e.g. an alkanoyl, an alkenoyl or an alkynol), branched or straight chain fatty acid, optionally substituted with one or more hydroxyl groups. The fatty acid residues may be C₃-C₂₀, e.g. C₃-₁₈, C₃-₁₆, C₃-₁₄, C₃-₁₂, C₃-₁₀, C₃-₈, C₃-₆, C₆-₂₀, C₆-₁₈, C₆-₁₆, C₆-₁₄, C₆-₁₂, C₆-₁₀, C₆₋₈, C₁₀₋₂₀, C₁₀₋₁₈, C₁₀₋₁₆, C₁₀₋₁₄, or C₁₀₋₁₂.

Specifically contemplated herein are naturally occurring polymyxins or functionally equivalent derivatives thereof which retain antibacterial efficacy, including fully and semi-synthetic forms. Thus included with the term polymyxin-class antibiotic are polymyxins A1, A2, B1, B1-I, B2, B3, B4, B5, B6, C (circulin A), D1, D2, E1 (colistin A), E2 (colistin B), F, K1, K2, M, P1, P2, S and T, e.g. as described, *inter alia*, in Velkov et al, J, Storm et al, Srinivasa and Ramachandran, and The Merck Index, 15th Ed., *supra.* Functionally equivalent derivatives are described in, inter alia, WO 2010/130007, WO 2008/017734, WO 2010/075416, WO 2012/168820, WO 2009/098357, WO 2014/188178, WO 2015/135976, WO 2013/072695, WO 2016/083531, WO 2016/100578.

By way of example a polymyxin-class antibiotic may be represented by antibacterial molecules of Formula II 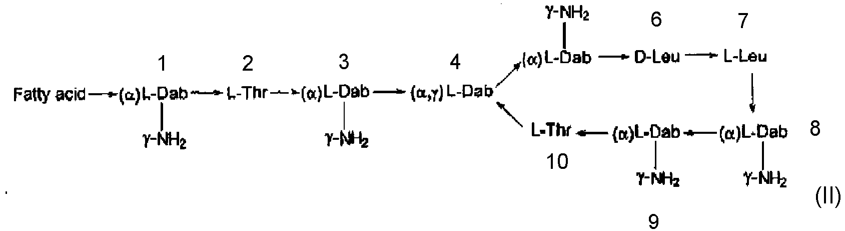 wherein Fatty acid and amino acid residue 1 are independently optional, wherein D-Leu is D-leucine; L-Leu is L-leucine; L-Thr is L-threonine and L-Dab is α,γ-diaminobutyric acid, and wherein none, or one or more, of amino acids 1 to 10 is replaced by another amino acid residue which may be selected from natural or non-genetically encoded amino acids, e.g. leucine, threonine, phenylalanine, arginine, histidine, lysine, asparagine, serine, cysteine, homolysine, ornithine, diaminobutyric acid (e.g. α,γ-diaminobutyric acid), diaminopimelic acid, diaminopropionic acid, homoarginine, trimethylysine, trimethylornithine, 4-aminopiperidine-4-carboxylic acid, 4-amino-1-carbamimidoylpiperidine-4-carboxylic acid and 4-guanidinophenylalanine. The substituted forms may be considered functional equivalent derivatives of polymyxin E1 and/or E2 (colistin), i.e derivatives which retain (e.g. have at least 70%, 80%, 90% or 95% of the) the antibacterial efficacy of polymyxin E1 and/or E2.

Preferably the substituting amino acid is an amino acid with a cationic side chain, i.e. an amino acid that has a side chain that has a net positive charge at the intracellular pH of a tumour cell, e.g. around pH 7.4. Of the genetically coded amino acids this would include lysine and arginine but any non-genetically coded or modified amino acid carrying such a net positive charge on its side chain may be used, e.g. those amino acids carrying a side-chain with a guanidino group or an amine group or another cationic moiety, e.g. derivatives of lysine, and arginine in which any hydrogen in the side chain, except the protonating hydrogen, is substituted with a halogen atom, e.g. fluorine, chlorine or bromine, or a linear, branched aliphatic unsaturated or saturated C₁-C₄ alkyl or alkoxy group, e.g. methyl, ethyl, propyl, iso-propyl, butyl, iso-butyl, sec-butyl, tert-butyl, ethylene, propylene, butylene, hydroxy, methoxy, ethyloxy, propyloxy, iso-propyloxy, butyloxy group, iso-butyloxy, sec-butyloxy, tert-butyloxy or halogen substituted versions thereof. Suitable non-genetically coded amino acids with cationic side chains include homolysine, ornithine, diaminobutyric acid, diaminopimelic acid, diaminopropionic acid and homoarginine as well as trimethylysine and trimethylornithine, 4-aminopiperidine-4-carboxylic acid, 4-amino-1-carbamimidoylpiperidine-4-carboxylic acid and 4-guanidinophenylalanine. The substituted forms of Formula II encompassed by the invention will retain (e.g. have at least 70%, 80%, 90% or 95% of the) antibacterial efficacy of the unsubstituted form of Formula II.

The "fatty acid" group may be any of those described above, preferably methyloctanoic acid or methylheptanoic acid, e.g. 6-methyloctanoic acid or 6-methylheptanoic acid.

An amino acid is a molecule containing an amine group, a carboxylic acid group and at least one carbon separating these two groups. Other groups may be attached to the separating carbon(s). These groups may be referred to as "side-chains" although at its most simple the side chains could be hydrogen (glycine). Amino acids with a single separating carbon are termed "a-amino acids" and have the generic formula H₂NCR₁R₂COOH, where R₁ and R₂ are substituent groups, i.e. are side-chains. The separating carbon is known as the α-carbon. Other types of amino acid exist where the amino and carboxylic acid groups are separated by more than a single carbon atom; for example, in β-amino acids the carbon atom to which the amino group is separated from the carboxylic acid group by two carbon atoms and in γ-amino acids three carbon atoms separate the amino and carboxylic acid groups. Preferably the amino acids in the polymyxin-class antibiotic of use in the invention will be α, β or γ-amino acids, more preferably α or β-amino acids and most preferably α-amino acids.

Amino acids, with the exception of glycine, may exist as two or more stereoisomers. In particular the α-carbon of an amino acid other than glycine is a chiral centre and so gives rise to two enantiomeric forms of each amino acid. These forms are often referred to as D and L forms, e.g. D-alanine and L-alanine. Amino acids with further chiral centres will exist in four or more possible stereoisomers, e.g. threonine has two chiral centres and so may exist in one of four stereoisomeric forms. Any stereoisomeric form of an amino acid may be present the polymyxin-class antibiotic molecules of use in the invention. For the purposes of describing the present invention, where the term "non-genetically encoded" is applied to amino acids, this does not include the D forms of amino acids that occur in nature in the L form.

In preferred embodiments the polymyxin-class antibiotic is selected from polymyxin B1, B1-I, B2, B3, B4, B5, B6, E1 or E2, more preferably selected from polymyxin B1, B1-I, B2, E1 or E2 and most preferably will be either polymyxin E1 or E2 or functional equivalent derivatives of any of the above, i.e derivatives which retain (e.g. have at least 70%, 80%, 90% or 95% of the) the antibacterial efficacy of the polymyxin in question.

In certain embodiments one or more free amine groups in the polymyxin-class antibiotic may be masked by modification, e.g. by sulfomethylation.

As noted above, alginates typically occur as polymers of an average molecular mass of at least 35,000 Daltons, i.e. approximately 175 to approximately 190 monomer residues, although typically much higher. An alginate oligomer for use in accordance with the present invention will, on the other hand, contain 2 to 75 monomer residues, and may contain 3, 4, 5 or 6 to 75, 2, 3, 4, 5 or 6 to 50, 2, 3, 4, 5 or 6 to 40, 2, 3, 4, 5 or 6 to 35 or 2, 3, 4, 5 or 6 to 30 residues. Thus, an alginate oligomer for use according to the invention will typically have an average molecular weight of 350, 550, 700, 900 or 1000 to 15,000 Daltons, 350, 550, 700, 900 or 1000 to 10,000 Daltons, 350, 550, 700, 900 or 1000 to 8000 Daltons, 350, 550, 700, 900 or 1000 to 7000 Daltons, or 350, 550, 700, 900 or 1000 to 6,000 Daltons.

Alternatively put, the alginate oligomer may have a degree of polymerisation (DP), or a number average degree of polymerisation (DPn) of 2 to 75, preferably 2 to 50, more preferably 2 to 40, 2 to 35, 2 to 30, 2 to 28, 2 to 25, 2 to 22, 2 to 20, 2 to 18, 2 to 17, 2 to 15 or 2 to 12.

Other representative ranges (whether for the number of residues, DP or DPn) include any one of 3, 4, 5, 6, 7, 8, 9, 10 or 11 to any one of 50, 45, 40, 39, 38, 37, 36, 35, 34, 33, 32, 31, 30, 29, 28, 27, 26, 25, 24, 23, 22, 21, 20, 19, 18, 17, 16, 15, 14, 13 or 12.

Other representative ranges (whether for the number of residues, DP or DPn) include any one of 8, 9, 10, 11, 12, 13, 14 or 15 to any one of 50, 45, 40, 39, 38, 37, 36, 35, 34, 33, 32, 31, 30, 29, 28, 27, 26, 25, 24, 23, 22, 21, 20, 19, 18, 17 or 16.

Other representative ranges (whether for the number of residues, DP or DPn) include any one of 11, 12, 13, 14, 15, 16, 17 or 18 to any one of 50, 45, 40, 39, 38, 37, 36, 35, 34, 33, 32, 31, 30, 29, 28, 27, 26, 25, 24, 23, 22, 21, 20 or 19.

To the extent permitted by the claims, it may in some embodiments be advantageous to select a larger alginate oligomer so as to create a conjugate of greater size. Larger conjugates may help deliver the polymyxin-class antibiotics selectively to sites and locations of infection because the vascular permeability of such areas within a subject is typically greater than in the vasculature of non-infected areas. Consequently larger conjugates are less likely to enter non-infected areas from the blood stream, but would able to enter the more permeable infected areas. A representative size range for such a larger oligomer may for example be 20 to 75 residues (or DP or DPn of 20 to 75) or any one of 21, 22, 23, 24 or 25, to any one of 75, 70, 65, 60, 55, 50, 45, 40, 35 or 30 residues (or DP or DPn of any one of these ranges) or any one of 30, 31, 32, 33, 34 or 35, to any one of 75, 70, 65, 60, 55, 50, 45 or 40 residues (or DP or DPn of any one of these ranges). Alternatively, these results might be also be achieved by increasing the numbers of alginate oligomers, even those of smaller size, in the conjugate.

An alginate oligomer will, as noted above, contain (or comprise) guluronate or guluronic acid (G) and/or mannuronate or mannuronic acid (M) residues or units. An alginate oligomer according to the invention will preferably be composed solely, or substantially solely (i.e. consist essentially of) uronate/uronic acid residues, more particularly solely or substantially solely of G and/or M residues. Alternatively expressed, in the alginate oligomer of use in the present invention, at least 80%, more particularly at least 85, 90, 95 or 99% of the monomer residues may be uronate/uronic acid residues, or, more particularly G and/or M residues. In other words, preferably the alginate oligomer will not comprise other residues or units (e.g. other saccharide residues, or more particularly other uronic acid/uronate residues).

The alginate oligomer is preferably a linear oligomer.

More particularly, the alginate oligomers proposed for use according to the present invention will contain at least 70% G residues (i.e. at least 70% of the monomer residues of the alginate oligomer will be G residues). Specific embodiments thus include alginate oligomers with (e.g. containing) 70 to 100% G (guluronate) residues.

Preferably at least 75% or 80%, more particularly at least 85% or 90%, even more particularly at least 91, 92, 93, 94, 95, 96, 97, 98 or 99% of the monomer residues are guluronate. In one embodiment the alginate oligomer may be an oligoguluronate (i.e. a homooligomer of G, or 100% G).

In a further preferred embodiment, the above described alginates of use in the invention have a primary structure wherein the majority of the G residues are in so called G-blocks. Preferably at least 50%, more preferably at least 70 or 75%, and most preferably at least 80, 85, 90, 92 or 95% of the G residues are in G-blocks. A G block is a contiguous sequence of at least two G residues, preferably at least 3 contiguous G residues, more preferably at least 4 or 5 contiguous G residues, most preferably at least 7 contiguous G residues.

In particular, at least 90% of the G residues are linked 1-4 to another G residue. More particularly at least 95%, more preferably at least 98%, and most preferably at least 99% of the G residues of the alginate are linked 1-4 to another G residue. More specifically at least 70% of the monomer residues in the oligomer are G residues linked 1-4 to another G-residue, or more preferably at least 75%, and most preferably at least 80, 85, 90, 92, 93, 94, 95, 96, 97, 98, 99% of the monomers residues of the oligomer are G residues linked 1-4 to another G residue. This 1-4 linkage of two G residues can be alternatively expressed as a guluronic unit bound to an adjacent guluronic unit.

The alginate oligomers of use in the invention are commonly referred to by the skilled person as "high G" or "G-block" oligomers i.e. having a high content of G residues or G-blocks (e.g. wherein at least 70% of the monomer residues are G, preferably arranged in G-blocks).

The alginate oligomer of use in the invention is preferably a 3- to 35-mer, more preferably a 3- to 28-mer, in particular a 4- to 25-mer, e.g. a 5- to 20-mer, especially a 6- to 22-mer, in particular an 8- to 20-mer, especially a 10- to 15-mer, e.g. having a molecular weight in the range 350 to 6400 Daltons or 350 to 6000 Daltons, preferably 550 to 5500 Daltons, preferably 750 to 5000 Daltons, and especially 750 to 4500 Daltons or 2000 to 3000 Daltons or 900 to 3500 Daltons. Other representative alginate oligomers include, as mentioned above, oligomers with 5, 6, 7, 8, 9, 10, 11, 12 or 13 to 50, 45, 40, 35, 28, 25, 22 or 20 residues.

It may be a single compound or it may be a mixture of compounds, e.g. of a range of degrees of polymerization. As noted above, the monomeric residues in the alginate oligomer, may be the same or different and not all need carry electrically charged groups although it is preferred that the majority (e.g. at least 60%, preferably at least 80% more preferably at least 90%) do. It is preferred that a substantial majority, e.g. at least 80%, more preferably at least 90% of the charged groups have the same polarity. In the alginate oligomer, the ratio of hydroxyl groups to charged groups is preferably at least 2:1, more especially at least 3:1.

The alginate oligomer of use in the invention may have a degree of polymerisation (DP), or a number average degree of polymerisation (DPₙ), of 3-28, 4-25, 6-22, 8-20 or 10-15, or 5-18 or 7-15 or 8-12, especially 10.

The alginate oligomer of use in the invention may have a degree of polymerisation (DP), or a number average degree of polymerisation (DPₙ), of 3-24, 4-23, 5-22, 6-21, 7-20, 8-19, 9-18, 10-17, 11-16, 12-15 or 13-14 (e.g. 13 or 14).

The alginate oligomer of use in the invention may have a degree of polymerisation (DP), or a number average degree of polymerisation (DPn), of 4-25, 5-24, 6-23, 7-22, 8-21, 9-20, 10-19, 11-18, 12-17, 13-16, 14-15 (e.g. 14 or 15).

The alginate oligomer of use in the invention may have a degree of polymerisation (DP), or a number average degree of polymerisation (DPₙ), of 5-26, 6-25, 7-24, 8-23, 9-22, 10-21, 11-20, 12-19, 13-18, 14-17 or 15-16 (e.g. 15 or 16).

The alginate oligomer of use in the invention may have a degree of polymerisation (DP), or a number average degree of polymerisation (DPₙ), of 4-50, 4-40, 4-35, 4-30, 4-28, 4-26, 4-22, 4-20, 4-18, 4-16 or 4-14.

The alginate oligomer of use in the invention may have a degree of polymerisation (DP), or a number average degree of polymerisation (DPₙ), of 5-50, 5-40, 5-25, 5-22, 5-20, 5-18, 5-23, 5-20, 5-18, 5-16 or 5-14.

The alginate oligomer of use in the invention may have a degree of polymerisation (DP), or a number average degree of polymerisation (DPₙ), of 6-50, 6-40, 6-35, 6-30, 6-28, 6-26, 6-24, 6-20, 6-19, 6-18, 6-16 or 6-14.

The alginate oligomer of use in the invention may have a degree of polymerisation (DP), or a number average degree of polymerisation (DPₙ), of 8-50, 8-40, 8-35, 8-30, 8-28, 8-25, 8-22, 8-20, 8-18, 8-16 or 8-14.

The alginate oligomer of use in the invention may have a degree of polymerisation (DP), or a number average degree of polymerisation (DPₙ), of 9-50, 9-40, 9-35, 9-30, 9-28, 9-25, 9-22, 9-20, 9-18, 9-16 or 9-14.

The alginate oligomer of use in the invention may have a degree of polymerisation (DP), or a number average degree of polymerisation (DPₙ), of 10-50, 10-40, 10-35, 10-30, 10-28, 10-25, 10-22, 10-20, 10-18, 10-16 or 10-14.

The alginate oligomer of use in the invention may have a degree of polymerisation (DP), or a number average degree of polymerisation (DPₙ), of 11-50, 11-40, 11-35, 11-30, 11-28, 11-25, 11-22, 11-20, 11-18, 11-16 or 11-14.

The alginate oligomer of use in the invention may have a degree of polymerisation (DP), or a number average degree of polymerisation (DPₙ), of 12-50, 12-40, 12-35, 12-30, 12-28, 12-25, 12-22, 12-20, 12-18, 12-16 or 12-14.

The alginate oligomer of use in the invention may have a degree of polymerisation (DP), or a number average degree of polymerisation (DPₙ), of 13-50, 13-40, 13-35, 13-30, 13-28, 13-25, 13-22, 13-20, 13-18, 13-16 or 13-14 (e.g. 13 or 14).

The alginate oligomer of use in the invention may have a degree of polymerisation (DP), or a number average degree of polymerisation (DPₙ), of 14-50, 14-40, 14-35, 14-30, 14-28, 14-25, 14-22, 14-20, 14-18, or 14-16.

The alginate oligomer of use in the invention may have a degree of polymerisation (DP), or a number average degree of polymerisation (DPₙ), of 15-50, 15-40, 15-35, 15-30, 15-28, 15-25, 15-22, 15-20, or 15-18.

The alginate oligomer of use in the invention may have a degree of polymerisation (DP), or a number average degree of polymerisation (DPₙ), of 18-50, 18-40, 18-35, 18-30, 18-28, 18-25, 18-22 or 18-20.

The alginate oligomer of use in the invention may have a degree of polymerisation (DP), or a number average degree of polymerisation (DPₙ), of 20-75, 20-70, 20-65, 20-60 , 20-55, 20-50, 20-45, 20-40, 20-35, 20-30 or 20-25.

The alginate oligomer of use in the invention may have a degree of polymerisation (DP), or a number average degree of polymerisation (DPn), of 30-75, 30-70, 30-65, 30-60, 30-55, 30-50, 30-45, 30-40 or 30-35.

Preferably the alginate oligomer of use in the invention is substantially free, preferably essentially free, of alginate oligomers having a degree of polymerisation outside of the ranges disclosed herein. This may be expressed in terms of the molecular weight distribution of the alginate oligomer of the invention, e.g. the percentage of each mole of the alginate oligomer being used in accordance with the invention which has a DP outside the relevant range. The molecular weight distribution is preferably such that no more than 10%, preferably no more than 9, 8, 7, 6, 5, 4, 3, 2, or 1% mole has a DP of three, two or one higher than the relevant upper limit for DPₙ. Likewise it is preferred that no more than 10%, preferably no more than 9, 8, 7, 6, 5, 4, 3, 2, or 1% mole has a DP below a number three, two or one smaller than the relevant lower limit for DPₙ.

Suitable alginate oligomers are described in WO2007/039754, WO2007/039760, WO 2008/125828, and WO2009/068841.

Representative suitable alginate oligomers have a DPₙ in the range 5 to 30, a guluronate fraction (F_{G}) of at least 0.80, a mannuronate fraction (F_{M}) of no more than 0.20, and at least 95 mole% of DP no more than 25.

Further suitable alginate oligomers have a number average degree of polymerization in the range 7 to 15 (preferably 8 to 12), a guluronate fraction (F_{G}) of at least 0.85 (preferably at least 0.90), a mannuronate fraction (F_{M}) of no more than 0.15 (preferably no more than 0.10), and having at least 95% mole with a degree of polymerization less than 17 (preferably less than 14).

Further suitable alginate oligomers have a number average degree of polymerization in the range 5 to 18 (especially 7 to 15), a guluronate fraction (F_{G}) of at least 0.80 (preferably at least 0.85, especially at least 0.92), a mannuronate fraction (F_{M}) of no more than 0.20 (preferably no more than 0.15, especially no more than 0.08), and having at least 95% mole with a degree of polymerization less than 20 (preferably less than 17).

Further suitable alginate oligomers have a number average degree of polymerization in the range 5 to 18, a guluronate fraction (F_{G}) of at least 0.92, a mannuronate fraction (F_{M}) of no more than 0.08, and having at least 95% mole with a degree of polymerization less than 20.

Further suitable alginate oligomers have a number average degree of polymerization in the range 5 to 18 (preferably 7 to 15, more preferably 8 to 12, especially about 10), a guluronate fraction (F_{G}) of at least 0.80 (preferably at least 0.85, more preferably at least 0.90, especially at least 0.92, most especially at least 0.95), a mannuronate fraction (F_{M}) of no more than 0.20 (preferably no more than 0.15, more preferably no more than 0.10, especially no more than 0.08, most especially no more than 0.05), and having at least 95% mole with a degree of polymerization less than 20 (preferably less than 17, more preferably less than 14).

Further suitable alginate oligomers have a number average degree of polymerization in the range 7 to 15 (preferably 8 to 12), a guluronate fraction (F_{G}) of at least 0.92 (preferably at least 0.95), a mannuronate fraction (F_{M}) of no more than 0.08 (preferably no more than 0.05), and having at least 95% mole with a degree of polymerization less than 17 (preferably less than 14).

Further suitable alginate oligomers have a number average degree of polymerization in the range 5 to 18, a guluronate fraction (F_{G}) of at least 0.80, a mannuronate fraction (F_{M}) of no more than 0.20, and having at least 95% mole with a degree of polymerization less than 20.

Further suitable alginate oligomers have a number average degree of polymerization in the range 7 to 15, a guluronate fraction (F_{G}) of at least 0.85, a mannuronate fraction (F_{M}) of no more than 0.15, and having at least 95% mole with a degree of polymerization less than 17.

Further suitable alginate oligomers have a number average degree of polymerization in the range 7 to 15, a guluronate fraction (F_{G}) of at least 0.92, a mannuronate fraction (F_{M}) of no more than 0.08, and having at least 95% mole with a degree of polymerization less than 17.

Further suitable alginate oligomers have a number average degree of polymerization in the range 5 to 20, a guluronate fraction (F_{G}) of at least 0.85 and a mannuronate fraction (F_{M}) of no more than 0.15.

Further suitable alginate oligomers have a number average degree of polymerization in the range 5 to 20, a guluronate fraction (F_{G}) of 0.9-0.95 and a mannuronate fraction (F_{M}) of 0.05-0.1, which may be expressed as an alginate oligomer having 90-95% G residues and an average molecular weight of 2600 Da. Further suitable alginate oligomers have a number average degree of polymerization about 13 (e.g. 12, 13 or 14), a guluronate fraction (F_{G}) of at least about 0.80, 0.85, 0.87, 0.88, 0.90 or 0.93 (e.g. 0.92, 0.93 or 0.94) and a corresponding mannuronate fraction (F_{M}) of no more than about 0.20, 0.15, 0.13, 0.12, 0.10, or 0.07 (e.g. 0.08, 0.07 or 0.06).

Further suitable alginate oligomers have a number average degree of polymerization about 21 (e.g. 20, 21 or 22), a guluronate fraction (F_{G}) of at least about 0.80 (e.g. 0.85, 0.87, 0.88, 0.90, 0.92, 0.94 or 0.95) and a corresponding mannuronate fraction (F_{M}) of no more than about 0.20 (e.g. 0.15, 0.13, 0.12, 0.10, 0.08, 0.06, 0.05).

Further suitable alginate oligomers have a number average degree of polymerization about 6 (e.g. 5, 6 or 7), a guluronate fraction (F_{G}) of at least about 0.80 (e.g. 0.85, 0.87, 0.88, 0.90, 0.92, 0.94 or 0.95) and a corresponding mannuronate fraction (F_{M}) of no more than about 0.20 (e.g. 0.15, 0.13, 0.12, 0.10, 0.08, 0.06, 0.05).

It will thus be seen that a particular class of alginate oligomers favoured for use according to the present invention is alginate oligomers defined as so-called "high G" or "G-block" oligomers i.e. having a high content of G residues or G-blocks (e.g. wherein at least 70% of the monomer residues are G, preferably arranged in G-blocks). However, other types of alginate oligomer may also be used, including in particular "high M" or "M-block" oligomers or MG-block oligomers, as described further below. Accordingly, it is alginate oligomers with high proportions of a single monomer type, and with said monomers of this type being present predominantly in contiguous sequences of that monomer type, that represent oligomers that are particularly preferred, e.g. oligomers wherein at least 70% of the monomer residues in the oligomer are G residues linked 1-4 to another G-residue, or more preferably at least 75%, and most preferably at least 80, 85, 90, 92, 93, 94, 95, 96, 97, 98, 99% of the monomers residues of the oligomer are G residues linked 1-4 to another G residue. This 1-4 linkage of two G residues can be alternatively expressed as a guluronic unit bound to an adjacent guluronic unit.

In a further embodiment at least, or more particularly more than, 50% of the monomer residues of the alginate oligomer may be M residues (i.e. mannuronate or mannuronic acid). In other words the alginate oligomer will contain at least or alternatively more than 50% mannuronate (or mannuronic acid) residues. Specific embodiments thus include alginate oligomers with (e.g. containing) 50 to 70% M (mannuronate) residues or e.g. 70 to 100% M (mannuronate) residues. Further specific embodiments also include oligomers containing 71 to 85% M residues or 85 to 100% M residues. Thus, a representative alginate oligomer for use according to this embodiment of the present invention will contain more than 70% M residues (i.e. more than 70% of the monomer residues of the alginate oligomer will be M residues).

In other embodiments at least 50% or 60%, more particularly at least 70% or 75%, even more particularly at least 80, 85, 90, 95 or 99% of the monomer residues are mannuronate. In one embodiment the alginate oligomer may be an oligomannuronate (i.e. a homooligomer of M, or 100% M).

In a further embodiment, the above described alginates of use in the invention have a primary structure wherein the majority of the M residues are in so called M-blocks. In this embodiment preferably at least 50%, more preferably at least 70 or 75%, and most preferably at least 80, 85, 90 or 95% of the M residues are in M-blocks. An M block is a contiguous sequence of at least two M residues, preferably at least 3 contiguous M residues, more preferably at least 4 or 5 contiguous M residues, most preferably at least 7 contiguous M residues.

In particular, at least 90% of the M residues are linked 1-4 to another M residue. More particularly at least 95%, more preferably at least 98%, and most preferably at least 99% of the M residues of the alginate are linked 1-4 to another M residue.

Other preferred oligomers are alginate oligomers wherein at least 70% of the monomer residues in the oligomer are M residues linked 1-4 to another M-residue, or more preferably at least 75%, and most preferably at least 80, 85, 90, 92, 93, 94, 95, 96, 97, 98, 99% of the monomers residues of the oligomer are M residues linked 1-4 to another M residue. This 1-4 linkage of two M residues can be alternatively expressed as a mannuronic unit bound to an adjacent mannuronic unit.

In a still further embodiment, the alginate oligomers of use in the invention comprise a sequence of alternating M and G residues. A sequence of at least three, preferably at least four, alternating M and G residues represents an MG block. Preferably the alginate oligomers of the invention comprise an MG block. Expressed more specifically, an MG block is a sequence of at least three contiguous residues consisting of G and M residues and wherein each non-terminal (internal) G residue in the contiguous sequence is linked 1-4 and 4-1 to an M residue and each non-terminal (internal) M residue in the contiguous sequence is linked 1-4 and 4-1 to a G residue. Preferably the MG block is at least 5 or 6 contiguous residues, more preferably at least 7 or 8 contiguous residues.

In a further embodiment the minority uronate in the alginate oligomer (i.e. mannuronate or guluronate) is found predominantly in MG blocks. In this embodiment preferably at least 50%, more preferably at least 70 or 75% and most preferably at least 80, 85, 90 or 95% of the minority uronate monomers in the MG block alginate oligomer are present in MG blocks. In another embodiment the alginate oligomer is arranged such that at least 50%, at least 60%, at least 70%, at least 80%, at least 85%, at least 90%, at least 95%, at least 99%, e.g. 100% of the G and M residues in the oligomer are arranged in MG blocks.

Although at its broadest, the invention extends to embodiments wherein at least 1% but less than 100% of the monomer residues of the oligomer are G residues (i.e. guluronate or guluronic acid), more particularly, and as defined further below, at least 30% of the monomer residues are G residues. Thus, at its broadest the MG block containing alginate oligomer may contain at least 1%, but less than 100%, guluronate (or guluronic acid) residues, but generally the MG block containing alginate oligomer will contain at least 30% (or at least 35, 40 or 45% or 50% G) but less than 100% G. Specific embodiments thus include MG block containing alginate oligomers with (e.g. containing) 1 to 30% G (guluronate) residues, 30 to 70% G (guluronate) residues or 70 to 99% G (guluronate) residues. Thus, a representative MG block containing alginate oligomer for use according to the present invention may contain more than 30%, but less than 70%, G residues (i.e. more than 30%, but less than 70%, of the monomer residues of the MG block alginate oligomer will be G residues).

Preferably more than 30%, more particularly more than 35% or 40%, even more particularly more than 45, 50, 55, 60 or 65%, but in each case less than 70%, of the monomer residues of the MG block containing alginate oligomer are guluronate. Alternatively, less than 70%, more preferably less than 65% or 60%, even more preferably less than 55, 50, 45, 40 or 35%, but in each case more than 30% of the monomer residues of the MG block containing alginate oligomer are guluronate. Any range formed by any combination of these values may be chosen. Therefore for instance the MG block containing alginate oligomer can have e.g. between 35% and 65%, 40% and 60% or 45% and 55% G residues.

In another embodiment the MG block containing alginate oligomer may have approximately equal amounts of G and M residues (e.g. ratios between 65% G/35% M and 35% G/65% M, for instance 60% G/40% M and 40% G/60% M; 55% G/45% M and 45% G/55% M; 53% G/47% M and 47% G/53% M; 51% G/49% M and 49% G/51% M; e.g. about 50% G and about 50% M) and these residues are arranged predominantly, preferably entirely or as completely as possible, in an alternating MG pattern (e.g. at least 50% or at least 60, 70, 80, 85, 90 or 95% or 100% of the M and G residues are in an alternating MG sequence).

In certain embodiments the terminal uronic acid residues of the oligomers of use in the invention do not have a double bond, especially a double bond situated between the C₄ and C₅ atom. Such oligomers may be described as having saturated terminal uronic acid residues. The skilled man would be able to prepare oligomers with saturated terminal uronic acid residues without undue burden. This may be through the use of production techniques which yield such oligomers, or by converting (saturating) oligomers produced by processes that yield oligomers with unsaturated terminal uronic acid residues.

The alginate oligomer will typically carry a charge and so counter ions for the alginate oligomer may be any physiologically tolerable ion, especially those commonly used for charged drug substances, e.g. sodium, potassium, ammonium, chloride, mesylate, meglumine, etc. Ions which promote alginate gelation e.g. group 2 metal ions may also be used.

While the alginate oligomer may be a synthetic material generated from the polymerisation of appropriate numbers of guluronate and mannuronate residues, the alginate oligomers of use in the invention may conveniently be obtained, produced or derived from natural sources such as those mentioned above, namely natural alginate source materials.

Polysaccharide to oligosaccharide cleavage to produce the alginate oligomer useable according to the present invention may be performed using conventional polysaccharide lysis techniques such as enzymatic digestion and acid hydrolysis. In one favoured embodiment acid hydrolysis is used to prepare the alginate oligomers use in the invention. In other embodiments enzymatic digestion is used with an additional processing step(s) to saturate the terminal uronic acids in the oligomers.

Oligomers may then be separated from the polysaccharide breakdown products chromatographically using an ion exchange resin or by fractionated precipitation or solubilisation or filtration. US 6,121,441 and WO 2008/125828 describe a process suitable for preparing the alginate oligomers of use in the invention. Further information and discussion can be found in for example in "Handbooks of Hydrocolloids", Ed. Phillips and Williams, CRC, Boca Raton, Florida, USA, 2000.

The alginate oligomers may also be chemically modified, including but not limited to modification to add charged groups (such as carboxylated or carboxymethylated glycans) and alginate oligomers modified to alter flexibility (e.g. by periodate oxidation).

Alginate oligomers (for example oligoguluronic acids) suitable for use according to the invention may conveniently be produced by acid hydrolysis of alginic acid from, but not limited to, *Laminaria hyperbora* and *Lessonia nigrescens*, dissolution at neutral pH, addition of mineral acid reduce the pH to 3.4 to precipitate the alginate oligomer (oligoguluronic acid), washing with weak acid, resuspension at neutral pH and freeze drying.

The alginates for production of alginate oligomers of use in the invention can also be obtained directly from suitable bacterial sources e.g. *Pseudomonas aeruginosa* or *Azotobacter vinelandii.*

In embodiments where alginate oligomers which have primary structures in which the majority of the G residues are arranged in G-blocks rather than as single residues are required, algal sources are expected to be most suitable on account of the fact that the alginates produced in these organisms tend to have these structures. The bacterial sources may be more suitable for obtaining alginate oligomers of different structures.

The molecular apparatus involved in alginate biosynthesis in *Pseudomonas fluorescens* and *Azotobacter vinelandii* has been cloned and characterised (WO 94/09124; Ertesvåg, H., et al, Metabolic Engineering, 1999, Vol 1, 262-269; WO 2004/011628; Gimmestad, M., *et al (supra)*; Remminghorst and Rehm, Biotechnology Letters, 2006, Vol 28, 1701-1712; Gimmestad, M. et al, Journal of Bacteriology, 2006, Vol 188(15), 5551-5560) and alginates of tailored primary structures can be readily obtained by manipulating these systems.

The G content of alginates (for example an algal source material) can be increased by epimerisation, for example with mannuronan C-5 epimerases from A. *vinelandii* or other epimerase enzymes. Thus, for example *in vitro* epimerisation may be carried out with isolated epimerases from *Pseudomonas* or *Azotobacter,* e.g. AlgG from *Pseudomonas fluorescens* or *Azotobacter vinelandii* or the AlgE enzymes (AlgE1 to AlgE7) from *Azotobacter vinelandii.* The use of epimerases from other organisms that have the capability of producing alginate, particularly algae, is also specifically contemplated. The *in vitro* epimerisation of low G alginates with *Azotobacter vinelandii* AlgE epimerases is described in detail in Ertesvåg *et al* (*supra*) and Strugala et al (Gums and Stabilisers for the Food Industry, 2004, 12, The Royal Society of Chemistry, 84 - 94).

To obtain G-block containing alginates or alginate oligomers, epimerisation with one or more *Azotobacter vinelandii* AlgE epimerases other than AlgE4 is preferred as these enzymes are capable of producing G block structures. On the other hand AlgE4 epimerase can be used to create alginates or alginate oligomers with alternating stretches of M/G sequence or primary structures containing single G residue as it has been found that this enzyme seems preferentially to epimerise individual M residues so as to produce single G residues linked to M residues rather than producing G blocks. Particular primary structures can be obtained by using different combinations of these enzymes.

Mutated versions of these enzymes or homologues from other organisms are also specifically contemplated as of use. WO 94/09124 describes recombinant or modified mannuronan C-5 epimerase enzymes (AlgE enzymes) for example encoded by epimerase sequences in which the DNA sequences encoding the different domains or modules of the epimerases have been shuffled or deleted and recombined. Alternatively, mutants of naturally occurring epimerase enzymes, (AlgG or AlgE) may be used, obtained for example by site directed or random mutagenesis of the AlgG or AlgE genes.

A different approach is to create *Pseudomonas* and *Azotobacter* organisms that are mutated in some or all of their epimerase genes in such a way that those mutants produce alginates of the required structure for subsequent alginate oligomer production, or even alginate oligomers of the required structure and size (or molecular weight). The generation of a number of *Pseudomonas fluorescens* organisms with mutated AlgG genes is described in detail in WO 2004/011628 and Gimmestad, M., *et al,* 2003 (*supra*). The generation of a number of *Azotobacter vinelandii* organisms with mutated AlgE genes is disclosed in Gimmestad, M., *et al*, 2006 (*supra*).

A further approach is to delete or inactivate the endogenous epimerase genes from an *Azotobacter* or a *Pseudomonas* organism and then to introduce one or more exogenous epimerase genes, which may or may not be mutated (i.e. may be wild-type or modified) and the expression of which may be controlled, for example by the use of inducible or other "controllable promoters". By selecting appropriate combinations of genes, alginates of predetermined primary structure can be produced.

A still further approach would be to introduce some or all of the alginate biosynthesis machinery of *Pseudomonas* and/or *Azotobacter* into a non-alginate producing organism (e.g. *E. coli*) and to induce the production of alginate from these genetically modified organisms.

When these culture-based systems are used, the primary structure of the alginate or alginate oligomer products can be influenced by the culture conditions. It is well within the capabilities of the skilled man to adjust culture parameters such as temperature, osmolarity, nutrient levels/sources and atmospheric parameters in order to manipulate the primary structure of the alginates produced by a particular organism.

References to "G residues/G" and "M residues/M" or to guluronic acid or mannuronic acid, or guluronate or mannuronate are to be read interchangeably as references to guluronic acid/guluronate and mannuronic acid/mannuronate (specifically α-L-guluronic acid/guluronate and β-D-mannuronic acid/mannuronate), and further include derivatives thereof in which one or more available side chains or groups have been modified without resulting in a capacity to reduce the host toxicity of said antibiotics without significantly reducing their antibacterial efficacy, and optionally also a capacity to prolong the antibacterial effects of the antibiotics, and optionally a capacity to maintain the early PK/PD profile of said antibiotics, which is substantially lower than that of the unmodified oligomer. Common saccharide modifying groups would include acetyl, sulphate, amino, deoxy, alcohol, aldehyde, ketone, ester and anhydro groups. The alginate oligomers may also be chemically modified to add charged groups (such as carboxylated or carboxymethylated glycans), and to alter flexibility (e.g. by periodate oxidation). The skilled man would be aware of still further chemical modifications that can be made to the monosaccharide subunits of oligosaccharides and these can be applied to the alginate oligomers of use in the invention.

The direct covalent bond between the alginate oligomer and the polymyxin-class antibiotic is a covalent bond formed by an atom of the alginate oligomer and an atom of the polymyxin-class antibiotic. The atoms contributing to the bond may together or independently be carbon, oxygen, sulphur, nitrogen and/or phosphorous. The bond may be single, double or triple. In certain embodiments the bond is part of an organic functional group. The skilled person would be entirely familiar with the options available for suitable organic functional groups which could act as linkers between the alginate oligomer and the polymyxin-class antibiotics. Non-limiting examples thereof may include ester, carbonate ester, orthoester, ketone, ketal, hemiketal, ketene, ether, acetal, hemiacteal, peroxy, methylenedioxy, carbamate, amide, amine, amine oxide, hydroxamic acid, imine, imide, imidate, azide, azo, oxime, carbodiimide, carbazone, hydrozone, sulfide, disulfide, sulfinyl, sulfonyl, carbonothioyl, thioamide, thioester, thioether, thioketone, thioketal, sulphonate ester, dithiocarbamate, semicarbazone, phosphine or phosphodiester functional groups. As shown in the Examples, the formation of amide and ester bonds may be convenient and advantageous.

The covalent molecular linker may be any molecule, typically an organic molecule, or part thereof, which has a structure formed from covalently bonded atoms which is capable of bonding covalently with an alginate oligomer and a polymyxin-class antibiotic. Within the conjugate there will be a continuous series of covalently bonded atoms from the alginate oligomer to polymyxin-class antibiotic via the molecular linker. In preferred embodiments at least one of the covalent bonds in in said series is as defined above. The molecular linker may however further comprise non-covalent, e.g. ionic bonds, in parts of the molecule which are not contributing to the covalent linkage between the polymyxin-class antibiotic and the alginate oligomer.

The covalent molecular linker may be linear, circular or branched. In certain embodiments the molecular linker will have a molecular weight of equal to or less than 1500 Daltons, e.g. equal to or less than 1250, 1000, 900, 800, 700, 600, 500, 400, 300, 200 or 100 Daltons.

In certain embodiments at least one direct covalent bond between the alginate oligomer and the covalent molecular linker is as defined above. In certain embodiments at least one direct covalent bond between the polymyxin-class antibiotic and the covalent molecular linker is as defined above. Each bond may be the same or different. The covalent linker molecule may comprise at least one covalent bond as defined above, preferably in the part of that molecule which contributes to the continuous series of covalently bonded atoms from the alginate oligomer to polymyxin-class antibiotic via the molecular linker.

The covalent molecular linker may be or comprise an amino acid or a peptide, e.g. of equal to or fewer than 15 amino acid residues, e.g. of equal to or fewer than 12, 10, 8, 6, 5, 4, 3 or 2 amino acid residues. The amino acid may be and the peptide may comprise any of the amino acids described above. Specific examples of peptide linkers which may be used include but are not limited to peptides of Gly and/or Ser residues (e.g. (Gly)₂₋₈, (Ser)₂₋₈, (GGGGS)₁₋₃); (EAAAK)₁₋₃; A(EAAAK)₁₋₃A; Leu-Glu; (Xaa-Pro)₁₋₆ (e.g. (Glu-Pro)₁₋₆, (Lys-Pro)₁₋₆, (Ala-Pro)₁₋₆; VSQTSKLTR↓AETVFPDV (Factor XIa/Factor VIIa sensitive cleavage); PLG↓LWA (matrix metalloprotease-1 sensitive cleavage); RVL↓AEA (HIV-1 protease sensitive cleavage; EDVVCC↓SMSY (NS3 protease sensitive cleavage; GGIEGR↓GS (Factor Xa sensitive cleavage); TRHRQPR↓GWE (furin sensitive cleavage); AGNRVRR↓SVG (furin sensitive cleavage); GFLG↓ (Cathepsin B sensitive cleavage).

The covalent molecular linker may be or comprise a monosaccharide or an oligosaccharide other than guluronate or mannuronate or a polymer formed therefrom, e.g. a saccharide of equal to or fewer than 12 amino acid residues, e.g. equal to or fewer than 10, 8, 6, 5, 4, 3 or 2 amino acid residues. Thus the covalent molecular linker may be a monosaccharide, disaccharide or trisaccharide or sugar derivatives thereof such as aldonic and uronic acids, deoxy or amino sugars, sulfated sugars, and sugar alcohols. The monosaccharide or one or more of the monosaccharide residues of the disaccharide or trisaccharide may be a triose, a tetrose, a pentose, a hexose, a heptose, an octose, a nonose or a decose in pyranose or furanose form and/or L- or D- form where appropriate and/or sugar derivatives thereof. Pentose or hexose saccharides/residues are preferred, e.g. mannose (e.g. D-mannose), galactose (e.g. D- galactose), glucose (e.g. D-glucose), fructose, fucose (e.g. L-fucose), N-acetyl-glucosamine, N-acetylgalactosamine, rhamnose, galactosamine, glucosamine (e.g. D-glucosamine), galacturonic acid, glucuronic acid, N-acetylneuraminic acid, methyl D-mannopyranoside (mannoside), α-methyl-glucoside, galactoside, ribose, xylose, arabinose, saccharate, mannitol, sorbitol, inositol, glycerol and derivatives of these monomers. The disaccharide may be exemplified by acarviosin, allolactose, cellobiose, chitobiose, galactose-alpha-1,3-galactose, dentiobiose, isomalt, isomaltose, isomaltulose, kojibiose, lactitol, lactobionic acid, lactose, lactulose, laminaribiose, maltitol, maltose, mannobiose, melibiose, melibiulose, neohesperidose, nigerose, robinose, rutinose, sambubiose, sophorose, sucralfate, sucralose, sucrose, sucrose acetate isobutyrate, sucrose octaacetate, trehalose, truranose, xylobiose or derivatives of these disaccharides.

The covalent molecular linker may be or comprise a nucleotide or an oligonucleotide, i.e. a nucleic acid, e.g. a ribonucleotide or a deoxyribonucleotide.

The linker may also be or comprise a straight chain, branched or cyclic, substituted or unsubstituted, alkyl, alkenyl or alkynl group (typically C₂₋₈) or derivative thereof such as aminohexanoic acid or one of a range of commercially available PEG (polyethylene glycol) linkers.

Further examples of suitable covalent linker molecules include but are not limited to acetyl, succinyl, aconityl (*cis* or *trans*), glutaryl, methylsuccinyl, trimellityl cysteamine, penicillamine, N-(2-mercaptopropionyl)glycine, 2-mercaptopropionic acid, homocysteine, 3-mercaptopropionic acid and deamino-penicillamine groups.

In certain embodiments the covalent linker molecule may be a plurality of the molecules and/or groups described above.

In certain embodiments the direct covalent bond or the covalent linker molecule (more specifically a covalent bond within the linker molecule, a covalent bond between the linker molecule the alginate oligomer and/or a covalent bond between and the linker molecule and the polymyxin-class antibiotic) is selected for its ability to be lysed under conditions representative, or advantageously essentially unique to, a target site or location within a subject, e.g. conditions representative of a bacterial infection, the respiratory tract (especially the lower respiratory tract including the lungs, more particularly the lungs of a patient with cystic fibrosis) or wounds (in particular chronic wounds). In this way delivery of the polymyxin-class antibiotic may be made more selective for the target site.

In specific embodiments a covalent bond, a functional group containing said covalent bond or linker molecule may be selected which is sensitive to (labile at, degrades at, lyses at) a pH which is lower than normal physiological pH (pH 7.2), i.e. acidic pH, e.g. a pH of from about 3 to about 7, 6.5, 6, 5.5, 5, 4.5, 4, or 3.5. Sites or locations of inflammation, especially inflammation caused by infection typically have a pH in these ranges. Functional groups including esters, cis-aconityl, disulphides and hydrozones may be sensitive to lower pHs, i.e. may be described as acid labile.

In specific embodiments a covalent bond, functional group or linker molecule may be selected which is sensitive to reactive oxygen species. Sites or locations of inflammation, especially inflammation caused by infection typically have high levels of reactive oxygen species. Functional groups including thioketals and thioethers may be sensitive to reactive oxygen species

In further specific embodiments a covalent bond, functional group or linker molecule may be selected which is lysed by enzymes produced or secreted only at the target site or overproduced or oversecreted at the target site. This may include enzymes such as glycosidases, nucleases and peptidases, in particular those secreted by infecting bacteria and those secreted by inflammatory cells of the host, e.g. lysozyme, alginate lyase, DNasel, restriction endonucleases, neutrophil elastase, cathepsins, phospholipases and β-lactamases. It may however be advantageous to choose a covalent bond, functional group or linker molecule which is not lysed by enzymes capable of degrading the alginate oligomer or the polymyxin-class antibiotic and as such separation of the alginate oligomer from the polymyxin-class antibiotic will occur separately to the degradation of the alginate oligomer or polymyxin-class antibiotic.

In other embodiments the direct covalent bond or the covalent linker molecule may be selected for its stability under conditions representative, of advantageously essentially unique to, a target site or location within a subject, e.g. the location described above or locations or sites the conjugate may encounter en route to those locations and sites following administration and/or which the conjugate may encounter during its bodily distribution. An amide bond, a thioether bond or a Gly-Gly peptide linker may be, for example, suitable here.

In further specific embodiments a covalent bond, functional group or linker molecule may be selected which results in a polymyxin-alginate oligomer conjugate which has an early PK/PD profile which is similar to, preferably substantially or essentially the same as, an early PK/PD profile of the polymyxin-class antibiotic. In accordance with the invention the PK/PD model may be a two compartment model of biological membrane diffusion, e.g. that described in Azzopardi, supra, and/or Example 4, and the profile is determined within 12, e.g. 10, 8, 6 or 4 hours from administration of the active agent under test. An ester bond may be, for example, suitable here.

In preferred embodiments the polymyxin-alginate oligomer conjugate consists of at least one alginate oligomer covalently bonded to a polymyxin-class antibiotic via an amide bond formed from a carboxyl group on the alginate and an amine group on the polymyxin. Preferably the polymyxin-class antibiotic is a colistin (e.g. polymyxin E1 or E2) or a polymyxin B (e.g. polymyxin B1, B1-I or B2). The alginate oligomer will preferably contain 2 to 100 monomer residues. The alginate oligomer may also have at least 70% G residues.

In preferred embodiments the polymyxin-alginate oligomer conjugate consists of at least one alginate oligomer covalently bonded to a polymyxin-class antibiotic via an ester bond formed from a carboxyl group on the alginate and hydroxyl group on the polymyxin. Preferably the polymyxin-class antibiotic is a colistin (e.g. polymyxin E1 or E2) or a polymyxin B (e.g. polymyxin B1, B1-I or B2). The alginate oligomer will preferably contain 2 to 100 monomer residues. The alginate oligomer may also have at least 70% G residues.

Multivalent arrangements are contemplated in which more than one alginate oligomer is covalently linked to the polymyxin-class antibiotic. The alginate oligomers may be the same or different and may be linked to the polymyxin-class antibiotic via the same type of covalent bond or covalent molecular linker. In other arrangements an alginate oligomer may be covalently linked to a plurality of polymyxin molecules in the manner decribed herein. The polymyxin molecules may be the same or different and may or may not be covalently linked to other alginate oligomers.

References to the polymyxin-alginate oligomer conjugates of the invention extends to pharmaceutically acceptable salts, solvates or hydrates thereof, diastereoisomers, tautomers, enantiomers, and active metabolites thereof. Suitable salts include acid addition salts from inorganic acids such as hydrochloric, sulphuric, phosphoric, nitric, carbonic, boric, sulfamic, and hydrobromic acids, or salts of pharmaceutically acceptable organic acids such as acetic, propionic, butyric, tartaric, maleic, hydroxymaleic, fumeric,, citric, lactic, mucic, gluconic, benzoic, succinic, oxalic, phenylacetic, methanesulphonic, toluenesulphonic, benezenesulphonic, salicyclic, sulphanilic, aspartic, glutamic, edetic. stearic, palmitic, oleic, lauric, pantothenic, tannic, ascorbic, fendizoic, 4-4'-methylenebis-3-hydroxy-2-naphthoic acid, o-(p- hydroxybenzoyl)benzoic, 4-4'-dihydroxytriphenylmethane-2-carboxylic acid and valeric acids. Base salts include, but are not limited to, those formed with pharmaceutically acceptable cations, such as sodium, potassium, lithium, calcium, magnesium, ammonium and alkylammonium.

In a further aspect the invention provides a method for the preparation of a polymyxin-alginate oligomer conjugate of the invention, said method comprising
(ia) providing an alginate oligomer, wherein said alginate oligomer has 2-75 monomer residues, and a polymyxin-class antibiotic and forming a direct covalent bond between two molecular groups thereon; or
(ib) providing an alginate oligomer, wherein said alginate oligomer has 2-75 monomer residues, a polymyxin-class antibiotic and a covalent molecular linker and forming a direct covalent bond between two molecular groups on the alginate oligomer and the linker molecule and forming a direct covalent bond between two molecular groups on the polymyxin-class antibiotic and the linker molecule; or
(ic) providing an alginate oligomer, wherein said alginate oligomer has 2 - 75 monomer residues, and a polymyxin-class antibiotic wherein one or both carry a covalent molecular linker molecule covalently bonded thereto and covalently linking the alginate oligomer to the polymyxin-class antibiotic via at least one of the linker molecules; and optionally
(ii) separating at least a portion of the polymyxin-alginate oligomer conjugate from the reaction mixture.

In certain embodiments the invention provides a method for the preparation of a polymyxin-alginate oligomer conjugate of the invention, said method comprising
(i) providing an aqueous solution of an alginate oligomer having an available carboxyl group, wherein said alginate oligomer has 2 -75 monomer residues;
(ii) contacting said alginate solution with 1-ethyl-3-[3-dimethylaminopropyl]carbodiimide hydrochloride (EDC) in an amount and under conditions sufficient to activate at least one carboxyl group in the alginate oligomer;
(iii) optionally contacting said carboxyl activated alginate oligomer with sulfo N-hydroxysuccinimide (sulfo-NHS) in an amount and under conditions sufficient to form an amine-reactive sulfo-NHS ester;
(iv) contacting said carboxyl activated alginate oligomer of step (ii) or the amine-reactive sulfo-NHS ester of step (iii) with an polymyxin-class antibiotic having an available primary amine group in an amount and under conditions sufficient to form an amide bond between the alginate oligomer and the polymyxin-class antibiotic; and
(v) separating at least a portion of the polymyxin-alginate oligomer conjugate from the reaction mixture.

In certain embodiments the invention provides a method for the preparation of a polymyxin-alginate oligomer conjugate of the invention, said method comprising
(i) providing a solution of an alginate oligomer having an available carboxyl group, wherein said alginate oligomer has 2 -75 monomer residues, preferably an organic (e.g. DMF and/or DMSO) solution;
(ii) contacting said alginate solution with dicyclohexylcarbodiimide (DCC) in an amount and under conditions sufficient to form an O-acylisourea intermediate;
(iii) contacting said O-acylisourea intermediate with an polymyxin-class antibiotic having an available hydroxyl group and 4-N,N-dimethylaminopyridine (DMAP) in amounts and under conditions sufficient to form an ester bond between the alginate oligomer and the polymyxin-class antibiotic; and
(iv) separating at least a portion of the polymyxin-alginate oligomer conjugate from the reaction mixture;
wherein steps (ii) and (iii) may be performed simultaneously.

As mentioned above, polymyxin-alginate oligomer conjugates are a class of novel chemical entities having essentially the same or similar antibacterial efficacy as non-conjugated polymyxin-class antibiotics but less host toxicity and potentially a longer duration of antibacterial effects. Antibacterial efficacy may for example be assessed on the basis of minimal inhibitory concentration (MIC) values, and a polymyxin-alginate oligomer conjugate may be judged as having a similar antibacterial efficacy if the MIC value (e.g. as calculated based on the mass of the conjugate per se, or calculated based on the polymyxin content of the conjugate) is less than 2-fold higher compared to the unconjugated antibiotic. For example, the conjugate may have a MIC value which is 1-fold higher or less. Further, an increased MIC may be tolerated (e.g. 2-fold higher or greater), and may still result in a beneficial or useful product if the conjugate has substantially reduced toxicity as compared to the unconjugated antibiotic. It has also been found that certain polymyxin-alginate oligomer conjugates may be prepared which, in certain physiological models, e.g. of the circulatory system, have an early PK/PD profile which is similar to the non-conjugated form of the polymyxin-class antibiotic. This combination of properties make the conjugates of the invention advantageous in the treatment (particularly in the systemic treatment) of bacterial, especially Gram negative, infections compared both to polymyxin-class antibiotics per se but also previously proposed polymyxin modifications in which masking entities have been employed to lower the host toxicity. In particular the use of small dextrin molecules has been proposed as masking entities and while the host toxicity of such conjugates is significantly lower, the resulting conjugates display significantly lower antibiotic efficacy, which can be only partially-restored by removal of the dextrin groups. Moreover the early PK/PD profiles of dextrin-polymyxin conjugates do not resemble the corresponding profiles of the non-conjugated polymyxin in two compartment models of biological membrane diffusion and this may complicate their clinical use (Azzopardi, supra).

On the other hand, as shown in the Examples, polymyxin-alginate oligomer conjugates have essentially the same antibiotic efficacy as the unmodified antibiotics. A further surprising property of the, polymyxin-alginate oligomer conjugates of the invention is the duration of their antibiotic effects, which can be significantly longer than the unmodified form of the polymyxin-class antibiotic. In addition, the Examples show that the polymyxin-alginate oligomer conjugates of the invention may be more effective in combating (e.g. disrupting, reducing, limiting or eliminating) biofilm than the non-conjugated form of the same polymyxin-class. Moreover, it is possible to design polymyxin-alginate oligomer conjugates which, in certain physiological models, e.g. of the circulatory system, have an early PK/PD profile which is similar to the non-conjugated form of the polymyxin-class antibiotic.

Thus, in a further aspect the invention provides a pharmaceutical composition comprising a polymyxin-alginate oligomer conjugate as defined herein and a pharmaceutically acceptable excipient, carrier or diluent. Suitable excipients, carriers or diluents are described and specific pharmaceutical compositions are detailed below.

The invention further relates to the use of the polymyxin-alginate oligomer conjugates as described herein and the pharmaceutical compositions comprising the same in the combat of bacterial infection. The term "combat" as used herein includes both therapy and prophylaxis (i.e. the treatment or prevention of a bacterial infection).

Thus in this aspect the invention provides the polymyxin-alginate oligomer conjugates of the invention as described herein and pharmaceutical compositions comprising them for use in therapy, particularly for use in the treatment or prevention of a bacterial infection.

The invention further provides a polymyxin-alginate oligomer conjugate of the invention as defined herein, for use in the treatment or prevention of a bacterial infection in a subject with, suspected to have, or at risk of, a bacterial infection.

An "effective", more particularly a "pharmaceutically effective", amount of the polymyxin-alginate oligomer conjugate is that amount of conjugate that provides a measurable treatment or prevention of the target bacterial infection. In certain embodiments the antibacterial effects of the conjugate are longer (i.e. last or persist longer) than those of the unconjugated form of the polymyxin-class antibiotic and so less conjugate may be required, e.g. the effective amount of the conjugate is comparatively lower than that of the unconjugated form of the polymyxin-class antibiotic.

In particular, in such uses the effective amount of polymyxin-alginate oligomer conjugate causes at most manageable, preferably minor or negligible, or no appreciable or clinically relevant host toxicity in the subject. In any event the host toxicity is reduced as compared to the corresponding unconjugated polymyxin.

In the above described embodiments the primary physiological result to be achieved is the contact of the site of infection (in particular the bacteria which are present in the infected site or location, and which may include multiple sites or locations of infection in the body, including also a systemic infection) and/or a site (e.g. a surface) at which the infection may occur (or is at risk of occurring) with the polymyxin-alginate oligomer conjugate. The secondary physiological outcome from the administration of the conjugate of the invention and its subsequent contact of the site of invention is a reduced or limited degree of host toxicity in the subject undergoing treatment, as compared to the corresponding unconjugated polymyxin antibiotic, e.g. manageable, preferably minor or negligible, or no appreciable or clinically relevant host toxicity is encountered.

Working the invnetuin may involve the use of an a polymyxin-alginate oligomer conjugate of the invention as defined herein for the manufacture of a medicament for use in the treatment or prevention of a bacterial infection in a subject with, suspected to have, or at risk of, a bacterial infection.

The term "bacterial infection" (or "infected by" or "infected with" and the like) is used broadly herein to indicate that the subject may comprise, or contain, or carry, the bacteria in question, i.e. that the bacteria may simply be present in or on the subject, and this may include any site or location in or on the body of the subject. It is not necessary that the infection of the subject be manifest as a clinical disease (i.e. that the infection result in clinical symptoms in the subject), although this is of course encompassed. A subject who is suspected to be infected or who is at risk of infection may be a subject who has been exposed to the bacteria or to an infected subject, or a subject presenting with clinical signs or symptoms of infection (in the case of a suspected infection), or a subject who is susceptible to infection, whether generally (e.g. due to the clinical status of the subject) or particularly to the bacteria in question.

Also in accordance with certain aspects of the invention there may be a preceding step of identifying a subject as being a subject with, suspected to have, or at risk of, a bacterial infection, or a step of diagnosing a subject as a subject with, suspected to have, or at risk of, a bacterial infection. In particular, the bacterial infection may be of a type which is known to be treated, or treatable, in usual clinical practice with a polymyxin-class antibiotic. In one embodiment the bacteria are identified as or suspected to be bacteria which are responsive to (i.e. sensitive to) a polymyxin-class antibiotic. In certain embodiments the sensitivity of that infection (or more particularly the bacteria within the infection) to a polymyxin-class antibiotic may be determined.

Alternatively or in addition to the above described preceding step, in accordance with the invention there may be a following step in which the subject's clinical indicators of the bacterial infection are assessed and preferably compared to a corresponding assessment made prior to, or earlier in, said treatment in order to determine any changes therein.

The diagnosis and monitoring of bacterial infections based on readily observable physiological indicators is entirely routine for clinicians. Molecular biological and microbiological methods may also be used to more confirm diagnoses and to provide more information on the causative agents, e.g. taxonomic information, possible indications of virulence and their sensitivity to antibiotics.

Alternatively or in addition to the above described preceding and/or following steps, in accordance with the invention there may be a following step in which the subject's clinical indicators of polymyxin toxicity are assessed and preferably compared to a corresponding assessment made prior to, or earlier in, said treatment in order to determine any changes therein. The monitoring of polymyxin toxicity in a subject based on readily observable physiological indicators is entirely routine for clinicians. This may include assessment of the general condition of the subject or more specific molecular markers, e.g. inflammatory markers (e.g. circulating or localised cytokine levels).

The invention encompasses the use of a single polymyxin-alginate oligomer conjugate or a mixture (multiplicity/plurality; two or more) of different polymyxin-alginate oligomer conjugates. Such mixtures may comprise conjugates carrying different polymyxin-class antibiotics and the same alginate oligomer. Such mixtures may comprise conjugates carrying the same polymyxin-class antibiotic and different alginate oligomers. Such mixtures may comprise conjugates carrying different polymyxin-class antibiotics and different alginate oligomers.

The bacterial infection targeted according to the invention may comprise bacteria from any genera or species of bacteria. Examples of genera or species of bacteria include, but are not limited to, *Abiotrophia, Achromobacter, Acidaminococcus, Acidovorax, Acinetobacter, Actinobacillus, Actinobaculum, Actinomadura, Actinomyces, Aerococcus, Aeromonas, Afipia, Agrobacterium, Alcaligenes, Alloiococcus, Alteromonas, Amycolata, Amycolatopsis, Anaerobospirillum, Anaerorhabdus, Arachnia, Arcanobacterium, Arcobacter, Arthrobacter, Atopobium, Aureobacterium, Bacteroides, Balneatrix, Bartonella, Bergeyella, Bifidobacterium, Bilophila Branhamella, Borrelia, Bordetella, Brachyspira, Brevibacillus, Brevibacterium, Brevundimonas, Brucella, Burkholderia, Buttiauxella, Butyrivibrio, Calymmatobacterium, Campylobacter, Capnocytophaga, Cardiobacterium, Catonella, Cedecea, Cellulomonas, Centipeda, Chlamydia, Chlamydophila, Chromobacterium, Chyseobacterium, Chryseomonas, Citrobacter, Clostridium, Collinsella, Comamonas, Corynebacterium, Coxiella, Cryptobacterium, Delftia, Dermabacter, Dermatophilus, Desulfomonas, Desulfovibrio, Dialister, Dichelobacter, Dolosicoccus, Dolosigranulum, Edwardsiella, Eggerthella, Ehrlichia, Eikenella, Empedobacter, Enterobacter, Enterococcus, Erwinia, Erysipelothrix, Escherichia, Eubacterium, Ewingella, Exiguobacterium, Facklamia, Filifactor, Flavimonas, Flavobacterium, Francisella, Fusobacterium, Gardnerella, Globicatella, Gemella, Gordona, Haemophilus, Hafnia, Helicobacter, Helococcus, Holdemania, Ignavigranum, Johnsonella, Kingella, Klebsiella, Kocuria, Koserella, Kurthia, Kytococcus, Lactobacillus, Lactococcus, Lautropia, Leclercia, Legionella, Leminorella, Leptospira, Leptotrichia, Leuconostoc, Listeria, Listonella, Megasphaera, Methylobacterium, Microbacterium, Micrococcus, Mitsuokella, Mobiluncus, Moellerella, Moraxella, Morganella, Mycobacterium, Mycoplasma, Myroides, Neisseria, Nocardia, Nocardiopsis, Ochrobactrum, Oeskovia, Oligella, Orientia, Paenibacillus, Pantoea, Parachlamydia, Pasteurella, Pediococcus, Peptococcus, Peptostreptococcus, Photobacterium, Photorhabdus, Plesiomonas, Porphyrimonas, Prevotella, Propionibacterium, Proteus, Providencia, Pseudomonas, Pseudonocardia, Pseudoramibacter, Psychrobacter, Rahnella, Ralstonia, Rhodococcus, Rickettsia Rochalimaea Roseomonas, Rothia, Ruminococcus, Salmonella, Selenomonas, Serpulina, Serratia, Shewenella, Shigella, Simkania, Slackia, Sphingobacterium, Sphingomonas, Spirillum, Staphylococcus, Stenotrophomonas, Stomatococcus, Streptobacillus, Streptococcus, Streptomyces, Succinivibrio, Sutterella, Suttonella, Tatumella, Tissierella, Trabulsiella, Treponema, Tropheryma, Tsakamurella, Turicella, Ureaplasma, Vagococcus, Veillonella, Vibrio, Weeksella, Wolinella, Xanthomonas, Xenorhabdus, Yersinia, and Yokenella;* e.g. Gram-positive bacteria such as *Staphylococcus aureus, Staphylococcus epidermidis, Staphylococcus equi, Streptococcus pyogenes, Streptococcus agalactiae, Listeria monocytogenes, Listeria ivanovii, Bacillus anthracis, B. subtilis, Nocardia asteroides, Actinomyces israelii, Propionibacterium acnes, Clostridium tetani, Clostridium perfringens, Clostridium botulinum, and Enterococcus species,* Gram-negative bacteria such as *Pseudomonas aeruginosa, Vibrio cholerae, Actinobacillus pleuropneumoniae, Pasteurella haemolytica, Pasteurella multocida, Legionella pneumophila, Salmonella typhi, Brucella abortus, , Coxiella burnetti, Escherichia coli, Neiserria meningitidis, Neiserria gonorrhea, Haemophilus influenzae, Haemophilus ducreyi, Yersinia pestis, Yersinia enterolitica, Escherichia hirae, Burkholderia cepacia, Burkholderia mallei, Burkholderia pseudomallei, Francisella tularensis, Bacteroides fragilis, Fusobascterium nucleatum, Cowdria ruminantium, Moraxella catarrhalis, Klebsiella pneumoniae, Proteus mirabilis, Enterobacter cloacae, Serratia marcescens, Helicobacter pylori, Salmonella enteritidis, Salmonella typhi and Acinetobacter baumannii, Acinetobacter Iwoffi, Providencia stuartii, Providencia rettgeri, Providencia alcalifaciens and Klebsiella oxytoca,* Gram non-responsive bacteria such as *Chlamydia trachomatis, Chlamydia psittaci* and mycobacteria such as *M. tuberculosis, M. bovis, M. typhimurium, M. bovis strain BCG, BCG substrains, M. avium, M. intracellulare, M. africanum, M. kansasii, M. marinum, M. ulcerans, M. avium subspecies paratuberculosis,*

Preferably the bacterial infection targeted according to the invention comprises bacteria selected from the following genera: *Achromobacter, Acinetobacter, Actinobacillus, Aeromonas, Agrobacterium, Alcaligenes, Alteromonas, Bacteroides, Bartonella, Borrelia, Bordetella, Brucella, Burkholderia, Campylobacter, Cardiobacterium, Chlamydia, Chlamydophila, Chromobacterium, Chyseobacterium, Chryseomonas, Citrobacter, Clostridium, Comamonas, Corynebacterium, Coxiella, Cryptobacterium, Edwardsiella, Eikenella, Enterobacter, Enterococcus, Erwinia, Kingella, Klebsiella, Lactobacillus, Lactococcus, Legionella, Leptospira, Leptotrichia, Leuconostoc, Listeria, Listonella, Mobiluncus, Moraxella, Morganella, Mycobacterium, Mycoplasma, Neisseria, Nocardia, Nocardiopsis, Pantoea, Parachlamydia, Pasteurella, Peptococcus, Peptostreptococcus, Prevotella, Propionibacterium, Proteus, Providencia, Pseudomonas, Ralstonia, Rickettsia, Salmonella, Shewenella, Shigella, Sphingobacterium, Sphingomonas, Staphylococcus, Stenotrophomonas, Streptobacillus, Streptococcus, Streptomyces, Treponem and Yersinia*

Thus, the invention may be used against Gram positive or Gram negative bacteria, or indeed Gram-indeterminate bacteria. Gram-negative bacteria, for instance those particularised above, are of importance. Within the Gram-negative bacteria the *Enterobacteriaceae* and the Gram-negative bacteria non-fermenting bacteria are of particular note.

*Enterobacteriaceae* include, but are not limited to, bacteria from the genera *Alishewanella, Alterococcus, Aquamonas, Aranicola, Azotivirga, Brenneria, Budvicia, Buttiauxella, Cedecea, Citrobacter, Cronobacter, Dickeya, Edwardsiella, Enterobacter, Erwinia, Escherichia, Ewingella, Grimontella, Hafnia, Klebsiella, Kluyvera, Leclercia, Leminorella, Moellerella, Morganella, Obesumbacterium, Pantoea, Pectobacterium, Phlomobacter, Photorhabdus, Plesiomonas, Pragia, Proteus, Providencia, Rahnella, Raoultella, Salmonella, Samsonia, Serratia, Shigella, Sodalis, Tatumella, Trabulsiella, Wigglesworthia, Xenorhabdus, Yersinia, Yokenella.* Preferred genera of *Enterobacteriaceae* include *Escherichia, Klebsiella, Salmonella, Shigella, Yersinia* and *Providencia.*

Non-fermenting Gram-negative bacteria include, but are not limited to, bacteria from the genera *Pseudomonas, Acinetobacter, Stenotrophomonas and Burkholderia, Achromobacter, Algaligenes, Bordetella, Brevundimonas, Comamonas, Elizabethkingia* (formerly *Chryseobacterium*), *Methylobacterium, Moraxella, Ochrobactrum, Oligella, Psychrobacter, Ralstonia, Roseomonas, Shewanella, Sphingobacterium,* e.g. *Pseudomonas aeruginosa, Acinetobacter baumannii, Stenotrophomonas maltophilia,* and *Burkholderia spp..*

Preferably the bacteria may be selected from the genera *Pseudomonas, Acinetobacter, Stenotrophomonas, Burkholderia, Escherichia, Klebsiella, Providencia, Streptococcus, Staphylococcus*, e.g. *Pseudomonas aeruginosa, Acinetobacter baumannii, Stenotrophomonas maltophilia, Burkholderia spp, E. coli, Klebsiella pneumoniae and Burkholderia cepacia, Burkholderia mallei, Burkholderia pseudomallei, Acinetobacter Iwoffi, Providencia stuartii, Providencia rettgeri, Providencia alcalifaciens, Klebsiella oxytoca, Pseudomonas anguilliseptica, Pseudomonas oryzihabitans, Pseudomonas plecoglossicida, Pseudomonas luteola, and MRSA.*

In certain aspects, the infection is a nosocomial infection, an infection in the respiratory tract of patients, e.g. in patients suffering from cystic fibrosis, chronic obstructive pulmonary disease, congestive obstructive airway disease / congestive obstructive airway pneumonia (COAD/COAP), pneumonia, emphysema, bronchitis or sinusitis; an infection in a wound, particularly a chronic wound (including burns), a device related infection associated with implantable or prosthetic medical devices e.g. prosthetic valve endocarditis or an infection of a line or a catheter or an artificial joints or a tissue replacements or an endotracheal or tracheotomy tube. Examples of the types of bacteria which commonly cause such infections include *Pseudomonas aeruginosa, Acinetobacter baumannii, Stenotrophomonas maltophilia, Burkholderia spp (e.g. B. cepacia), E. coli, Klebsiella pneumoniae, Staphylococcus aureus*, Methicillin Resistant *Staphylococcus aureus* (MRSA), *Clostridium difficile, Mycobacterium tuberculosis, Enterococcus and Vancomycin-Resistant Enterococcus* and *Providencia stuartii.* Other infections of importance in accordance with the invention include infections by Bartonella (e.g. *Bartonella hensela),* mycobacteria *(e.g. Mycobacterium avium* Complex (MAC), *M. kansasii*, *M. marinum, M. ulcerans, M. xenopi),* Haemophilus influenzae type b (Hib) or Legionella *(e.g. Legionella pneumophila;* Legionnaire's disease), leprosy (*M. leprae*), human granulocytic anaplasmosis (*Anaplasma phagocytophilum),* brucellosis (*Brucella melitensis),* meningococcal disease (*Neisseria meningitides*) and anthrax *(Bacillus anthracis).*

The bacteria may be multidrug resistant, e.g. bacteria which are resistant to antibiotics from at least 3, or at least 4, 5, 6, 7, 8, 9 or 10 antibiotic classes, e.g. the aminoglycosides (e.g. amikacin, gentamicin, kanamycin, capreomycin, neomycin, netilmicin, streptomycin, tobramycin); the β-lactams (e.g. the carbecephems (e.g. loracarbef); the 1st generation cephalosporins (e.g. cefadroxil, cefazolin, cephalexin); 2nd generation cephalosporins (e.g. cefaclor, cefamandole, cephalexin, cefoxitin, cefprozil, cefuroxime); 3rd generation cephalosporins (e.g. cefixime, cefdinir, cefditoren, cefoperazone, cefotaxime, cefpodoxime, ceftazidime, ceftibuten, ceftizoxime, ceftriaxone); 4th generation cephalosporins (e.g. cefepime); the monobactams (e.g. aztreonam)); the macrolides (e.g. azithromycin, clarithromycin, dirithromycin, erythromycin, troleandomycin); the monobactams (e.g. aztreonam); the penicillins (e.g. amoxicillin, ampicillin, carbenicillin, cloxacillin, dicloxacillin, nafcillin, oxacillin, penicillin G, penicillin V, piperacillin, ticarcillin); the polypeptide antibiotics (e.g. bacitracin, colistin and polymyxin B, but in certain embodiments not the polymyxin of the polymyxin-alginate oligomer conjugate to be used); the quinolones (e.g. ciprofloxacin, enoxacin, gatifloxacin, levofloxacin, lomefloxacin, moxifloxacin, norfloxacin, ofloxacin, trovafloxacin); the sulfonamides (e.g. mafenide, sulfacetamide, sulfamethizole, sulfasalazine, sulfisoxazole, trimethoprim- sulfamethoxazole); the tetracyclines (e.g. demeclocycline, doxycycline, minocycline, oxytetracycline, tetracycline); the glycylcyclines (e.g. tigecycline); the carbapenems (e.g. imipenem, meropenem, ertapenem, doripenem, panipenem/betamipron, biapenem, PZ-601); and other antibiotics including chloramphenicol; clindamycin, ethambutol; fosfomycin; isoniazid; linezolid; metronidazole; nitrofurantoin; pyrazinamide; quinupristin/dalfopristin; spectinomycin; fosfomycin and vancomycin.

Susceptibility, that is sensitivity, (and conversely resistance and tolerance) to an antibiotic can be measured in any convenient way, e.g. with dilution susceptibility tests and/or disk diffusion tests. Preferably the susceptibility of a bacterial strain to an antibiotic is expressed in terms of the Minimum Inhibitory Concentration (MIC) of that antibiotic for that microorganism (Jorgensen et al., Manual of Clinical Microbiology, 7th ed. Washington, D.C: American Society for Microbiology, 1999; 1526-43), i.e. that concentration of antibiotic that completely inhibits growth of that bacterial strain.

The skilled man would appreciate that the extent of the difference in tolerance/susceptibility sufficient to constitute resistance will vary depending on the antibiotic and bacterial strain under test and the test used. Many regulatory bodies, e.g. the European Committee on Antimicrobial Susceptibility Testing (EUCAST) set so called "breakpoints" for specific antimicrobials and microorganisms which are discriminatory antimicrobial concentrations used in the interpretation of results of susceptibility testing to define isolates as susceptible, intermediate or resistant to the antimicrobial agent under test. The skilled person can utilise such information to ascertain whether or not the bacterial infection being treated by the invention is resistant to the antibiotic in question under these definitions.

As shown in the Examples, the polymyxin-alginate oligomer conjugates of the invention may be more effective in combating (e.g. disrupting, reducing, limiting or eliminating) biofilm than the non-conjugated form of the same polymyxin-class antibiotic. In certain embodiments therefore the target infection will be bacteria in a biofilm. However, in other embodiments the bacterium will not be in a biofilm.(e.g. will be growing planktonically). Put differently, the bacterium will be, or will not be, in a biofilm mode of growth; or will be, or will not be, in a non-biofilm mode of growth.

By "biofilm" it is meant a community of microorganisms characterized by a predominance of sessile cells that are attached to a substratum or interface or to each other (some motile cells may also be present) and that are embedded in a matrix of extracellular polymers (more specifically extracellular polymers that they have produced) characterised in that the microorganisms of this colony exhibit an altered phenotype with respect to growth rate and gene transcription (for example as compared to their "non-biofilm" or free-floating or planktonic counterparts). By "in a biofilm" it is meant that the bacterium targeted by the method of the invention is within (completely or in part), on or associated with the polymer matrix of a biofilm. Viewed differently, bacteria that are "not in a biofilm" are organisms that are either in isolation, e.g. planktonic, or if in an aggregation of a plurality of organisms, that aggregation is unorganised and/or is devoid of the matrix characteristic of a biofilm. In each case, the individual bacteria do not exhibit an altered phenotype that is observed in their biofilm dwelling counterparts.

In certain embodiments the bacterial infection does not contain bacteria from the genus Acinetobacter. In certain embodiments the bacterial infection to be treated or prevented in accordance with the invention does not contain bacteria which are multidrug resistant.

In particular embodiments the invention may provide for the treatment or prevention of respiratory infections or conditions associated therewith (e.g. cystic fibrosis, pneumonia, COPD, COAD, COAP, bronchitis, sinusitis, an infection in a chronic wound (including burns), a device related infection associated with implantable or prosthetic medical devices, bacteraemia, septicaemia, septic shock, or sepsis.

In one preferred embodiment the bacterial infection is a respiratory infection in a subject suffering from an underlying respiratory disorder or condition, including notably CF, COPD/COAD, or asthma.

"Treatment" when used in relation to the treatment of a bacterial infection/medical condition in a subject in accordance with the invention is used broadly herein to include any therapeutic effect, i.e. any beneficial effect in relation to the infection or on the condition. Thus, not only included is eradication or elimination of the infection, or cure of the subject or infection, but also an improvement in the infection or condition of the subject. Thus included for example, is an improvement in any symptom or sign of the infection or condition, or in any clinically accepted indicator of the infection/condition (for example a decrease in wound size or an acceleration of healing time). Treatment thus includes both curative and palliative therapy, e.g. of a pre-existing or diagnosed infection/condition, i.e. a reactionary treatment.

"Prevention" as used herein refers to any prophylactic or preventative effect. It thus includes delaying, limiting, reducing or preventing the infection/condition or the onset of the infection/condition, or one or more symptoms or indications thereof, for example relative to the infection/condition or symptom or indication prior to the prophylactic treatment. Prophylaxis thus explicitly includes both absolute prevention of occurrence or development of the infection/condition, or symptom or indication thereof, and any delay in the onset or development of the infection/condition or symptom or indication thereof, or reduction or limitation of the development or progression of the infection/condition or symptom or indication thereof.

The subject may be any human or non-human animal subject, but more particularly may be a human or a non-human vertebrate, e.g. a non-human mammal, bird, amphibian, fish or reptile. In a preferred embodiment the subject is a mammalian subject. The animal may be a livestock or a domestic animal or an animal of commercial value, including laboratory animals or an animal in a zoo or game park. Representative animals therefore include dogs, cats, rabbits, mice, guinea pigs, hamsters, horses, pigs, sheep, goats and cows. Veterinary uses of the invention are thus covered. The subject may be viewed as a patient. Preferably the subject is a human. In some embodiments the subject is not a ruminant mammal.

The term "in a subject" is used broadly herein to include sites or locations inside a subject or on a subject, e.g. an external body surface, and may include in particular infection of a medical device e.g. an implanted or "in-dwelling" medical device. The term "in a patient" should be interpreted consistently with this.

The location of the infection may therefore be a surface in the oral cavity (e.g. teeth, gingiva, gingival crevice, periodontal pocket), the reproductive tract (e.g. cervix, uterus, fallopian tubes), the peritoneum, middle ear, prostate, the urinary tract, vascular intima, the eye, i.e. ocular tissue (e.g. the conjunctiva, corneal tissue, lachrymal duct, lachrymal gland, eyelid) the respiratory tract, lung tissue (e.g. bronchial and alveolial), heart valves, the gastrointestinal tract, skin, scalp, nails and the interior of wounds, particularly chronic wounds and surgical wounds, which may be topical or internal wounds. Other surfaces include the exterior of organs, particularly those undergoing transplantation, for example, heart, lungs, kidney, liver, heart valve, pancreas, intestine, corneal tissue, arterial and venous grafts and skin.

The infection may therefore also be present in body fluids (e.g. blood, plasma, serum, cerebrospinal fluid, GI tract contents, semen, sputum and other pulmonary secretions) and tissues (e.g. adrenal, hepatic, renal, pancreatic, pituitary, thyroid, immune, ovarian, testicular, prostate, endometrial, ocular, mammary, adipose, epithelial, endothelial, neural, muscle, pulmonary, epidermis, osseous).

The infection may further be found on any "in-dwelling" medical or surgical equipment or devices. This may include any kind of line, including catheters (e.g. central venous and urinary catheters), prosthetic devices e.g., heart valves, artificial joints, false teeth, dental crowns, dental caps and soft tissue implants (e.g. breast, buttock and lip implants). Any kind of implantable medical device is included (e.g. stents, intrauterine devices, pacemakers, intubation tubes (e.g. endotracheal or tracheostomy tubes), prostheses or prosthetic devices, lines or catheters). An "in-dwelling" medical device may include a device in which any part of it is contained within the body, i.e. the device may be wholly or partly in-dwelling.

The infection may be acute, or alternatively chronic, e.g. an infection that has persisted for at least 5 or at least 10 days, particularly at least 20 days, more particularly at least 30 days, most particularly at least 40 days.

A bacterial infection can occur in any subject but some subjects will be more susceptible to infection that others. Subjects who are susceptible to bacterial infection include, but are not limited to, subjects whose epithelial and/or endothelial barrier is weakened or compromised, subjects whose secretion-based defences to microbial infection have been abrogated, disrupted, weakened or undermined, and subjects who are immunocompromised, immunodeficient or immunosuppressed (i.e. a subject in whom any part of the immune system is not working normally, or is working sub-normally, in other words in whom any part of the immune response, or an immune activity is reduced or impaired, whether due to disease or clinical intervention or other treatment, or in any way).

Representative examples of subjects who are susceptible to bacterial infection include, but are not limited to, subjects with a pre-established infection (e.g. with bacteria, viruses, fungi or parasites such as protozoa), especially subjects with HIV, subjects with bacteraemia, sepsis and subjects with septic shock; subjects with immunodeficiency, e.g. subjects preparing for, undergoing or recovering from chemotherapy and/or radiotherapy, organ (e.g. bone marrow, liver, lung, heart, heart valve, kidney, etc.) transplant subjects (including autograft, allograft and xenograft patients); subjects with AIDS; subjects resident in a healthcare institution, e.g. hospital, especially subjects in intensive care or critical care (i.e. those units concerned with the provision of life support or organ support systems to patients); subjects on respiratory ventilators; subjects suffering from trauma; subjects with burns, subjects with acute and/or chronic wounds; neonatal subjects; elderly subjects; subjects with cancer (defined broadly herein to include any neoplastic condition; malignant or non-malignant), especially those with cancers of the immune system (e.g. leukaemias, lymphomas and other haematological cancers); subjects suffering from auto-immune conditions such as rheumatoid arthritis, diabetes mellitus type I, Crohn's disease, especially those undergoing immunosuppression treatment for those diseases; subjects with reduced or abrogated epithelial or endothelial secretion (e.g. mucous, tears, saliva) and/or secretion clearance (e.g. subjects with poorly functioning cilia on mucosal tissue and/or patients with hyperviscous mucous (e.g. smokers and subjects with COPD, COAD, COAP, bronchitis, cystic fibrosis, emphysema, lung cancer, asthma, pneumonia or sinusitis)) and subjects fitted with a medical device.

The polymyxin-alginate oligomer conjugates of the invention may be administered to the subject in any convenient form or by any convenient means in order to deliver effective amounts to the bacteria of the target infection and/or to the site carrying the invention or the site at risk of infection, e.g. by parenteral (e.g. intravenous, intraspinal, intramuscular, subcutaneous), topical, enteral (e.g. oral, buccal, sublingual, rectal), or by inhalation (including nasal inhalation). Administration may achieve systemic distribution or localised distribution, by which it is meant that delivery is effected to the bacteria of the target infection and/or to the site carrying the infection or to the site at risk of infection, but essentially no other location in the patient. The skilled person would be able to select an appropriate administration means to suit any particular target infection and/or site carrying the infection or site at risk of infection.

The comparatively low host toxicity of the polymyxin-alginate oligomer conjugates of the invention make these entities suitable for systemic use in subjects, that is systemic administration to treat a systemic infection and systemic administration to treat a localised infections, e.g. an infection in the lungs or a wound. In contrast the use of polymyxin-class antibiotics is typically restricted to topical or at least closely localised treatments because of the associated host toxicity of these antibiotics. Systemic use of polymyxin-class antibiotics is restricted to the most severe and grave cases of life threatening infection by multidrug resistant bacteria because in these scenarios the problems caused by host toxicity are outweighed by the immediate risk to the subject's life posed by the infection. The finding that certain polymyxin-alginate oligomer conjugates, e.g. those with an ester bond linker, may have early PK/PD profiles in certain physiological models, e.g. those which mimic the circulatory system, which are similar to the non-conjugated form of the polymyxin-class antibiotic, may indicate that such polymyxin-alginate oligomer conjugates are particularly suited to systemic use.

Thus in certain embodiments of the invention the polymyxin-alginate oligomer conjugate of the invention as defined herein is provided for use in a method for the treatment or prevention of a bacterial infection in a subject with, suspected to have, or at risk of, a bacterial infection, said method comprising systemically administering to said subject an effective amount of a polymyxin-alginate oligomer conjugate of the invention as defined herein. In certain embodiments the bacterial infection is a systemic bacterial infection e.g. sepsis (septicaemia) or an infection involving multiple loci in a subject.

The skilled man will be able to formulate the polymyxin-alginate oligomer conjugates of the invention into pharmaceutical compositions that are adapted for these routes of administration and body distribution according to any of the conventional methods known in the art and widely described in the literature.

More specifically, the polymyxin-alginate oligomer conjugates of the invention may be incorporated, optionally together with other active agents, with one or more conventional carriers, diluents and/or excipients, to produce conventional galenic preparations such as tablets, pills, granules (e.g. in free form or enclosed in capsules), powders (e.g. inhalable powders, including dry inhalable powders), lozenges, sachets, cachets, elixirs, suspensions, emulsions, solutions, syrups, aerosols (as a solid or in a liquid medium), sprays (e.g. nasal sprays), compositions for use in nebulisers, ointments, creams, salves, soft and hard gelatine capsules, suppositories, pessaries, sterile injectable solutions, sterile packaged powders, and the like. Enteric coated solid or liquid compositions, e.g. enteric coated tablets and enteric coated granules (which may be provided in an enteric-coated capsule or in a non-enteric-coated capsule i.e. in which the coating may or may not be an enteric coating); sterile inhalable and sterile injectable compositions are of particular note.

Examples of suitable carriers, excipients, and diluents are lactose, dextrose, sucrose, sorbitol, mannitol, starches, gum acacia, calcium phosphate, inert alginate polymers, tragacanth, gelatine, calcium silicate, microcrystalline cellulose, polyvinylpyrrolidone, cellulose, water syrup, water, water/ethanol, water/ glycol, water/polyethylene, hypertonic salt water, glycol, propylene glycol, methyl cellulose, methylhydroxybenzoates, propyl hydroxybenzoates, talc, magnesium stearate, mineral oil or fatty substances such as hard fat or suitable mixtures thereof. Excipients and diluents of note are mannitol and hypertonic salt water (saline).

The compositions may additionally include lubricating agents, wetting agents, emulsifying agents, suspending agents, preserving agents, sweetening agents, flavouring agents, and the like.

Parenterally administrable forms, e.g. solutions suitable for delivery intravenously, should be sterile and free from physiologically unacceptable agents, and should have low osmolarity to minimize irritation or other adverse effects upon administration and thus solutions should preferably be isotonic or slightly hypertonic, e.g. hypertonic salt water (saline). Suitable vehicles include aqueous vehicles customarily used for administering parenteral solutions such as sterile water for injection, Sodium Chloride Injection, Ringer's Injection, Dextrose Injection, Dextrose and Sodium Chloride Injection, Lactated Ringer's Injection and other solutions such as are described in Remington's Pharmaceutical Sciences, 15th ed., Easton: Mack Publishing Co., pp. 1405-1412 and 1461-1487 (1975) and The National Formulary XIV, 14th ed. Washington: American Pharmaceutical Association (1975)). The solutions can contain preservatives, antimicrobial agents, buffers and antioxidants conventionally used for parenteral solutions, excipients and other additives which are compatible with the polymyxin-alginate oligomer conjugates and which will not interfere with the manufacture, storage or use of products.

Simple sterile solutions of polymyxin-alginate oligomer conjugates or simple sterile liquid compositions comprising polymyxin-alginate oligomer conjugates may be especially convenient for use during surgical procedures and for delivery to the lungs, e.g. by nebuliser, or to the paranasal sinuses, e.g. by a nasal spray device.

Solid or liquid formulations of the polymyxin-alginate oligomer conjugates may be provided with an enteric coating that prevents degradation in the stomach and/or other parts of the upper GI tract but permits degradation in the lower GI tract, e.g. the small intestine. Such coatings are routinely prepared from polymers including fatty acids, waxes, shellac, plastics, and plant fibres. Specific examples thereof include but are not limited to methyl acrylate-methacrylic acid copolymers, methyl methacrylate-methacrylic acid copolymers, cellulose acetate succinate, hydroxypropyl methylcellulose phthalate, hydroxypropyl methylcellulose acetate succinate (hypromellose acetate succinate), polyvinyl acetate phthalate (PVAP), cellulose acetate trimellitate, and sodium alginate polymer. Enteric coated tablets and enteric coated granules (which may be provided in an enteric-coated capsule or in a non-enteric coated capsule) are of particular note. Enteric coated granules may be prepared in accordance with the teachings of WO 1989008448 and Al-Khedairy, E.B.H, 2006, Iraqi J.Pharm.Sci., Vol.15 (1) 49, although the skilled person would be aware of further alternative techniques which may be used.

For topical administration the polymyxin-alginate oligomer conjugates can be incorporated into creams, ointments, gels, salves, transdermal patches and the like. Further topical systems that are envisaged to be suitable are in situ drug delivery systems, for example gels where solid, semi-solid, amorphous or liquid crystalline gel matrices are formed in situ and which may comprise the alginate oligomer (which may be any alginate oligomer as herein defined). Such matrices can conveniently be designed to control the release of the polymyxin-alginate oligomer conjugates from the matrix, e.g. release can be delayed and/or sustained over a chosen period of time. Such systems may form gels only upon contact with biological tissues or fluids, e.g. mucosal surfaces. Typically the gels are bioadhesive and/or mucoadhesive. Delivery to any body site that can retain or be adapted to retain the pre-gel composition can be targeted by such a delivery technique. Such systems are described in WO 2005/023176.

The relative content of the polymyxin-alginate oligomer conjugates in the compositions of the invention can vary depending on the dosage required and the dosage regime being followed but will be sufficient to achieve an effective amount at the target treatment site, i.e. the bacteria of the target infection and/or the site carrying the infection, or the site at risk of infection, taking account of variables such as the physical size of the subject to be treated, the nature of the subject's particular ailments, and the location and identity of the target treatment area. The skilled man would know that the amounts of the polymyxin-alginate oligomer conjugates can be reduced if a multiple dosing regime is followed or increased to minimise the number of administrations or applications.

A representative aqueous solution for delivery of the polymyxin-alginate oligomer conjugate of the invention by injection (e.g. by intravenous, intraspinal, intramuscular or subcutaneous injection) will be sterile and may contain 6 to 25%, e.g. 6 to 20%, 6 to 15%, 6 to 10%, 8 to 25%, 8 to 20%, 8 to 15 %, 9 to 25%, 9 to 20%, 9 to 15 %, 10 to 15%, 10 to 20%, 10 to 25%, 15 to 20%, or 15 to 25% w/v of the polymyxin-alginate oligomer conjugate, the remainder being comprised of water and pharmaceutically acceptable excipients and/or other active agents if being used.

For administration to the nose or paranasal sinuses a sterile aqueous and/or oil-based liquid formulation (e.g. an emulsion) may be used; administered for instance by a nasal spray device, e.g. propellant-free or propellant-assisted. A representative formulation may contain 1 to 25%, 1 to 20%, e.g. 1 to 15%, 1 to 10%, 1 to 9%, 1 to 8%, 1 to 7% or 1 to 6%, 5 to 25%, 5 to 20%, 5 to 15%, 5 to 10%, 5 to 9%, 5 to 8%, 5 to 7%, 5 to 6%, 8 to 25%, 8 to 20%, 8 to 15%, 8 to 10%, 9 to 25%, 9 to 20%, or 9 to 15% w/v or w/w of the polymyxin-alginate oligomer conjugate, the remainder being comprised of pharmaceutically acceptable excipients, e.g. water, and/or other active agents if being used.

A representative inhalable solution to be used to administer a polymyxin-alginate oligomer conjugate of the invention to the upper respiratory tract typically will be sterile and may contain 6 to 25%, e.g. 6 to 20%, 6 to 15%, 6 to 10%, 8 to 25%, 8 to 20%, 8 to 15 %, 9 to 25%, 9 to 20%, 9 to 15 %, 10 to 15%, 10 to 20%, 10 to 25%, 15 to 20% or 15 to 25% w/v of the polymyxin-alginate oligomer conjugate, the remainder being comprised of pharmaceutically acceptable excipients, e.g. water, and/or other active agents if being used.

A representative inhalable powder to be used to administer a polymyxin-alginate oligomer conjugates of the invention to the lower respiratory tract may contain up to 90%, e.g. up to 85%, 80%, 75% or 70%, e.g. 50 to 90%, 55 to 90%, 60 to 90%, 65 to 90%, 70 to 90%, 75 to 90%, 80 to 90%, 85 to 90%, 50 to 85%, 55 to 85%, 60 to 85%, 65 to 85%, 70 to 85%, 75 to 85%, 80 to 85%, 50 to 80%, 55 to 80%, 60 to 80%, 65 to 80%, 70 to 80%, 75 to 80%, 50 to 70%, 55 to 70%, 60 to 70%, or 65 to 70% w/v or w/w of the polymyxin-alginate oligomer conjugate, the remainder being comprised of pharmaceutically acceptable excipients and/or other active agents if being used in the same composition.

In other embodiments a slow, delayed or sustained release formulations may be used for delivery, e.g. to the nose or paranasal sinuses. A representative formulation may be a powder containing the polymyxin-alginate oligomer conjugate or a suspension of said powder, said powder containing up to 90%, e.g. up to 85%, 80%, 75% or 70%, e.g. 50 to 90%, 55 to 90%, 60 to 90%, 65 to 90%, 70 to 90%, 75 to 90%, 80 to 90%, 85 to 90%, 50 to 85%, 55 to 85%, 60 to 85%, 65 to 85%, 70 to 85%, 75 to 85%, 80 to 85%, 50 to 80%, 55 to 80%, 60 to 80%, 65 to 80%, 70 to 80%, 75 to 80%, 50 to 70%, 55 to 70%, 60 to 70%, or 65 to 70% w/v or w/w of the polymyxin-alginate oligomer conjugate, the remainder being comprised of pharmaceutically acceptable excipients and/or other active agents if being used. The powder may comprise a coating that controls release of the polymyxin-alginate oligomer conjugate.

A representative topical formulation, e.g. a cream, ointment or salve, which may be used to administer a polymyxin-alginate oligomer conjugate of the invention to the skin or cervix or other parts of the lower female reproductive system might contain 1 to 25%, 1 to 20%, 1 to 15%, 1 to 10%, 1 to 9%, 1 to 8%, 1 to 7%, 1 to 6%, 5 to 25%, 5 to 20%, 5 to 15%, 5 to 10%, 5 to 9%, 5 to 8%, 5 to 7%, 5 to 6%, 8 to 25%, 8 to 20%, 8 to 15%, 8 to 10%, 9 to 25%, 9 to 20%, or 9 to 15% w/v of the polymyxin-alginate oligomer conjugate, the remainder being comprised of pharmaceutically acceptable excipients, and/or other active agents if being used. Delivery devices designed for the application of topical formulations to the female reproductive system are known and may be employed to deliver the above mentioned formulations if convenient.

A representative tablet to be used to administer a polymyxin-alginate oligomer conjugate of the invention to the lower GI tract may contain up to 99%, up to 95%, 90%, 85% or 80%, e.g. 50 to 95%, 55 to 95%, 60 to 95%, 65 to 95%, 70 to 95%, 75 to 95%, 80 to 95%, 85 to 95%, 90 to 95%, 50 to 90%, 50 to 90%, 55 to 90%, 60 to 90%, 65 to 90%, 70 to 90%, 75 to 90%, 80 to 90%, 85 to 90%, 50 to 90%, 55 to 85%, 60 to 80% or, 65 to 75% w/v or w/w of the polymyxin-alginate oligomer conjugate, the remainder being comprised of pharmaceutically acceptable excipients and/or other active agents if being used. The tablet may be a multi-layered tablet.

An enteric coated tablet may also be effective in administering a polymyxin-alginate oligomer conjugate of the invention to the lower GI tract. A representative enteric coated tablet may contain up to 95%, e.g. up to 90%, 85% or 80%, e.g. 55 to 90%, 60 to 90%, 65 to 90%, 70 to 90%, 75 to 90%, 80 to 90%, 85 to 90%, 55 to 85%, 60 to 85%, 65 to 85%, 70 to 85%, 75 to 85%, 80 to 85%, 50 to 80%, 55 to 80%, 60 to 80%, 65 to 80%, 70 to 80%, or 75 to 80% w/v or w/w of the polymyxin-alginate oligomer conjugate, the remainder being comprised of pharmaceutically acceptable excipients, including the enteric coating (e.g. polymers including fatty acids, waxes, shellac, plastics, and plant fibres) and/or other active agents if being used. The tablet may be a multi-layered tablet, e.g. as described above.

Enteric coated granules may also be effective in administering a polymyxin-alginate oligomer conjugate of the invention to the lower GI tract. Such granules may be provided in a capsule which itself may or may not be provided with an enteric coating. A representative enteric coated granule may contain up to 95%, e.g. up to 90%, 85% or 80%, e.g. 55 to 90%, 60 to 90%, 65 to 90%, 70 to 90%, 75 to 90%, 80 to 90%, 85 to 90%, 55 to 85%, 60 to 85%, 65 to 85%, 70 to 85%, 75 to 85%, 80 to 85%, 50 to 80%, 55 to 80%, 60 to 80%, 65 to 80%, 70 to 80%, or 75 to 80% w/v or w/w of the polymyxin-alginate oligomer conjugate, the remainder being comprised of pharmaceutically acceptable excipients, including the enteric coating (e.g. polymers including fatty acids, waxes, shellac, plastics, and plant fibres) and/or other active agents if being used.

A pessary may be used to administer a polymyxin-alginate oligomer conjugate of the invention to the lower parts of the female reproductive tract. A representative formulation may contain 1 to 25%, 1 to 20%, e.g. 1 to 15%, 1 to 10%, 1 to 9%, 1 to 8%, 1 to 7%, 1 to 6%, 5 to 25%, 5 to 20%, 5 to 15%, 5 to 10%, 5 to 9%, 5 to 8%, 5 to 7%, 5 to 6%, 8 to 25%, 8 to 20%, 8 to 15%, 8 to 10%, 9 to 25%, 9 to 20%, or 9 to 15% w/v or w/w of the polymyxin-alginate oligomer conjugate, the remainder being comprised of pharmaceutically acceptable excipients, including solid excipients, and/or other active agents if being used. Rectal suppositories may be formulated similarly.

The polymyxin-alginate oligomer conjugates may be used at a daily dose of 0.1g to 10g, e.g. 0.5g to 5g, 0.8g to 3g, 1g to 2g, e.g. about 2g, which may be administered at one or more times per day (e.g. bis daily) and in one or more dosage forms or administration events (e.g. two tablets bis daily).

The polymyxin-alginate oligomer conjugates as defined herein may be used in conjunction or combination with one or more further therapeutically active agents, which may include other anti-microbial (e.g. antibacterial, antibiotic, antifungal and antiviral) agents, immunostimulatory agents, corticosteroids, non-steroidal antiinflammatory drugs (NSAIDs), bronchodilators, mucus viscosity-reducing agents (i.e. an agent which reduces the viscosity of mucus and which terms are used interchangeably with the term "mucolytic agent") or CFTR modulators (also known as "CFTR modifiers").

The further agent, e.g. antibiotic, may be unconjugated to an alginate oligomer or to any other conjugating moiety.

The polymyxin-alginate oligomer conjugate and the further therapeutically active agent may, for example, be administered together, in a single pharmaceutical formulation or composition, or separately (i.e. separate, sequential or simultaneous administration). Thus, polymyxin-alginate oligomer conjugate and the further therapeutically active agent may be combined, e.g. in a pharmaceutical kit or as a combined ("combination") product.

Thus a further aspect of the invention provides a product (e.g. a pharmaceutical combination or a kit) comprising a polymyxin-alginate oligomer conjugate as defined herein together with a further therapeutically active agent (e.g. those described above) as combined preparation for separate, sequential or simultaneous use in treating or preventing a bacterial infection in a subject.

More generally this aspect of the invention also provides a kit comprising a polymyxin-alginate oligomer conjugate as defined herein together with a further therapeutically active agent (e.g. those described above).

Combinations comprising a polymyxin-alginate oligomer conjugate as herein defined and an antibiotic, an antifungal, a CFTR modulator and/or a mucus viscosity reducing agent are especially preferred. Such pharmaceutical products and pharmaceutical compositions are preferably adapted for use in the medical methods of the invention.

The further therapeutically active agent may conveniently be applied before, simultaneously with or following the polymyxin-alginate oligomer conjugate. Conveniently the further therapeutically active agent is applied at substantially the same time as the polymyxin-alginate oligomer conjugate or afterwards. In other embodiments the further therapeutically active agent may conveniently be applied or administered before the polymyxin-alginate oligomer conjugate. The further therapeutically active agent can also be given (e.g. administered or delivered) repeatedly at time points appropriate for the agent used. The skilled person is able to devise a suitable dosage regimen. In long term treatments the polymyxin-alginate oligomer conjugate can also be used repeatedly. The polymyxin-alginate oligomer conjugate can be applied as frequently as the further therapeutically active agent, or more or less frequently. The frequency required may depend on the site or location in or on the patient to which the polymyxin-alginate oligomer conjugate is administered and also the overall nature of the clinical condition displayed by the particular patient undergoing treatment.

The polymyxin-alginate oligomer conjugate and the further therapeutically active agent may therefore be formulated together or separately, that is in the same or in different formulations or pharmaceutical compositions, and may be provided for administration by the same or different routes. The use of polymyxin-alginate oligomer conjugate as herein defined to manufacture such pharmaceutical products and pharmaceutical compositions for use in the medical methods of the invention is also contemplated.

Representative antibiotics which may be used in conjunction or combination with the polymyxin-alginate oligomer conjugates as defined herein include, but are not limited to, the aminoglycosides (e.g. amikacin, gentamicin, kanamycin, neomycin, netilmicin, streptomycin, tobramycin); the β-lactams (e.g. the carbecephems (e.g. loracarbef); the 1st generation cephalosporins (e.g. cefadroxil, cefazolin, cephalexin); 2nd generation cephalosporins (e.g. cefaclor, cefamandole, cephalexin, cefoxitin, cefprozil, cefuroxime); 3rd generation cephalosporins (e.g. cefixime, cefdinir, cefditoren, cefoperazone, cefotaxime, cefpodoxime, ceftazidime, ceftibuten, ceftizoxime, ceftriaxone); 4th generation cephalosporins (e.g. cefepime); the monobactams (e.g. aztreonam); the macrolides (e.g. azithromycin, clarithromycin, dirithromycin, erythromycin, troleandomycin); the monobactams (e.g. aztreonam); the penicillins (e.g. amoxicillin, ampicillin, carbenicillin, cloxacillin, dicloxacillin, nafcillin, oxacillin, penicillin G, penicillin V, piperacillin, ticarcillin); the polypeptide antibiotics (e.g. bacitracin, colistin, polymyxin B, but in certain embodiments not the polymyxin of the polymyxin-alginate oligomer conjugate to be used); the quinolones (e.g. ciprofloxacin, enoxacin, gatifloxacin, levofloxacin, lomefloxacin, moxifloxacin, norfloxacin, ofloxacin, trovafloxacin); the sulfonamides (e.g. mafenide, sulfacetamide, sulfamethizole, sulfasalazine, sulfisoxazole, trimethoprim- sulfamethoxazole); the tetracyclines (e.g. demeclocycline, doxycycline, minocycline, oxytetracycline, tetracycline); the glycylcyclines (e.g. tigecycline); the carbapenems (e.g. imipenem, meropenem, ertapenem, doripenem, panipenem/betamipron, biapenem, PZ-601); other antibiotics include chloramphenicol; clindamycin, ethambutol; fosfomycin; isoniazid; linezolid; metronidazole; nitrofurantoin; pyrazinamide; quinupristin/dalfopristin; rifampin; spectinomycin; and vancomycin.

Representative antifungals include, but are not limited to the polyenes (e.g. natamycin, rimocidin, filipin, nystatin, amphotericin B, candicin; the imidazoles (e.g. miconazole, ketoconazole, clotrimazole, econazole, bifonazole, butoconazole, fenticonazole, isoconazole, oxiconazole, sertaconazole, sulconazole, tioconazole); the triazoles (e.g. fluconazole, itraconazole, isavuconazole, ravuconazole, posaconazole, voriconazole,terconazole); the allylamines (e.g. terbinafine, amorolfine, naftifine, butenafine); and the echinocandins (e.g. anidulafungin, caspofungin, micafungin).

Representative antivirals include, but are not limited to abacavir, acyclovir, adefovir, amantadine, amprenavir, arbidol, atazanavir, atripla, boceprevir, cidofovir, combivir, darunavir, delavirdine, didanosine, docosanol, edoxudine, efavirenz, emtricitabine, enfuvirtide, entecavir, famciclovir, fomivirsen, fosamprenavir, foscarnet, fosfonet, ganciclovir, ibacitabine , imunovir, idoxuridine, imiquimod, indinavir, inosine, interferon type III, interferon type II, interferon type I, lamivudine, lopinavir, loviride, maraviroc, moroxydine, nelfinavir, nevirapine, nexavir, oseltamivir, penciclovir, peramivir, pleconaril, podophyllotoxin, raltegravir, ribavirin, rimantadine, ritonavir, saquinavir , stavudine, tenofovir, tenofovir disoproxil, tipranavir, trifluridine, trizivir, tromantadine, truvada, valaciclovir, valganciclovir, vicriviroc, vidarabine, viramidine, zalcitabine, zanamivir, and zidovudine.

Representative immunostimulatory agents include, but are not limited to, cytokines e.g. TNF, IL-1, IL-6, IL-8 and immunostimulatory alginates, such as high M -content alginates as described for example in US 5,169,840, WO91/11205 and WO03/045402 which are explicitly incorporated by reference herein in their entirety, but including any alginate with immunostimulatory properties.

Representative examples of suitable corticosteroids include but are not limited to prednisone, flunisolide, triamcinolone, fluticasone, budesonide, mometasone, beclomethasone, amcinonide, budesonide, desonide, fluocinonide, fluocinolone, halcinonide, hydrocortisone, cortisone, tixocortol, prednisolone, methylprednisolone, prednisone, betamethasone, dexamethasone, fluocortolone, aclometasone, prednicarbate, clobetasone, clobetasol, and fluprednidene.

Representative NSAIDs include, but are not limited to, the salicylates (e.g. aspirin (acetylsalicylic acid), choline magnesium trisalicylate, diflunisal, salsalate, the propionic acid derivatives (e.g. ibuprofen, dexibuprofen, dexketoprofen, fenoprofen, flurbiprofen, ketoprofen, loxoprofen, naproxen, oxaprozin), the acetic acid derivatives (e.g. aceclofenac, diclofenac, etodolac., indomethacin, ketorolac, nabumetone, tolmetin, sulindac), the enolic acid derivatives (e.g. droxicam, isoxicam, lornoxicam, meloxicam, piroxicam, tenoxicam), the anthranilic acid derivatives (e.g. flufenamic acid, meclofenamic acid, mefenamic acid, tolfenamic acid) and the selective COX-2 inhibitors (Coxibs; e.g. celecoxib, etoricoxib, lumiracoxib, parecoxib, rofecoxib, valdecoxib). The propionic acid derivatives (e.g. ibuprofen, dexibuprofen, dexketoprofen, fenoprofen, flurbiprofen, ketoprofen, loxoprofen, naproxen, oxaprozin) are preferred, ibuprofen being most preferred.

Representative examples of suitable bronchodilators include but are not limited to the β2 agonists (e.g. the short-acting β2 agonists (e.g. pirbuterol, epinephrine, salbutamol, levosalbutamol, clenbuterol, terbutaline, procaterol, metaproterenol, fenoterol, bitolterol mesylate, ritodrine, isoprenaline); the long-acting β2 agonists (e.g. salmeterol, formoterol, bambuterol, clenbuterol); and the ultra-long-acting β2 agonists (e.g. indacaterol)), the anticholinergics (e.g. ipratropium, oxitropium, tiotropium) and theophylline.

As used herein, the terms "mucolytic agent" and "mucus viscosity reducing agent" are intended to encompass agents which reduce the intrinsic viscosity of mucus and agents which reduce the attachment of mucus to underlying epithelium, in particular agents which directly or indirectly disrupt the molecular interactions within or between the components of mucus, agents which affect the hydration of mucus and agents which modulate the ionic microenvironment of the mucosal epithelium (particularly the levels of divalent cations, e.g. calcium). Representative examples of suitable mucus viscosity reducing agents include, but are not limited to, a nucleic acid cleaving enzyme (e.g. a DNase such as DNase I or dornase alfa), hypertonic saline, gelsolin, a thiol reducing agent, an acetylcysteine, an uncharged low molecular weight polysaccharide (e.g. dextran, mannitol), arginine (or other nitric oxide precursors or synthesis stimulators), an agonist of the P2Y2 subtype of purinergic receptors (e.g. denufosol) or an anionic polyamino acid (e.g. poly ASP or poly GLU). Ambroxol, bromhexine, carbocisteine, domiodol, eprazinone, erdosteine, letosteine, mesna, neltenexine, sobrerol, stepronin, tiopronin are specific mucolytics of note. DNase I and hypertonic saline are preferred.

CFTR modulators are small molecules which can redress, at least partially, a CFTR dysfunction. Present CFTR modulators fall into three main groups: CFTR potentiators, CFTR correctors and read-through agents (Derichs, N., Eur. Respir. Rev., 2013, 22(127), 58-65; Petit, R.S. and Fellner, C., Pharmacy and Therapeutics, 2014, 39(7), 500-511; the contents of which are incorporated herein by reference). CFTR potentiators are CFTR modulators which increase the activity of the CFTR ion channel present on the epithelial cell surface. CFTR correctors are CFTR modulators which increase the amount of CFTR protein delivered or retained at the epithelial cell surface. Read-through agents (also known as "premature stop codon suppressors" (PSC suppressors) or "premature termination codon suppressors" (PTC suppressors, which terms are used interchangeably herein) are CFTR modulators which cause the translation machinery of the cell to pass over any premature termination codon in the CFTR mRNA thereby increasing the amount of substantially full length and functional CFTR produced.

Representative examples of suitable CFTR potentiators include, but are not limited to, ivacaftor (VX-770; N-(2,4-di-tert-butyl-5-hydroxyphenyl)-1,4-dihydro-4-oxoquinoline-3-carboxamide) and VRT-532 (4-methyl-2-(5-phenyl-1H-pyrazol-3-yl)-phenol) of Vertex Pharmaceuticals^{™}).

Representative examples of suitable CFTR correctors include, but are not limited to, Prototypical lumacaftor (VX-809) and VX-661 of Vertex Pharmaceuticals^{™} and N6022 (3-[1-(4-carbamoyl-2-methylphenyl)-5-(4-imidazol-1-ylphenyl)pyrrol-2-yl]propanoic acid).

Representative examples of suitable read-through agents include, but are not limited to, ataluren (PTC124) of PTC Therapeutics and gentamicin.

The invention will be further described with reference to the following non-limiting Example in which:

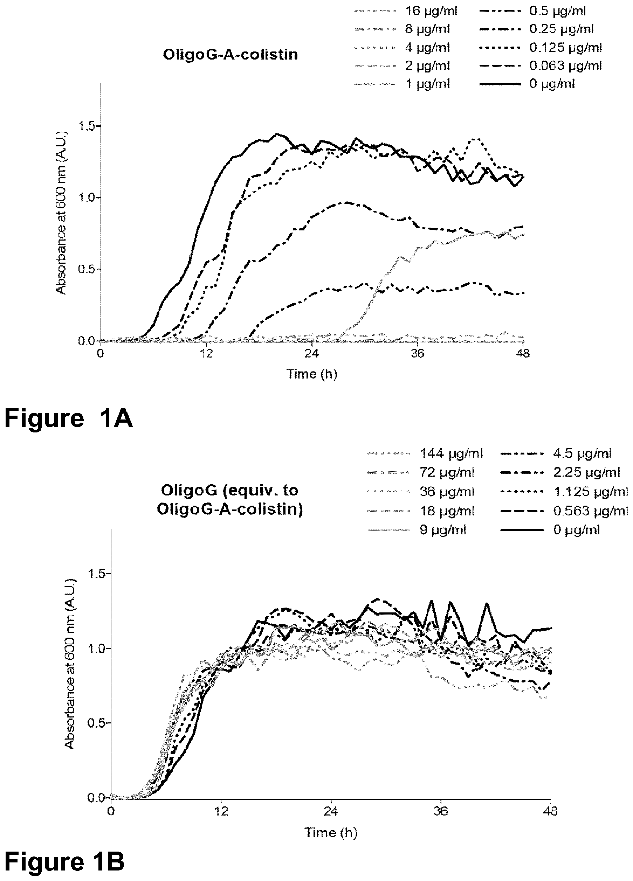

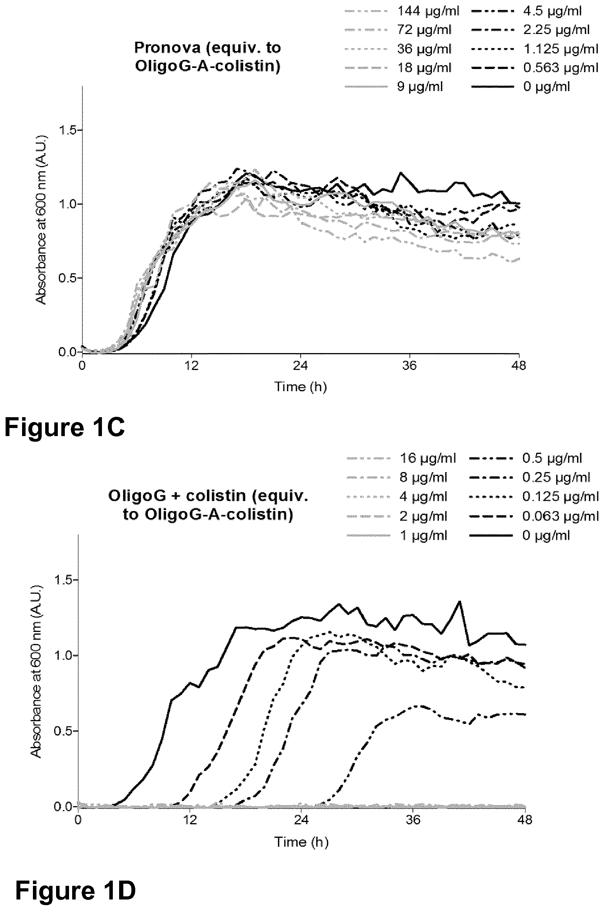

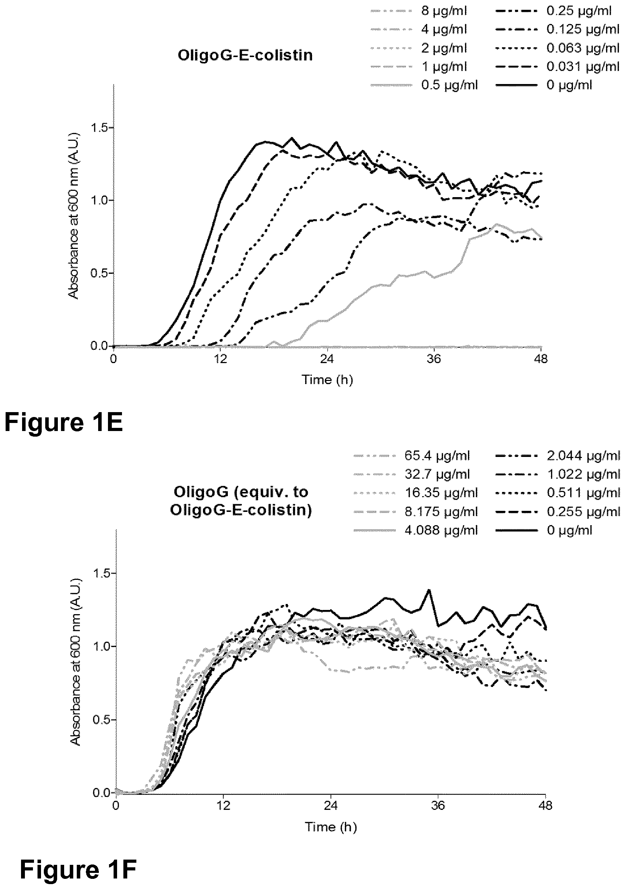

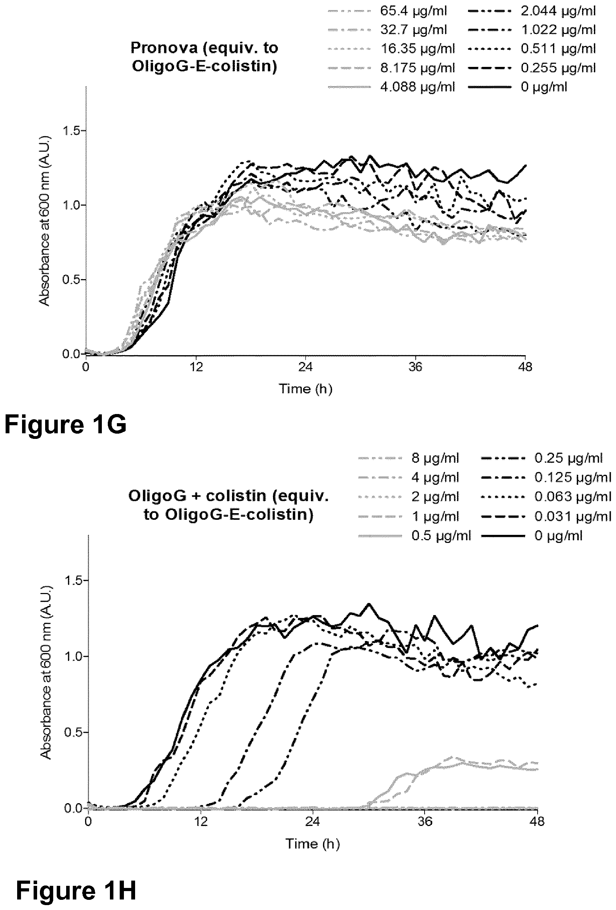

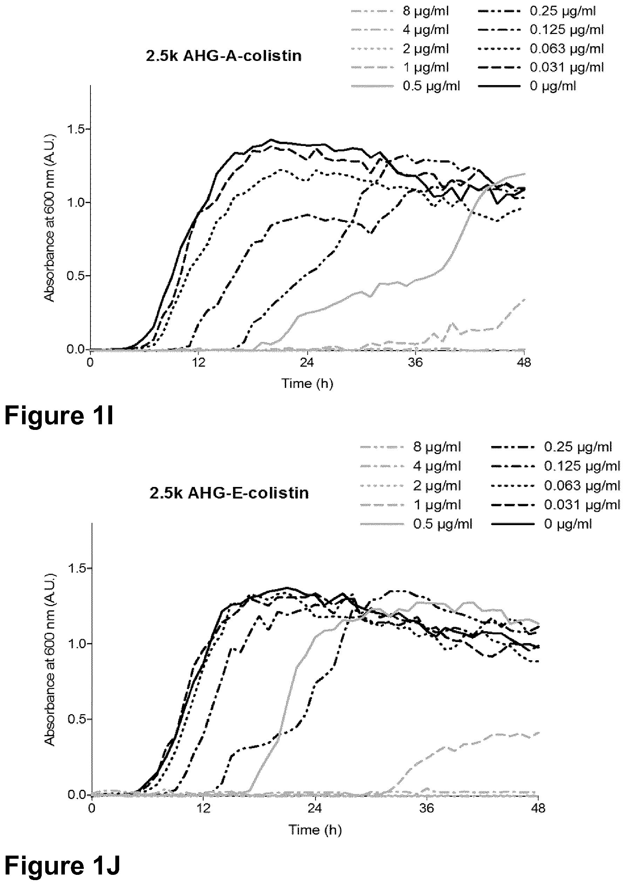

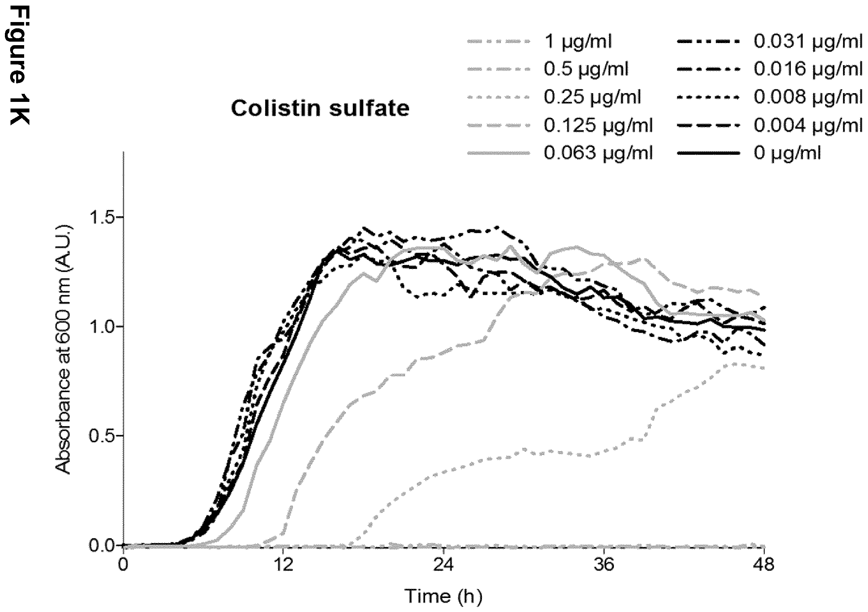

Figure 1 shows graphical representations of the growth of **V2** ***Pseudomonas aeruginosa*** under varying concentrations of **A:** OligoG-A-colistin (median MIC 1µg/mL); **B:** OligoG (at equivalent concentrations to the OligoG in the OligoG-A-colistin conjugates of A); **C:** Pronova alginate (at equivalent concentrations to the OligoG in the OligoG-A-colistin conjugates of A); **D:** OligoG (at equivalent concentrations to the OligoG in the OligoG-A-colistin conjugates of A) and colistin (at equivalent concentrations to the colistin in the OligoG-A-colistin conjugates of A); **E** OligoG-E-colistin (median MIC 0.5µg/mL); **F**: OligoG (at equivalent concentrations to the OligoG in the OligoG-E-colistin conjugates of E); **G**: Pronova alginate (at equivalent concentrations to the OligoG in the OligoG-E-colistin conjugates of E); **H**: OligoG (at equivalent concentrations to the OligoG in the OligoG-E-colistin conjugates of E) and colistin (at equivalent concentrations to the colistin in the OligoG-E-colistin conjugates of E); **I**: 2.5k AHG-A-colistin (median MIC 0.5µg/mL); **J**: 2.5k AHG-E-colistin (median MIC 0.5µg/mL); and **K**: colistin sulphate (median MIC 0.063µg/mL).

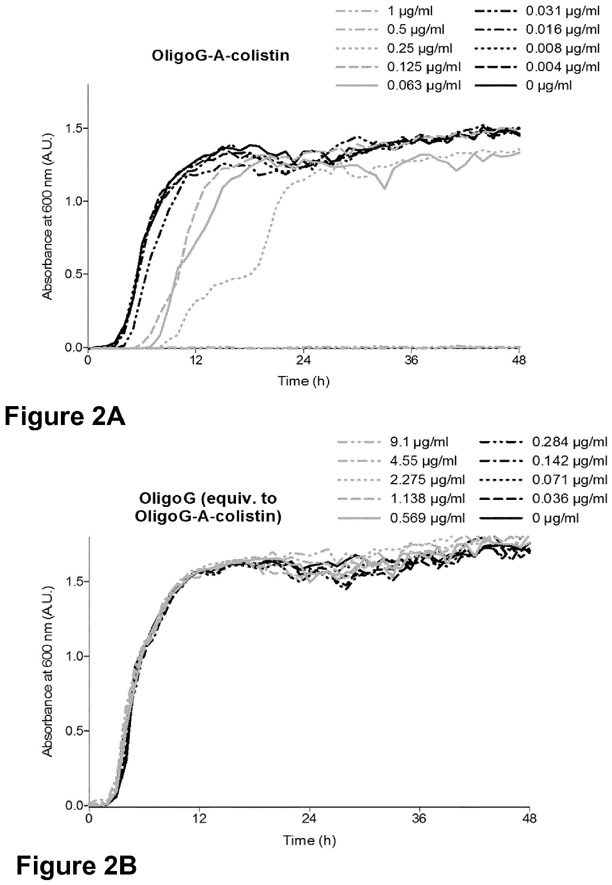

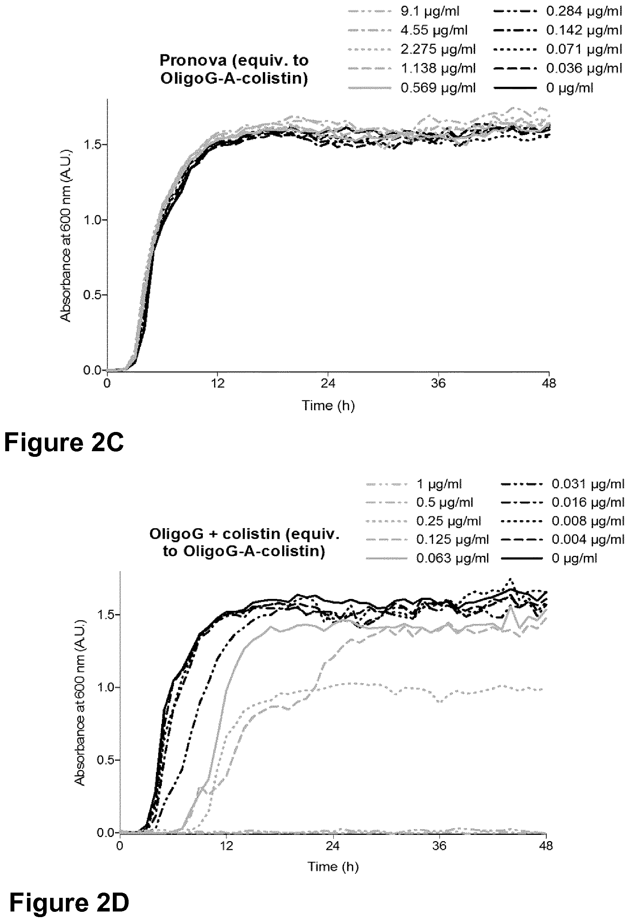

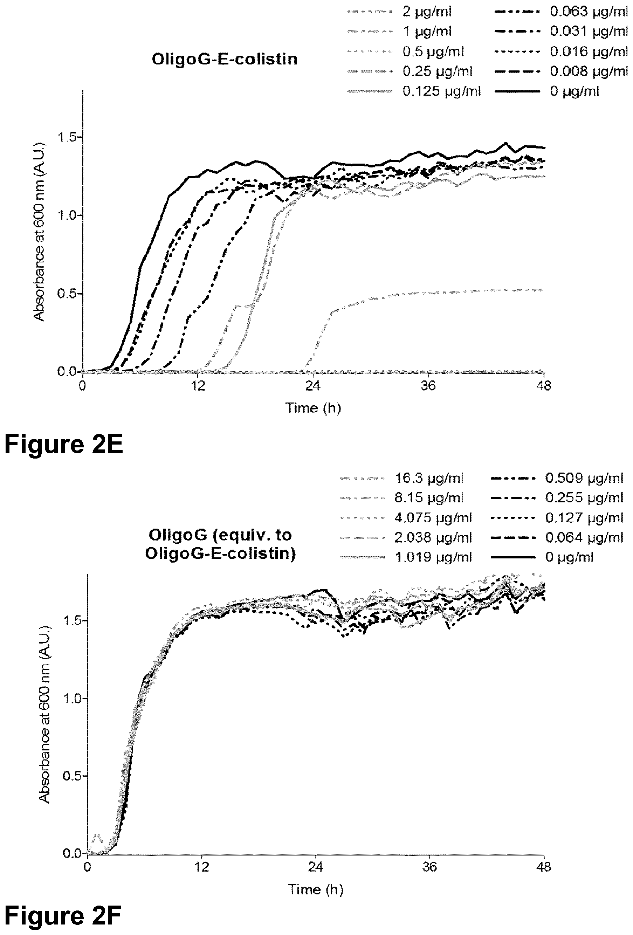

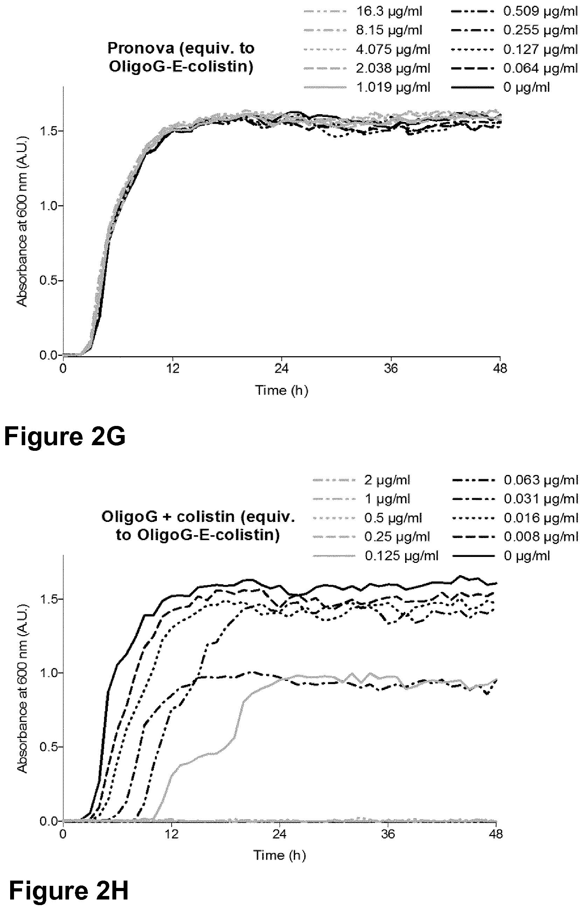

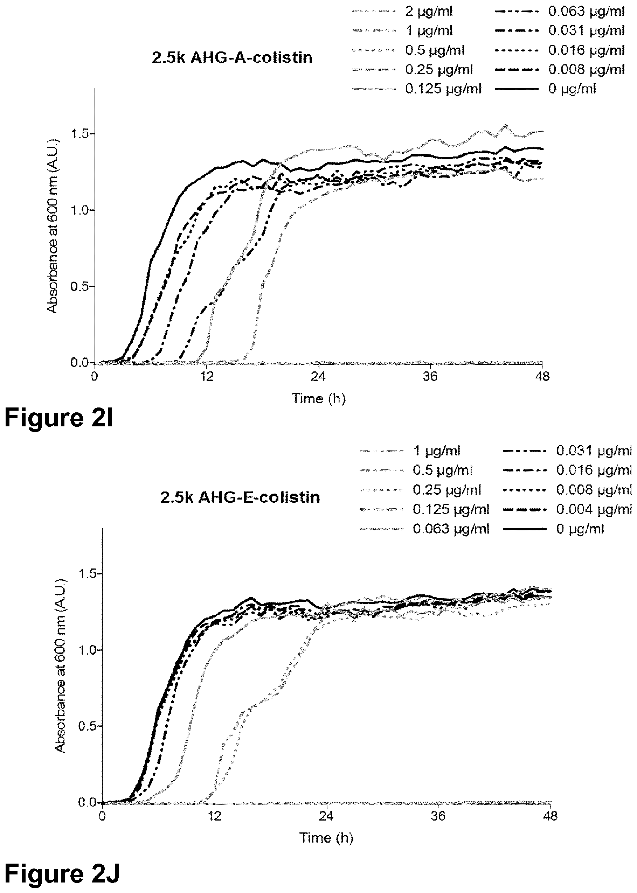

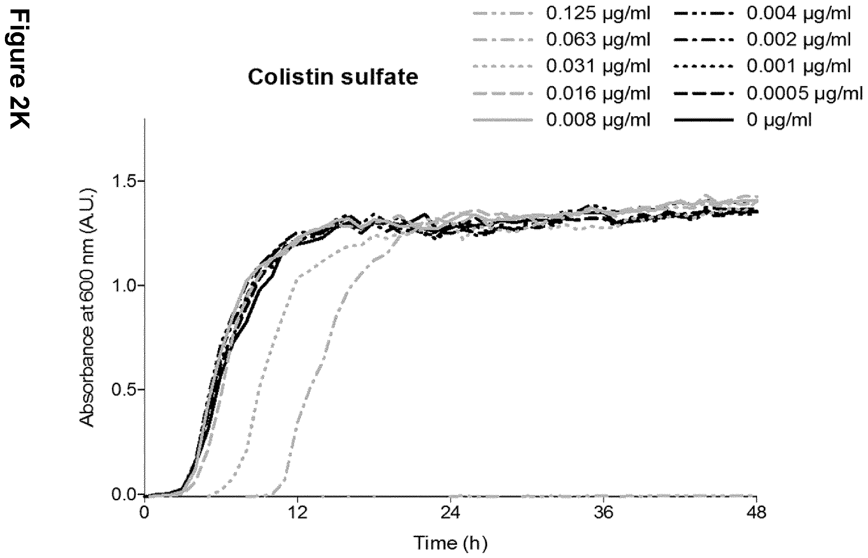

Figure 2 shows graphical representations of the growth of **V3 *Klebsiella pneumoniae*** under varying concentrations of **A:** OligoG-A-colistin (median MIC 0.063µg/mL); **B:** OligoG (at equivalent concentrations to the OligoG in the OligoG-A-colistin conjugates of A); **C:** Pronova alginate (at equivalent concentrations to the OligoG in the OligoG-A-colistin conjugates of A); **D:** OligoG (at equivalent concentrations to the OligoG in the OligoG-A-colistin conjugates of A) and colistin (at equivalent concentrations to the colistin in the OligoG-A-colistin conjugates of A); **E** OligoG-E-colistin (median MIC 0.125µg/mL); **F:** OligoG (at equivalent concentrations to the OligoG in the OligoG-E-colistin conjugates of E); **G:** Pronova alginate (at equivalent concentrations to the OligoG in the OligoG-E-colistin conjugates of E); **H:** OligoG (at equivalent concentrations to the OligoG in the OligoG-E-colistin conjugates of E) and colistin (at equivalent concentrations to the colistin in the OligoG-E-colistin conjugates of E); **I:** 2.5k AHG-A-colistin (median MIC 0.125µg/mL); **J:** 2.5k AHG-E-colistin (median MIC 0.063µg/mL); and **K:** colistin sulphate (median MIC 0.008µg/mL).

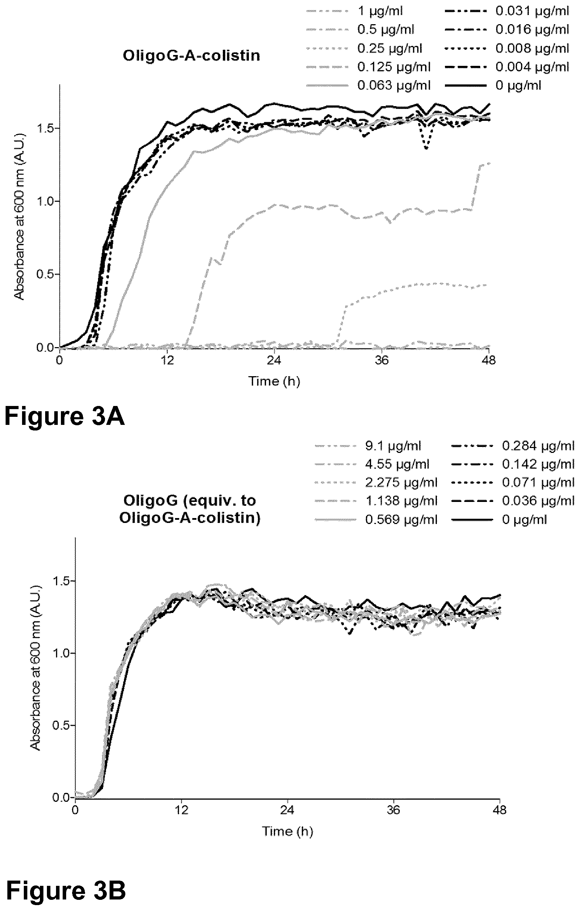

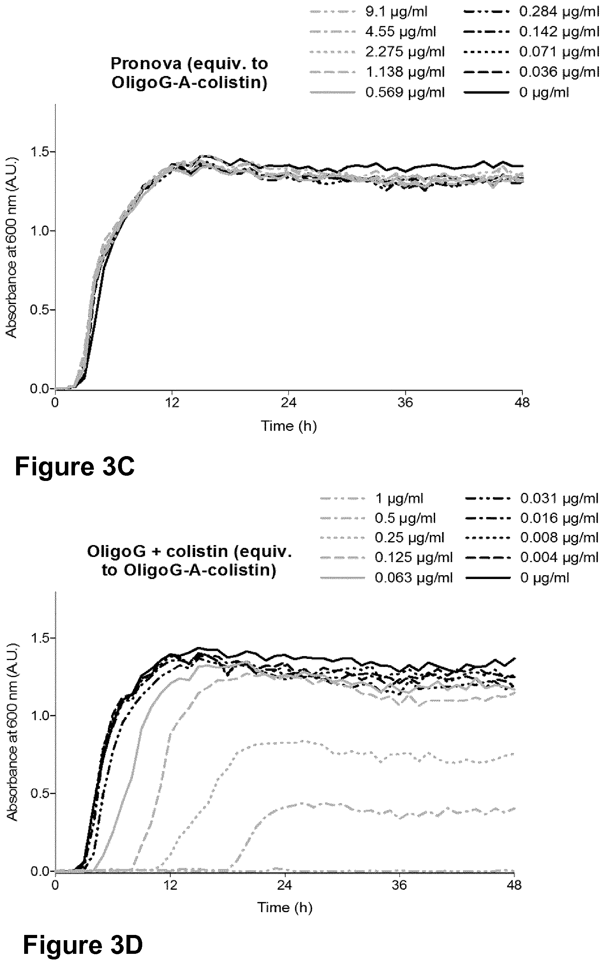

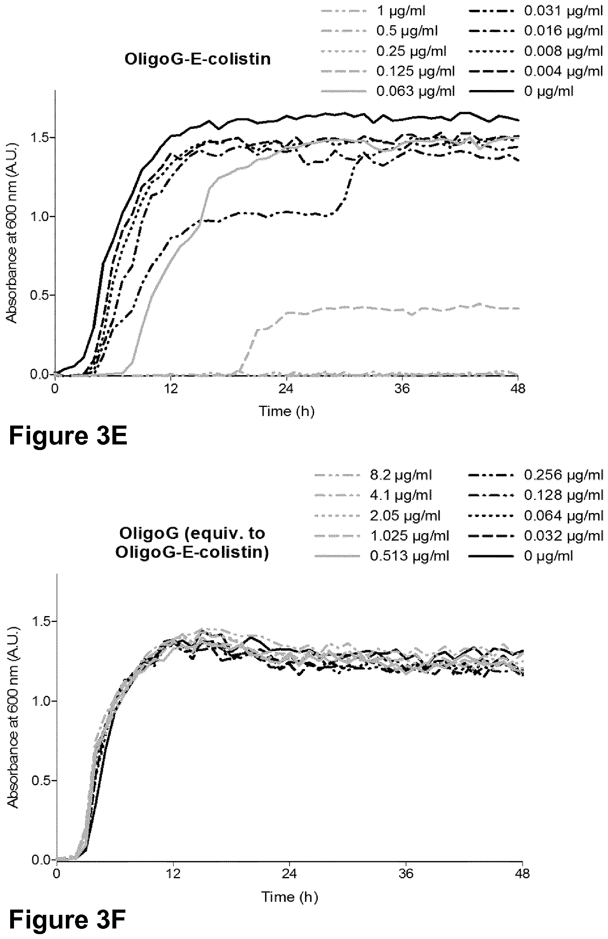

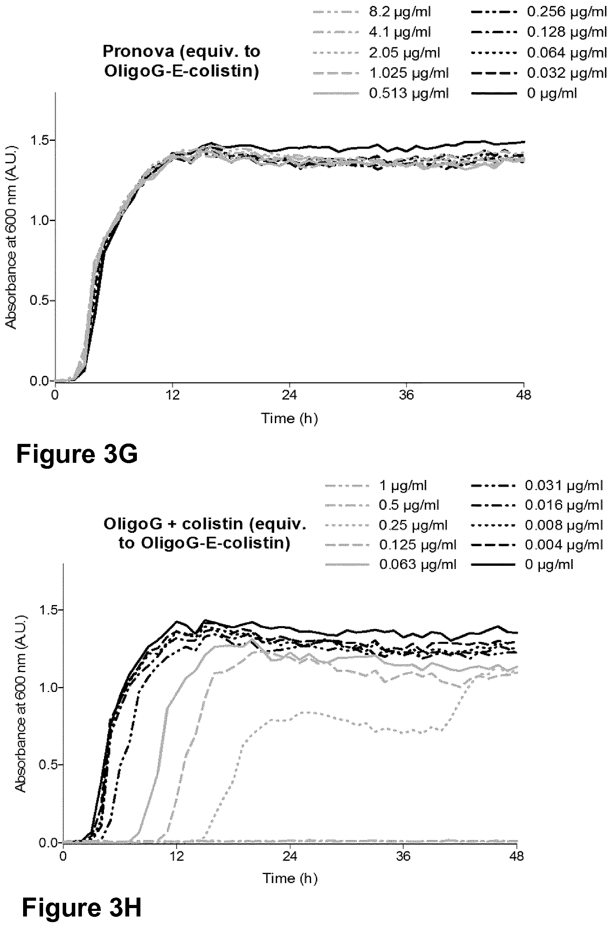

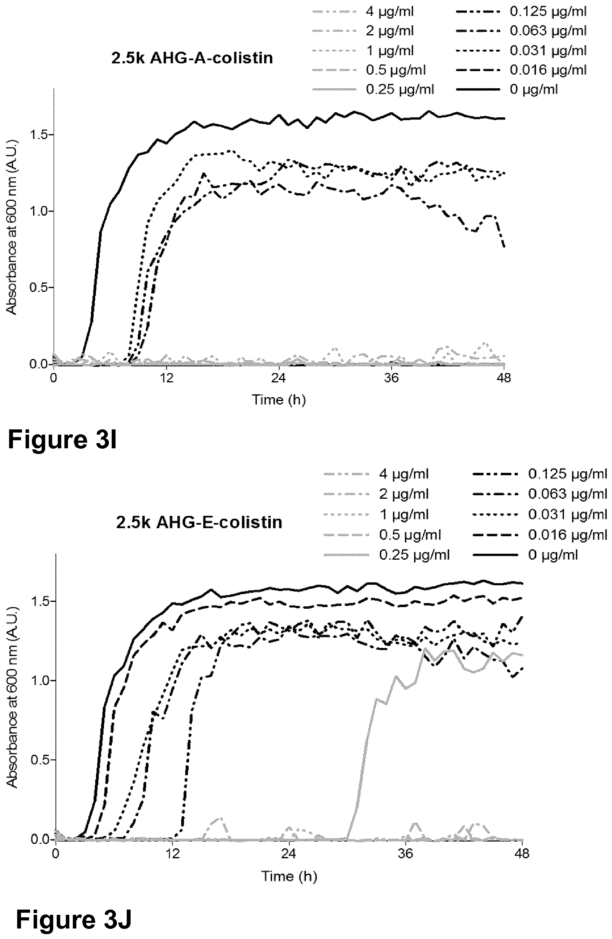

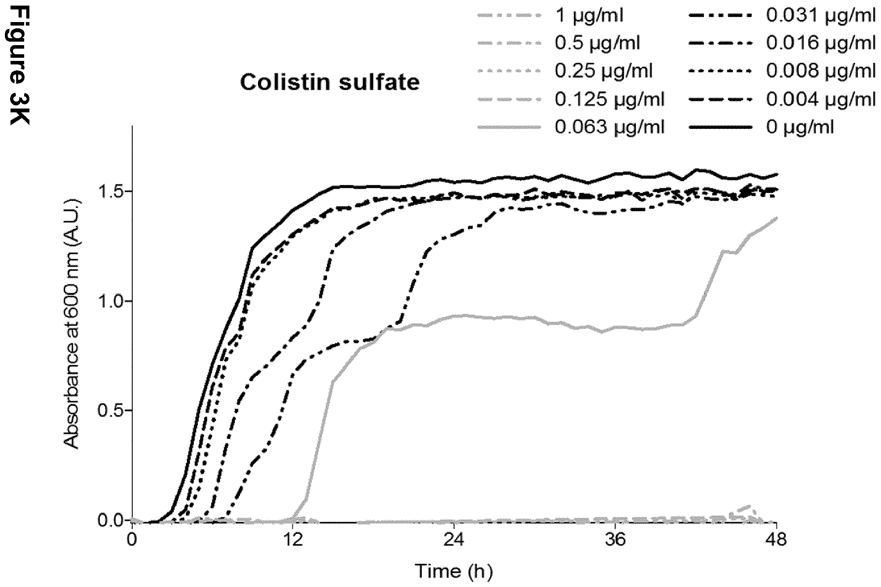

Figure 3 shows graphical representations of the growth of **V19 *Acinetobacter baumannii*** under varying concentrations of **A:** OligoG-A-colistin (median MIC 0.063µg/mL); **B:** OligoG (at equivalent concentrations to the OligoG in the OligoG-A-colistin conjugates of A); **C:** Pronova alginate (at equivalent concentrations to the OligoG in the OligoG-A-colistin conjugates of A); **D:** OligoG (at equivalent concentrations to the OligoG in the OligoG-A-colistin conjugates of A) and colistin (at equivalent concentrations to the colistin in the OligoG-A-colistin conjugates of A); **E** OligoG-E-colistin (median MIC 0.125µg/mL); **F:** OligoG (at equivalent concentrations to the OligoG in the OligoG-E-colistin conjugates of E); **G:** Pronova alginate (at equivalent concentrations to the OligoG in the OligoG-E-colistin conjugates of E); **H:** OligoG (at equivalent concentrations to the OligoG in the OligoG-E-colistin conjugates of E) and colistin (at equivalent concentrations to the colistin in the OligoG-E-colistin conjugates of E); **I:** 2.5k AHG-A-colistin (median MIC 0.125µg/mL); **J:** 2.5k AHG-E-colistin (median MIC 0.125µg/mL); and **K:** colistin sulphate (median MIC 0.002µg/mL).

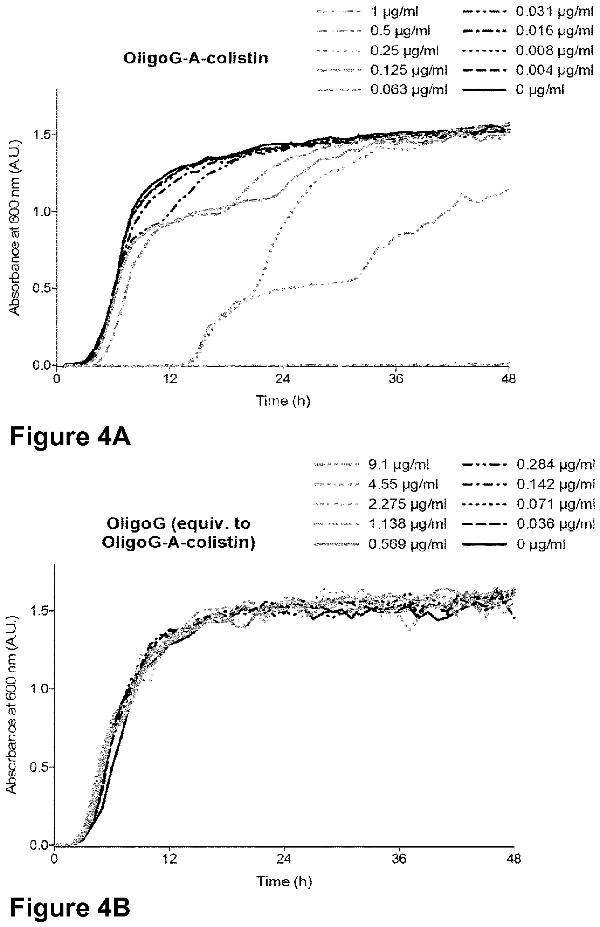

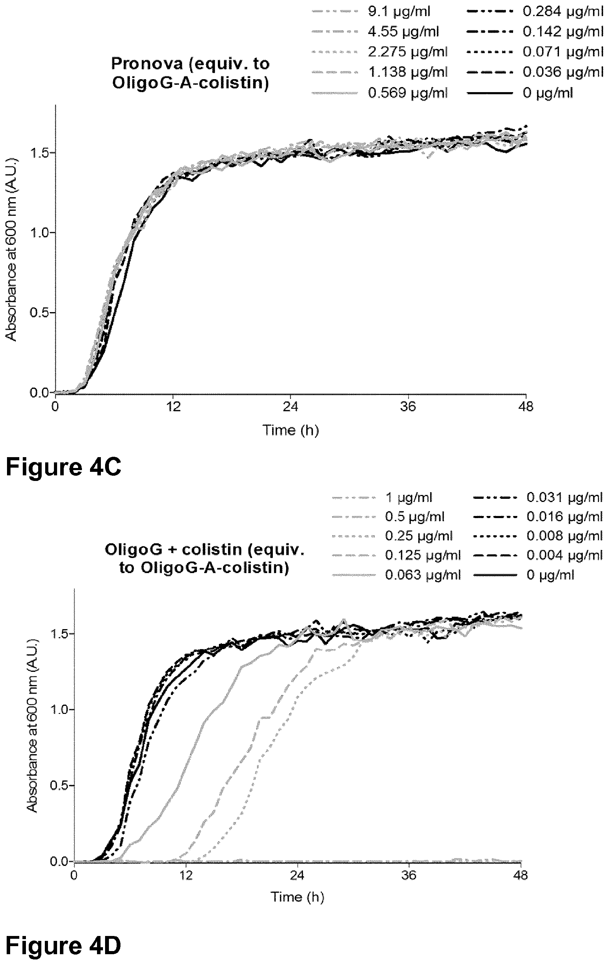

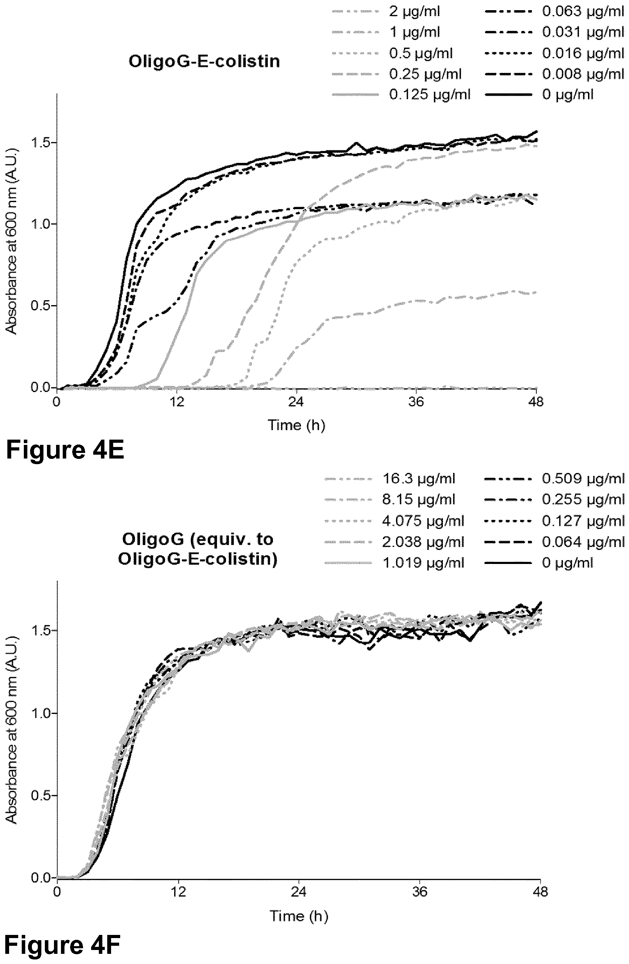

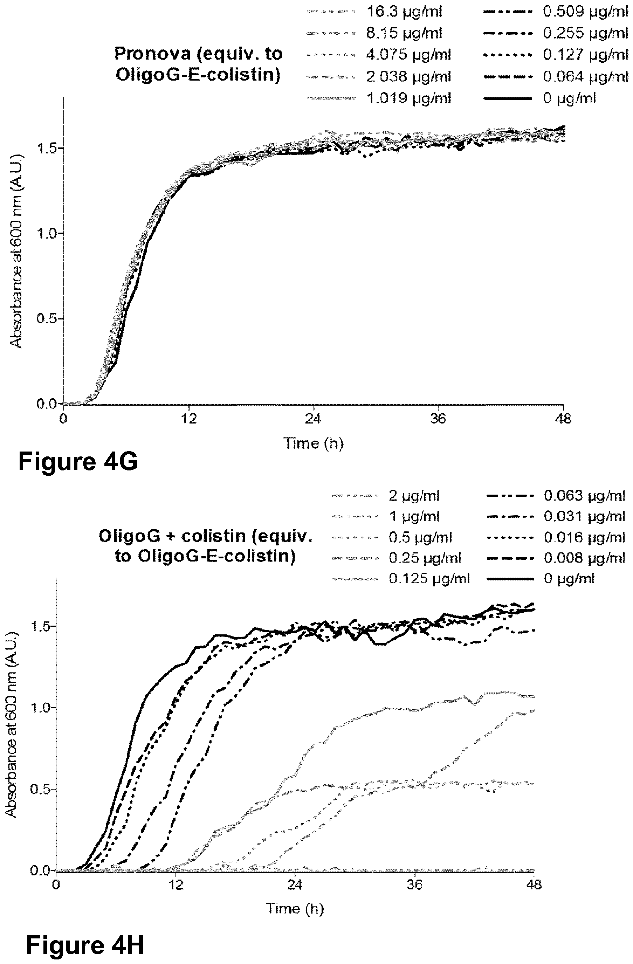

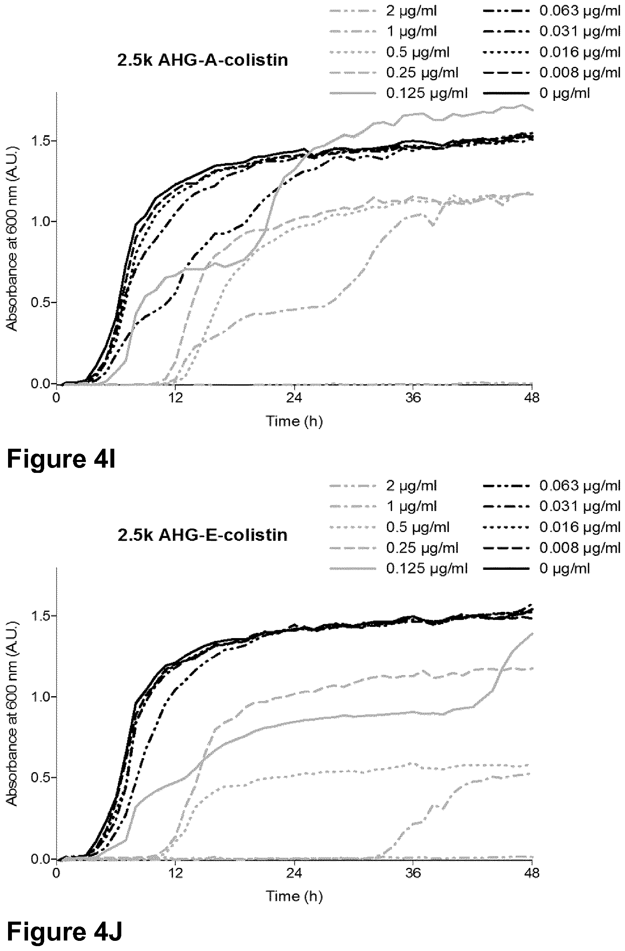

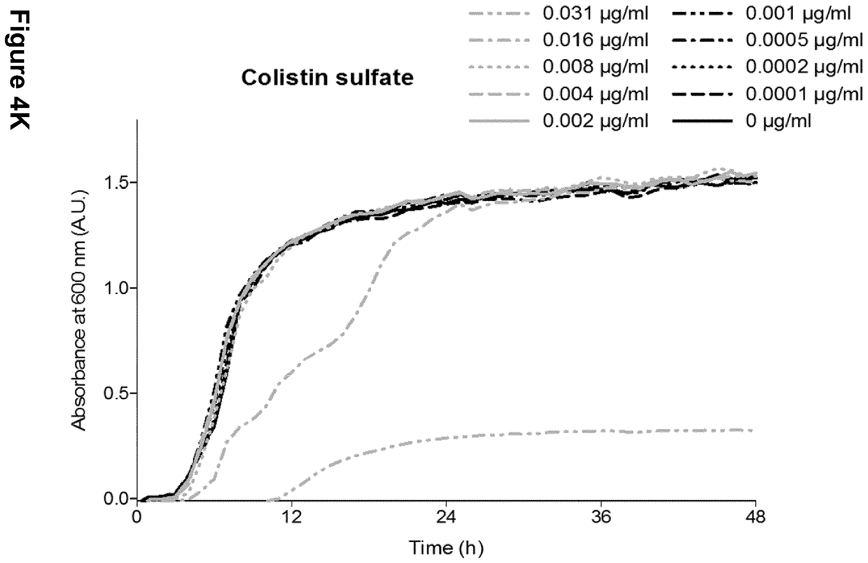

Figure 4 shows graphical representations of the growth of **V7 *Escherichia coli*** under varying concentrations of **A:** OligoG-A-colistin (median MIC 0.063µg/mL); **B:** OligoG (at equivalent concentrations to the OligoG in the OligoG-A-colistin conjugates of A); **C:** Pronova alginate (at equivalent concentrations to the OligoG in the OligoG-A-colistin conjugates of A); **D:** OligoG (at equivalent concentrations to the OligoG in the OligoG-A-colistin conjugates of A) and colistin (at equivalent concentrations to the colistin in the OligoG-A-colistin conjugates of A); **E** OligoG-E-colistin (median MIC 0.125µg/mL); **F:** OligoG (at equivalent concentrations to the OligoG in the OligoG-E-colistin conjugates of E); **G:** Pronova alginate (at equivalent concentrations to the OligoG in the OligoG-E-colistin conjugates of E); **H:** OligoG (at equivalent concentrations to the OligoG in the OligoG-E-colistin conjugates of E) and colistin (at equivalent concentrations to the colistin in the OligoG-E-colistin conjugates of E); **I:** 2.5k AHG-A-colistin (median MIC 0.5µg/mL); **J:** 2.5k AHG-E-colistin (median MIC 0.5µg/mL); and **K:** colistin sulphate (median MIC 0.063µg/mL).

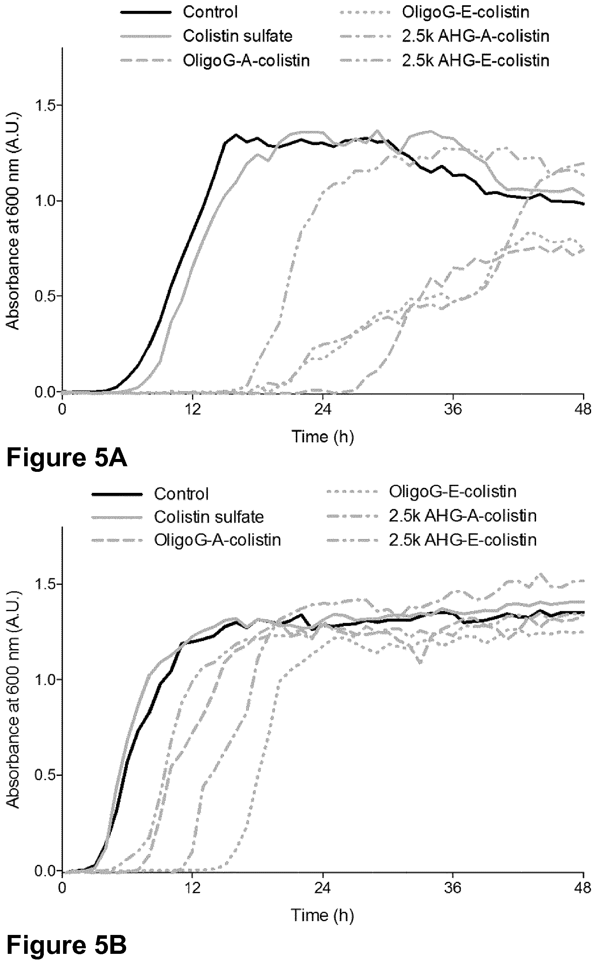

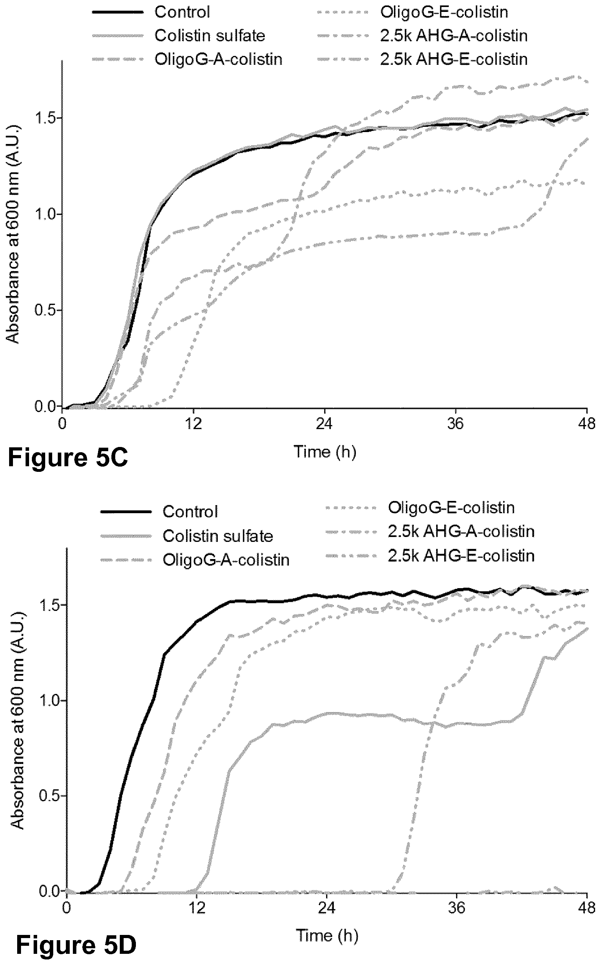

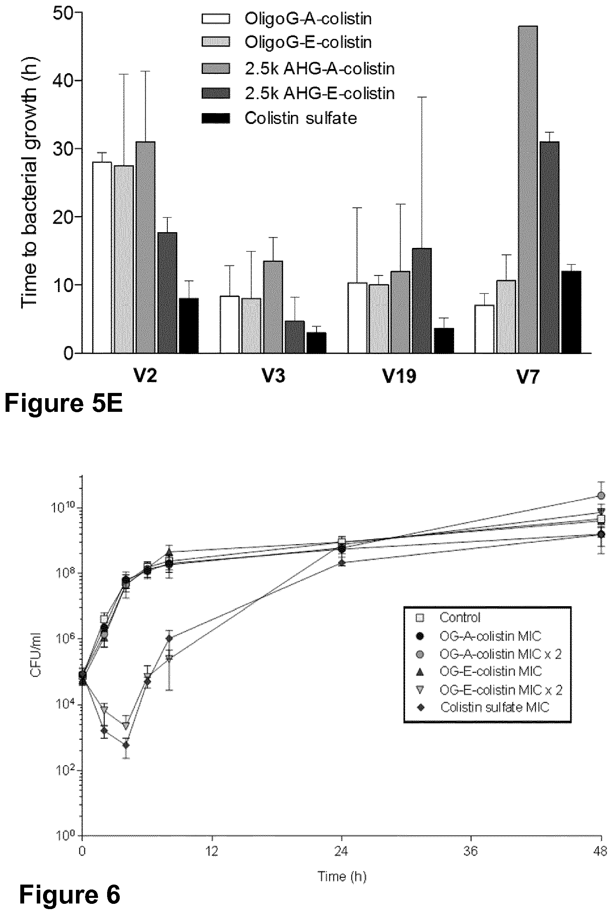

Figure 5 shows graphical representations of the growth of **A:** V2 *Pseudomonas aeruginosa,* **B*:*** V3 *Klebsiella pneumonia,* **C*:*** V19 *Acinetobacter baumannii* and **D:** V7 *Escherichia coli* in the presence of OligoG-A-colistin, OligoG-E-colistin, 2.5k AHG-A-colistin, 2.5k AHG-E-colistin and colistin sulphate at the respective MIC of each agent; and **E** a comparison of the mean times for the initiation of bacterial growth for each strain in the presence of each agent at the respective MIC of each agent.
Figure 6 shows a graphical representation of CFU number against incubation time for *Acinetobacter baumanii* (V19) in the two compartment membrane diffusion model of Example 4 following exposure to OligoG-A-colistin at MIC (black circles), OligoG-A-colistin at MIC x2 (grey circles), OligoG-E-colistin at MIC (triangles), OligoG-E-colistin at MIC x2 (inverted triangles) colistin sulphate at MIC (diamonds), or untreated control (squares).

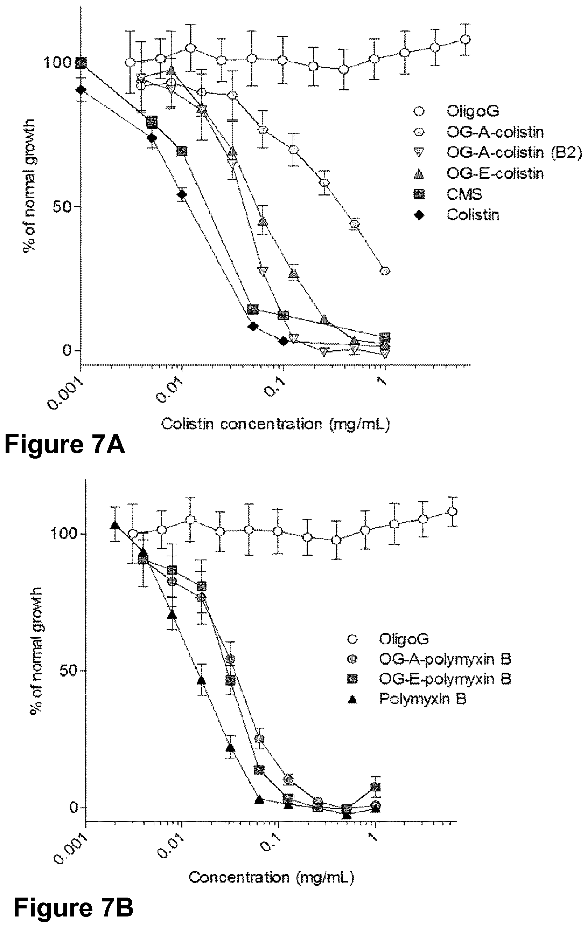

Figure 7 shows graphical represenations of the cytotoxicity of increasing concentrations of **A:** OligoG (circles), OligoG-A-colistin (two batches; hexagons and inverted triangles), OligoG-E-colistin (triangles), colistin methanesulphonate (CMS; squares) and colistin sulphate (diamonds); and **B:** OligoG (empty circles), OligoG-E-polymyxin B (squares), OligoG-A-polymyxin B (filled circles), and polymyxin B (triangles).

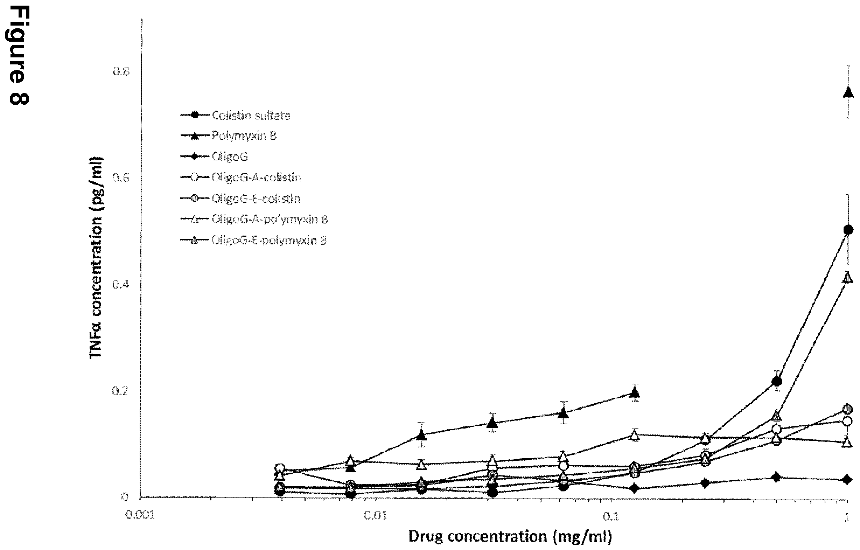

Figure 8 shows a graphical representation of TNFα release from HK2 cells, adjusted for cell viability, upon exposure to increasing concentrations of polymyxin B (black triangles), colistin sulphate, (black circles), OligoG-E-polymyxin B (grey triangles), OligoG-E-colistin (grey circles), OligoG-A-colistin (white circles), OligoG-A-polymyxin B (white triangles), OligoG. Data expressed as mean ± SEM (n = 6).

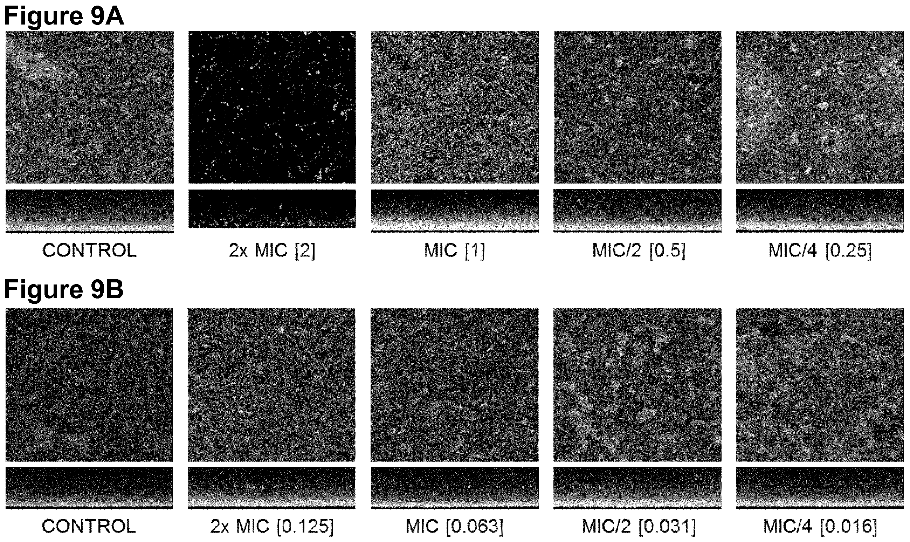

Figure 9 shows the effects of increasing concentrations of **A:** OligoG-A-colistin (median MIC = 1 µg/mL) and **B:** colistin sulphate (median MIC = 0.063 µg/mL) on the structure of and cell viability within *Pseudomonas aeruginosa* biofilms formed in the presence of said agents.

### EXAMPLES

### Example 1 - Preparation of polymyxin-alginate oligomer conjugates

### Preparation of alginate oligomers by acid hydrolysis

Sodium alginate, containing >60% guluronate monomers (PRONOVA UP MVG), was dissolved in dH₂O (10 mg/mL), the solution adjusted to 0.01 M HCI and then placed in a water bath at 80°C for up to 24 h. To terminate the hydrolysis, the acid hydrolysate was cooled and pH was increased to pH 7 by addition of 10 M NaOH. Solutions were lyophilised and re-suspended in minimal dH₂O.

To obtain acid-hydrolysed G-fragments (AHGs) with acceptable number average degree of polymerisation as well as molecular weight distribution, the solutions were filtered once by dialysis to remove LMW oligomers and salts. Typically, the following reaction times and dialysis membrane cut-offs were used to produce AHGs with a desired molecular weight:

| **Desired MW (g/mol)** | **Approx. reaction time (h:min)** | **Dialysis membrane cut-off (g/mol)** |
|---|---|---|
| 2,500 | 21:00 | 1,000 |
| 7,500 | 9:45 | 2,000 |
| 15,000 | 5:30 | 8,000 |

The final products were lyophilised and characterised by FT-IR and gel permeation chromatography (GPC). The GPC system comprised of two TSK G5000PW_{XL} and G3000PW_{XL} columns (Polymer Laboratories, UK) in series, mobile phase PBS (pH 7.4) and flow rate of 1 mL/min. Molecular weight and polydispersity were calculated relative to pullulan standards. Alginate standards are not commercially available, which necessitated the use of pullulan. These calibration standards do not give correct molecular weight for alginates since GPC separates according to hydrodynamic volume, which differs with varying macromolecular architecture. Alginates are more expanded than pullulans, resulting in a six-fold overestimate of alginate's molecular weight by GPC with pullulan. Consequently, a scaling factor (divide by 6) was applied to the apparent molecular weights calculated from pullulan calibration, to adjust for different macromolecular architecture of pullulan MW standards (Andersen T., et al., 2012, Alginates as biomaterials in tissue engineering. Carbohydrate Chemistry: Chemical and Biological Approaches, Vol. 37, 232-233*)*.

Samples for GPC were prepared in PBS (3 mg/mL) and the eluate was monitored using a differential refractometer (Optilab t-Rex (Wyatt, UK)). PL Caliber Instrument software, version 7.0.4, from Polymer Laboratories (UK) was used for data analysis.

Acid-hydrolysed G-fragments (AHGs) were generated with molecular weights between 2,500-26,000 g/mol (polydispersity 2.5-3.5). Typical characteristics of AHGs used in these studies are summarised in the table below.

| **Name** | **Apparent molecular weight (g/mol)*** | **PDI** | **DPₙ** |
|---|---|---|---|
| OligoG | 15,600 [2,600] | 1.8 | 13 |
| '2.5k' | 15,000 [2,500] | 1.4 | 13 |
| '6k' | 36,207 *[6,035]* | 4.0 | 30 |
| '7.5k' | 42,000 [7,000] | 2.6 | 35 |
| '9k' | 52,000 *[8,670]* | 3.0 | 45 |
| '13.5k' | 81,543 *[13,590]* | 2.9 | 68 |
| '15k' | 72,000 [12,000] | 3.5 | 60 |
| '16k' | 93,500 *[15,580]* | 3.6 | 81 |
| '20k' | 119,362 *[19,894]* | 3.1 | 100 |
| '26k' | 152,500 *[25,420]* | 3.5 | 132 |
| PRONOVA UP MVG⁺ | 651,000 *[108,500]* | 2.5 | 565 |

| | | | |
|---|---|---|---|
| *relative to pullulan MW standards; *value in [ ] indicates estimated actual MW after applying scaling factor to adjust for different macromolecular architecture of pullulan MW standards.* *⁺Elutes in void volume, therefore MW is likely underestimated.* OligoG CF-5/20 for conjugation was produced as described previously (Khan et al. 2012, Antimicrobial Agents and Chemotherapy 56(10), 5134-5141). OligoG is a 5-20mer alginate oligomer with at least 85%G residues. | | | |

### Ester conjugation ('E')

Briefly, for 2,500 g/mol AHG conjugation to colistin, alginate oligomer (200 mg, 0.08 mmol) was dissolved under stirring in anhydrous DMF (4 mL) (or anhydrous DMSO for Batches 3 and later) in a 10 mL round-bottomed flask. To this, DCC (16.5 mg, 0.08 mmol), DMAP (1.6 mg, 0.01 mmol) and colistin sulfate (37.5 mg, 0.03 mmol) were added, and the reaction allowed to proceed at room temperature overnight, under stirring. To stop the reaction, the mixture was poured into excess chloroform (up to 20 mL), then the resulting precipitate was collected by filtration, re-dissolved in distilled water (dH₂O, 2 mL) and purified by FPLC (see below).

The other ester-linked conjugates recited herein were prepared analogously.

### Amide conjugation ('A')

Briefly, for 2,500 g/mol AHG conjugation to colistin, alginate oligomer (200 mg, 0.08 mmol) was dissolved under stirring in dH₂O (2 mL) in a 10 mL round-bottomed flask. To this, EDC (19.9 mg, 0.1 mmol) and sulfo-NHS (22.6 mg, 0.1 mmol) were added, and the mixture was left stirring for 15 min. Subsequently, colistin sulfate (37.5 mg, 0.03 mmol) was added, and the reaction mixture was left stirring for 2 h at room temperature, the stored at -20 °C until purification by FPLC (see below).

The other amide-linked conjugates recited herein were prepared analogously.

### Purification of conjugates by FPLC

Conjugates were purified from the reaction mixture by fast protein liquid chromatography (FPLC) (AKTA FPLC; Amersham Pharmacia Biotech, UK) using a pre-packed HiLoad Superdex 75 16/600 PG column with a UV detector and data analysis using Unicorn 4.0 software (Amersham Pharmacia Biotech, UK). Samples of the reaction mixture (2 mL) were injected into a 2 µL loop using PBS (pH 7.4) at 0.5 mL/min as a mobile phase. Fractions (5 mL) were collected, dialysed against deionised water (8 water changes) and assayed for protein content (BCA assay) before pooling fractions containing conjugate. The final conjugate was lyophilised and stored at - 20 °C.

### Example 2 - Characterisation of alginate oligomer-antibiotic conjugates

### Purity, molecular weight and drug content

Alginate oligomer-antibiotic conjugates were characterised by FPLC and GPC to assess purity and estimate molecular weight, and the total drug content of the conjugate was determined by the BCA assay using free drug as calibrant.

The FPLC system described above for purification was used again for final conjugate characterisation, with a pre-packed Superdex 75 10/300 GL column. Samples (200 µL) were dissolved in PBS (pH 7.4) and injected into a 100 mL loop at 0.5 mL/min. The GPC system described above as used again for final conjugate characterisation, and the same scaling factor was applied.

For the amide linked conjugates a ninhydrin assay was used to determine how many of colistin's amine groups were used for binding to alginate oligomers via amide bonds. First, a 4 M lithium acetate buffer solution was prepared by dissolving lithium acetate dihydrate (40.81 g) in 60 mL dH₂O. Sufficient acetic acid (glacial) was added until pH 5.2 was reached. The volume was made up to a final volume of 100 mL with dH₂O. Next, ninhydrin (0.2 g) and hydrindantin (0.03 g) were dissolved in 7.5 mL DMSO and 2.5 mL lithium acetate buffer. Buffered ninhydrin reagent (86 µL) was added to an equal quantity of sample/ standard solution (1.5 mL eppendorf) and heated in a water bath at 100 °C for 15 min. The mixture was subsequently cooled to room temperature and 130 µL of 50% v/v ethanol was added. The solution was mixed before adding 200 µL of the final solution into wells of a 96-well plate. Spectrophotometric analysis was performed at 570 nm. Calibration of the assay was achieved using ethanolamine (0-0.1158 mM).

### Stability of alginate oligomers and alginate oliqomer-antibiotic conjugates

To compare the rate of degradation of alginate oligomers, solutions (3 mg/mL) were prepared in PBS at pH 7 containing bacterial alginate lyase (0, 1-1000 U/mL)) and incubated at 37 °C for up to 48 h. Alginate oligomer-antibiotic conjugate solutions were prepared (3 mg/mL) in either i) PBS at pH 5, ii) PBS at pH 7, or iii) PBS at pH 7 containing bacterial alginate lyase (1 U/mL)) and incubated at 37 °C for up to 48 h. At various time points, samples (300 mL) were taken, immediately snap-frozen in liquid nitrogen to stop the reaction and then stored at -20 °C until analysis by GPC to determine the change in molecular weight over time and by FPLC (conjugates only) to determine the change in free colistin over time.

### Results

Using these alginate oligomers, a library of conjugates has been prepared, with a typical reaction yield of -50, with molecular weights of 24,000-120,000 g/mol (relative to pullulan molecular weight 59 standards); containing 1-11%% w/w drug loading (equivalent to 3-6 AHGs: 1 drug molecule).

### Batch 1

| **Conjugate** | **Initial drug ratio (% w/w)** | **Drug loading (% w/w) (% yield)** | **Molar ratio (1 antibiotic: x AHG)** | **Molecular weight (g/mol)** | **PDI** | **Free drug (%)** |
|---|---|---|---|---|---|---|
| OligoG-AMIDE-colistin | 18.0 | 10.8 (60%) | 4.65 | 24,000 | 1.7 | 1.2 |
| OligoG-ESTER-colistin | 18.0 | 10.6 (59%) | 4.75 | 25,000 | 1.8 | 0.5 |
| 6k AHG-AMIDE-colistin* | 7.8 | 4.0 (51%) | 5.63 | 44,000 | 2.8 | 5.9 |
| 6k AHG-ESTER-colistin* | 7.8 | 1.2 (15%) | 19.32 | 42,750 | 2.7 | 6.4 |
| 9k AHG-AMIDE-colistin* | 5.4 | 2.9 (54%) | 5.24 | 55,250 | 2.8 | 9.0 |
| 9k AHG-ESTER-colistin | 5.4 | 6.0(111%) | 2.45 | 56,250 | 3.2 | 4.0 |
| 13.5k AHG-ESTER-colistin* | 3.5 | 0.8 (23%) | 12.93 | 82,750 | 3.1 | 1.2 |
| 16k AHG-AMIDE-colistin* | 3.0 | 2.7 (67%) | 3.17 | 109,500 | 3.4 | 2.7 |
| 16k AHG-ESTER-colistin | 3.0 | 5.0 (167%) | 1.67 | 111,000 | - | 2.3 |
| OligoG-AMIDE-polymyxin B | 17.8 | 9.7 (54%) | 5.16 | 22,500 | 1.7 | 0.9 |
| OligoG-ESTER-polymyxin B* | 17.8 | 2.0 (11%) | 27.17 | 20,000 | 2.1 | 4.1 |
| 6k AHG-AMIDE-polymyxin B* | 7.7 | 1.6(21%) | 14.21 | 39,000 | 3.4 | 0.7 |
| 6k AHG-ESTER-polymyxin B* | 7.7 | 3.0 (39%) | 7.47 | 42,500 | 3.0 | 9.9 |
| 9k AHG-AMIDE-polymyxin B | 5.4 | 2.4 (54%) | 6.26 | 55,500 | 2.6 | 3.1 |
| 9k AHG-ESTER-polymyxin B* | 5.4 | 0.8(15%) | 19.10 | 60,250 | 3.3 | 1.3 |
| 13.5k AHG-ESTER-polymyxin B* | 3.4 | 0.7 (21%) | 14.56 | 84,750 | 3.3 | 6.6 |
| 16k AHG-AMIDE-polymyxin B | 3.0 | 2.3 (77%) | 3.68 | 119,000 | 3.1 | 1.0 |

| | | | | | | |
|---|---|---|---|---|---|---|
| *Purified by ion exchange instead of size exclusion chromatography | | | | | | |

### Batch 2

| **Conjugate** | **Drug loading (% w/w)** | **Conjugated NH₂ per molecule*** |
|---|---|---|
| OligoG-A-colistin | 13.5 | 1.53 |
| OligoG-E-colistin | 7.5 | - |
| 2.5k AHG-A-colistin | 6 | 1.72 |
| 2.5k AHG-E-colistin | 3.6 | - |
| 7.5k AHG-A-colistin | 2.4 | 2.41 |
| 7.5k AHG-E-colistin | 0.8 | - |
| 15k AHG-A-colistin | 3.2 | 2.68 |
| 15k AHG-E-colistin | 0.9 | - |
| OligoG-A-polymyxin B | 19.6 | 2.56 |
| OligoG-E- polymyxin B | 2.3 | - |
| 2.5k AHG-A- polymyxin B | 3.8 | 1.56 |
| 2.5k AHG-E- polymyxin B | 0.6 | - |
| 7.5k AHG-A- polymyxin B | 1.2 | 1.83 |
| 15k AHG-A- polymyxin B | 0.8 | 0.95 |

| | | |
|---|---|---|
| *usually 5 free NH₂ per colistin and 5 free NH₂ per polymyxin B | | |

### Batch 3

| **Conjugate** | **Drug loading (% w/w)** | **Molecular weight (g/mol)** | **PDI** | **Conjugated NH₂ per molecule*** |
|---|---|---|---|---|
| OligoG-A-colistin | 10.0 | 26,250 | 1.9 | 3.51 |
| OligoG-E-colistin (DMF)* | 7.3 | 28,750 | 1.9 | - |
| OligoG-E-colistin (DMSO)* | 10.9 | 26,000 | 1.8 | - |
| 2.5k AHG-A-colistin | 6.0 | 36,000 | 2.2 | 2.44 |
| 2.5k AHG-E-colistin | 7.6 | 37,750 | 2.2 | - |

| | | | | |
|---|---|---|---|---|
| *Conjugation was tested in anhydrous DMF and anhydrous DMSO. Drug loading and yield were higher in DMSO, but antimicrobial activity of conjugates was equivalent, therefore ester conjugates thereafter were synthesised in anhydrous DMSO. | | | | |

### Batch 4

| **Conjugate** | **Mn* (g/mol)** | **Mw* (g/mol)** | **PDI** | **Free drug (%)** | **Drug loading (% w/w)** |
|---|---|---|---|---|---|
| Colistin sulfate | 10,250 | 11,500 | 1.1 | | |
| OligoG-A-colistin | 9,750 | 25,500 | 2.6 | 5.7 | 8.7 |
| Polymyxin B | 9,500 | 10,500 | 1.1 | | |
| OligoG-A-polymyxin B | 8,750 | 23,250 | 2.7 | 1.6 | 8.0 |
| OligoG | 7,000 | 16,750 | 2.4 | | |

### Batch 5

| **Conjugate** | **Drug loading (% w/w)** |
|---|---|
| OligoG-A-colistin (1) | 12.9 |
| OligoG-A-colistin (2) | 8.4 |
| OligoG-E-colistin | 13.5 |
| OligoG-A-polymyxin B | 8.3 |
| OligoG-E-polymyxin B | 9.7 |

### Batch 6

| Compound | M_{w} (g/mol) (M_{w}/Mₙ) | Protein content (% w/w) | Conjugated NH₂ per molecule | Free protein (%) |
|---|---|---|---|---|
| OligoG-A-colistin | 25500 (2.6) | 8.7 | 2.8 | 5.7 |
| OligoG-A-colistin | 22500 (2.3) | 8.8 | 2.7 | 0.7 |
| OligoG-E-colistin | 14500 (2.3) | 12.9 | 3.0 | 2.1 |
| OligoG-A-polymyxin B | 23000 (2.7) | 8.0 | 2.0 | 1.6 |
| OligoG-A-polymyxin B | 23500 (2.2) | 6.1 | 1.9 | 2.1 |
| OligoG-E-polymyxin B | 15500 (2.1) | 7.0 | 3.2 | 2.1 |

Incubation of OligoG with varying concentrations of AlgL resulted in a concentration-dependent decrease in molecular weight (data not shown). Both ester and amide conjugates of OligoG-colistin, 16k AHG-colistin and OligoG-polymyxin B incubated in PBS at either pH 5 or pH 7 showed no significant decrease in MW (data not shown). Conjugates were slightly less stable at pH 7, compared to pH 5. Conversely, AlgL effectively triggers drug release (increase in % free drug) from these conjugates at 1 U/mL (data not shown). There was little difference in drug release from amide- and ester-linked conjugates (except 16k AHG-colistin conjugate where the ester conjugate showed a more rapid release and a greater total release).

It is expected that at sites of bacterial infection and inflammation, pH would be lower with parallel increases in the activity of ROS, esterases and alginate lyase (in bacterial infections) and hence release of drug.

### Example 3 - Antimicrobial activity

### Bacterial isolates and strains:

The strains used for susceptibility testing include both culture collection strains and clinical isolates **(Table 1).** To the extent indicated below, their known relevant genotypes and origin have been described by Khan et al. *supra.*

**Table 1: Bacterial isolates used in this study**

| **Code** | **Isolate** | **Description** |
|---|---|---|
| V1 | *Pseudomonas aeruginosa* R22 | VIM-2, China. Khan *et al., supra.* |
| V2 | *Pseudomonas aeruginosa* (301) | MDR-PSA isolate, multi drug resistant isolate defined as being resistant to piperacillin, ceftazidime, imipenem, and gentamicin. Origin; Poland. Khan *et al., supra.* |
| NH57388A | *Pseudomonas aeruginosa* | Stable mucoid cystic fibrosis isolate |
| V3 | *Klebsiella pneumoniae* | Khan *et al., supra.* |
| V4 | *Acinetobacter baumanii* MDR ACB | MDR, Libya. Khan *et al., supra.* |
| V5 | *Escherichia coli* | Khan *et al.,* supra. |
| V6 | *Klebsiella pneumoniae* IR25 | NDM-1, India. Khan *et al., supra.* |
| V7 | *Escherichia coli* | Khan *et al., supra.* |
| V11 | *Escherichia coli* | Extended spectrum beta-lactamase resistance (ESBL^{R}), Wales |
| V19 | *Acinetobacter baumannii* | Khan *et al., supra.* |
| V33 | *Burkholderia cepacia* (ATCC 25416) | Khan *et al., supra.* |
| E68 | *Staphylococcus aureus* NCTC 6571 | *Staphylococcus aureus* control Khan *et al., supra.* |
| E75 | *Staphylococcus aureus* NCTC 12493 | Methicillin-resistant *Staphylococcus aureus* (MRSA) control NCTC strain |

### Determination of antimicrobial activity

The MICs were determined using the broth microdilution method in accordance with standard guidelines (CLSI 2012). Test organisms were suspended in Mueller Hinton cation adjusted (MH) broth (100 µL, 1-5 × 10⁴ CFU/mL) and incubated in 96-well microtitre plates in serial two-fold dilutions of the test compounds. The MIC was defined as the lowest concentration of test compound that produced no visible growth after 16-20 hours. Unless otherwise stated, results show median values of 3 experiments.

To investigate whether alginate oligomer degradation is required for antimicrobial activity, MIC assays were conducted in the presence of alginate lyase (1, 10 and 50 U/mL), whereby alginate lyase was added to the MH broth during microtitre plate set up. In addition, alginate oligomer-colistin conjugates (3 mg/mL) were incubated in PBS at pH 7 containing bacterial alginate lyase (1 U/mL) at 37 °C for 24 h, before preparing microtitre plates as described above.

Finally, bacterial growth curves, using the mean of the previously determined MIC values for OligoG-colistin conjugates (amide and ester linked) and colistin sulfate, were used to compare their pharmacokinetic profiles *in vitro.* For comparison, bacterial growth in the presence of i) OligoG, ii) PRONOVA (high molecular weight alginate) and iii) OligoG plus colistin, both at concentrations corresponding to the amounts present in the corresponding conjugates, was measured. Sterile 96-well microtitre plates were set up for MIC assay as previously described (range: MIC × 16 to MIC/16, 0). Plates were then wrapped in parafilm and placed in a microtitre plate reader at 37 °C, 5 % CO₂ and absorbance at 600 nm was measured hourly for 48 h. Experiments were conducted in triplicate and results were presented as mean values (n=3).

### Results

None of the alginate fractions, or OligoG showed any antimicrobial activity, whereas unconjugated polymyxin-class antibiotics showed high antimicrobial activity. The antibacterial activity of alginate oligomer-conjugated colistin and polymyxin is preserved, and in some cases, enhanced. This is in marked contrast to previously described colistin-dextrin conjugates linked via succinyl groups where MIC values are actually worsened.

Antimicrobial activity was greatest for conjugates containing low molecular weight alginate oligomers (including OligoG) conjugated to the antibiotic via an ester bond. Conjugates showed reproducible batch variability in antimicrobial activity.

### Batch 2

*MIC values (µg*/*mL) for each compound tested against the relevant bacteria (n=3, median)*

| **Conjugate** | **Drug loading (% w/w)** | **V2** | **V3** | **V5** | **V19** | **E68** | **E75** |
|---|---|---|---|---|---|---|---|
| OligoG-A-colistin | 13.5 | 2 | 0.25 | 0.0005 | 0.25 | - | - |
| OligoG-E-colistin | 7.5 | 1 | 0.25 | 0.00003 | 0.125 | - | - |
| 2.5k AHG-A-colistin | 6 | 1 | 0.25 | 0.00006 | 0.5 | - | - |
| 2.5k AHG-E-colistin | 3.6 | 2 | 0.25 | 0.001 | 0.5 | - | - |
| 7.5k AHG-A-colistin | 2.4 | 32 | 4 | 0.25 | 8 | - | - |
| 7.5k AHG-E-colistin | 0.8 | 4 | 0.5 | 0.004 | 2 | - | - |
| 15k AHG-A-colistin | 3.2 | 16 | 2 | 0.125 | 4 | - | - |
| 15k AHG-E-colistin | 0.9 | 8 | 1 | 0.002 | 2 | - | - |
| Colistin sulfate | - | 0.25 | <0.125 | 0.00002 | <0.0625 | - | - |
| OligoG-A-polymyxin B | 19.6 | 4 | <0.125 | 0.004 | 2 | - | - |
| OligoG-E- polymyxin B | 2.3 | 0.25 | <0.125 | 0.00002 | 0.25 | - | - |
| 2.5k AHG-A- polymyxin B | 3.8 | 1 | 1 | 0.00003 | 0.5 | - | - |
| 2.5k AHG-E- polymyxin B | 0.6 | 2 | 2 | 0.002 | 1 | - | - |
| 7.5k AHG-A- polymyxin B | 1.2 | 64 | 32 | 2 | 4 | - | - |
| 15k AHG-A- polymyxin B | 0.8 | 4 | 2 | 0.031 | 1 | - | - |
| Polymyxin B | - | 0.25 | <0.125 | <0.000008 | <0.0625 | - | - |

### Batch 3

*MIC values (µg*/*mL) for each compound tested against the relevant bacteria (n=3, median)*

| **Conjugate** | **Drug loading (% w/w)** | **V2** | **V3** | **V5** | **V19** |
|---|---|---|---|---|---|
| OligoG-A-colistin | 10.0 | 1 | 0.063 | 0.00002 | 0.063 |
| OligoG-E-colistin (DMF)* | 7.3 | 0.5 | 0.125 | 0.00003 | 0.125 |
| OligoG-E-colistin (DMSO)* | 10.9 | 0.5 | 0.125 | 0.000008 | 0.125 |
| 2.5k AHG-A-colistin | 6.0 | 0.5 | 0.125 | 0.00002 | 0.125 |
| 2.5k AHG-E-colistin | 7.6 | 0.5 | 0.063 | 0.0001 | 0.125 |

### Batch 4

*MIC values (µg*/*mL) for each compound tested against the relevant bacteria (n=3, median)*

| **Conjugate** | **Drug loading (% w/w)** | **V1** | **V2** | **V3** | **V4** | **V5** | **V6** | **V7** | **V19** |
|---|---|---|---|---|---|---|---|---|---|
| OligoG-A-colistin | 8.7 | 1 | 1 | 0.25 | 1 | <0.016 | 1 | 0.5 | 0.5 |
| Colistin sulfate | | 0.25 | 0.25 | <0.063 | 0.5 | <0.016 | 0.0625 | 0.125 | <0.016 |
| OligoG-A-polymyxin B | 8.0 | 4 | 4 | 1 | 2 | <0.125 | 4 | 2 | 2 |
| Polymyxin B | | 0.25 | 0.5 | 0.125 | 0.125 | <0.016 | 0.125 | 0.063 | 0.031 |

### Batch 6

### MIC values (µg/mL) for each compound tested against the relevant bacteria (n=3, median)

| **Conjugate** | **V1** | **V2** | **NH57-388A** | **V3** | **V6** | **V4** | **V19** | **V5** | **V7** | **V11** |
|---|---|---|---|---|---|---|---|---|---|---|
| OligoG-A-colistin | 2 | 1 | 0.5 | 0.125 | 1 | 1 | 0.125 | 0.008 | 1 | 0.063 |
| OligoG-E-colistin | 1 | 0.5 | 0.25 | 0.125 | - | - | 0.125 | 0.008 | 0.25 | 0.031 |
| Colistin sulfate | 0.5 | 0.5 | 0.25 | 0.125 | 0.063 | 0.5 | 0.25 | <0.008 | 0.25 | 0.031 |
| OligoG-A-polymyxin B | 4 | 2 | 1 | 0.5 | 4 | 2 | 0.5 | 0.063 | 2 | 0.25 |
| OligoG-E-polymyxin B | - | 0.5 | 0.25 | 0.25 | - | - | 0.5 | 0.016 | 0.5 | 0.063 |
| Polymyxin B | 0.25 | 0.5 | 0.25 | 0.125 | 0.125 | 0.125 | 0.125 | <0.004 | 0.5 | 0.063 |

When the antimicrobial activity of the conjugates was assessed in the presence of AlgL or following pre-incubation with AlgL (Batch 3), no significant change was observed for either amide- or ester-bonded conjugates (data not shown). These results suggest that either OligoG degradation is not necessary for antibiotic activity, or that alginate oligomers are broken down by bacterial enzymes or those contained in the culture broth.

Bacterial growth curves **(****Figs. 1-5****)** showed that alginate oligomer-colistin conjugates (Batch 2) delayed bacterial growth in a concentration-dependent manner, and slowed the rate of regrowth compared to untreated bacteria. On the other hand colistin sulfate alone showed more rapid bacterial regrowth. Overall, at the median of previously determined MIC values across all batches, OligoG-colistin conjugates typically showed the longest inhibition of bacterial re-growth, while colistin sulfate showed the shortest inhibition of bacterial re-growth (indeed, equivalent to the no antibiotic control for V3 and V19). OligoG-colistin conjugates inhibited bacterial re-growth for up to 48 h at ≥ 2x MIC, while colistin sulfate inhibits bacterial re-growth for up to 48 h at ≥ 8x MIC (i.e. higher equivalent concentrations of colistin were required to inhibit growth for as long as OligoG-colistin conjugate).

There was no difference in time to onset of bacterial regrowth between OligoG-colistin conjugates and 2.5k alginate oligomer-colistin conjugates, nor was there a difference between amide- and ester-linked conjugates. Altogether, this might suggest a more stable and controlled colistin release from the conjugates which prolongs the antibacterial effects of the colistin.

The combination of OligoG plus colistin at equivalent concentrations to the OligoG and colistin in the corresponding conjugates showed concentration-dependent growth inhibition. Typically, colistin that was covalently conjugated to OligoG showed equivalent activity to unconjugated colistin plus OligoG at equivalent concentrations. OligoG or Pronova, at an equivalent concentration to the OligoG in the OligoG-colistin conjugates (amide- and ester-linked), had no significant effect in reducing bacterial growth.

### Example 4 - Pharmacokinetic/pharmacodynamic modeling of OligoG-polymyxin conjugates

Studies were undertaken to investigate the PK/PD profiles of the OligoG-colistin conjugates (Batch 6) as compared to non-conjugated colistin *in vitro.* This is particularly important in nanoscale drug delivery systems where the incorporation of conventional small molecule drugs into nano-sized structures is associated with PK/PD properties that are substantially altered from the original drug. The model system previously described in Azzopardi (supra) was employed.

The unmasking of OligoG-colistin conjugates was simulated using the Azzopardi two-compartment static dialysis bag model under infinite sink conditions (total volume, 20 ml; inner-compartment volume, 5 ml). Dialysis membrane (10,000 g/mol molecular weight cutoff [MWCO]) was pre-soaked for 15 to 30 min in distilled water (dH₂O), secured with dialysis clips, and suspended from an injection port to separate the inner compartment (IC) from the outer compartment (OC) in a sterilized 25-ml beaker sealed with a sterile medical-grade polyurethane membrane (Tegaderm).

The model system was prepared under aseptic conditions in a class 2 laminar airflow cabinet and transferred to a shaking incubator set at 37°C in ambient air and constant orbital agitation at 70 rpm for 48 h. At 0 h, *Acinetobacter baumanii* (V19) in Mueller-Hinton broth (5 × 10⁵ CFU/ml) was transferred to the OC and the IC was loaded with the agent under test (Batch 6) in PBS at previously determined MIC or double said MIC (Example 3; Batch 6 - colistin MIC: 0.25 µg/ml; OligoG-E-colistin MIC: 0.125 µg/ml; OligoG-A-colistin MIC: 0.125 µg/ml). Samples were collected from the OC at various time points and characterised by bacterial colony counts (CFU/ml) using Miles and Misra Method.

### Results

Results are shown in **Figure 6****.** The PK/PD model demonstrated that colistin sulfate, at its previously determined MIC (0.25 µg/ml) and OligoG-ester-colistin conjugate at 2x MIC (0.25 µg/ml) reduced viable bacterial counts (~2 fold) after 4 h and overall the early PK/PD profiles are essentially the same. No significant activity was observed with OligoG-amide-colistin conjugates at MIC and 2x MIC. These results demonstrate the ability of the ester linked conjugates, but not the amide linked conjugates, to diffuse through the dialysis membrane and exert activity in the outer membrane compartment to an extent and with a time course which is similar to non-conjugated colistin. The lack of activity observed with the more stable amide linked conjugates suggests that these are being retained in the IC whereas the more labile ester linked conjugates are rapidly degrading thereby allowing the unmasked colistin to diffuse out of the IC and exert its antibacterial effects on the bacteria in the OC.

### Example 5 - Measurement of in vitro cytotoxicity: MTT assay

An MTT assay was used to assess cell viability in a human kidney (HK-2) cell line (72 h incubation). HK-2 cells were seeded into sterile 96-well microtitre plates (1 × 10⁵ cells/mL) in 0.1 mL/well of media (K-SFM) containing L-glutamine, EGF and BPE. They were allowed to adhere for 24 h. The medium was then removed and a range of concentrations of test compounds (0.2 µm filter-sterilized) dissolved in fresh culture media were added to the cells. After a further 67 h incubation, MTT, 3-(4,5-dimethylthiazol-2-yl)-2,5-diphenyltetrazolium bromide, (20 µL of a 5 mg/mL solution in PBS) was added to each well and the cells were incubated for a further 5 h. The medium was then removed and the precipitated formazan crystals solubilized by addition of optical grade DMSO (100 µL) over 30 min. Absorbance was measured at 540 nm using a microtitre plate reader. Cell viability was expressed as a percentage of the viability of untreated control cells. The IC₅₀ values were expressed as mean (n=18).

### Results

OligoG conjugation markedly decreased the toxicity of colistin and polymyxin B **(****Fig. 7****).** The IC₅₀ values summarised below represent the concentration of the antibiotic (polymyxin B, colistin sulphate or colistin methanesulphonate (CMS)) or the antibiotic within the conjugate required to inhibit HK2 cells growth by half. OligoG is included as a control.

| | ***Batch 5*** | | | ***Batch 4*** | | |
|---|---|---|---|---|---|---|
| **Drug** | **Drug loading (% w/w)** | **IC₅₀ (mg/mL)** | ***Fold-change*** | **Drug loading (% w/w)** | **IC₅₀ (mg/mL)** | **Fold-chang e** |
| OligoG | | > 50 | | | >10 | |
| | | | | | | |
| Colistin sulfate | | 0.01098 | | | 0.025 | |
| CMS | | 0.01397 | | | | |
| OligoG-A-colistin (1) | 12.9 | 0.6017 | 55 | 8.7 | 0.22 | 9 |
| OligoG-A-colistin (2) | 8.4 | 0.04393 | 4 | | | |
| OligoG-E-colistin | 13.5 | 0.05687 | 5 | | | |
| | | | | | | |
| Polymyxin B | | 0.01263 | | | 0.012 | |
| OligoG-A-polymyxin B | 8.3 | 0.03886 | 3 | 8.0 | 0.35 | 29 |
| OligoG-E-polymyxin B | 9.7 | 0.03175 | 2.5 | | | |

### Example 6 - Measurement of in vitro cytotoxicity: TNFα release from HK2 cells

As a model inflammatory cytokine involved in polymyxin cyto toxicity,TNFα release from HK2 cells in response to polymyxins and OligoG-polymyxin conjugates was measured by ELISA. HK-2 cells were seeded into sterile 96-well microtitre plates (1 × 10⁵ cells/mL) in 0.1 mL/well of media (K-SFM) containing L-glutamine, EGF and BPE. They were allowed to adhere for 24 h. The medium was then removed and a range of concentrations of test compounds (0.2 µm filter-sterilized) dissolved in fresh culture media were added to the cells. After 72 h incubation, microtiter plates were centrifuged (1500 rpm, 3 min) and the supernatant was transferred into a new 96-well microtitre plate.

TNFα ELISA Assay was carried out according the manufacturer's instructions (Thermo Scientific, ESS0001). Briefly, 100 µL of coating antibody (1:100 dilution in PBS) was added to each well of 96-well microtitre plate. Plate was covered with plastic seal and incubated overnight at room temperature (22-25°C). Coating antibody solution was aspirated and 300 µL of blocking buffer (4% BSA, 5% sucrose in PBS) was added to each well and then incubated for 1 h at room temperature (22-25°C) and aspirated. Standards were reconstituted with reagent diluent (4% BSA in PBS, pH 7.4) according vial label and diluted 1:2 to prepare top standard concentration (1000 pg/mL). Serial two-fold dilutions were performed in duplicate across rows A & B of 96-well plate (0, 3.9-1000 pg/mL). The supernatant of HK-2 cells (diluted 1:1 in reagent diluent, 100 µL) was added to each well of 96-well microtitre plate and incubated overnight at room temperature (22-25°C). Solution was aspirated and plate was washed three times with wash buffer (0.05% Tween-20 in PBS, pH 7.4) using 300 µL/well. Then, 100 µL of detection antibody (1:100 dilution in reagent diluent) was added to each well. Plate was incubated for 1 h at room temperature (22-25°C) and washed three times with wash buffer, using 300 µL/well. Next, 100 µL of Streptavidin-HRP (1:400 dilution in reagent diluent) was added to each well and incubated for 30 min at room temperature (22-25°C) and then washed three times with wash buffer, using 300 µL/well. Substrate solution (100 µL) was added to each well of 96-well microtitre plate and incubated in the dark for 20 min at room temperature (22-25°C). The reaction was stopped by adding 100 µL of stop solution to each well. Absorbance was measured at 450 nm minus 550 nm.

Standard curves were generated by plotting the average absorbance obtained for each standard concentration on the vertical (Y) axis versus the corresponding human TNFα concentration (pg/mL) on the horizontal (X) axis. Human TNFα amount in each sample was determined by interpolating from the absorbance value (Y-axis) to human TNFα concentration (X-axis) using the standard curve. Interpolated value obtained from the standard curve was multiplied by the dilution factor (x2) and adjusted to cell viability for each drug concentration (MTT assay results) to calculate pg/mL of human TNFα in the sample.

### Results

Results are shown in **Figure 8****.** As can be seen, polymyxin B and colistin induced greater TNFα release compared to the conjugates where TNFα release was very low or undetectable, except for OligoG-E-polymyxin B, which showed similar effects to colistin but less than polymixin B. Overall, OligoG conjugation markedly decreased the release of inflammatory cytokines by the polymixin class antibiotics.

### Example 7 - Disruption of biofilm development

### Methods

These experiments investigated whether OligoG-colistin conjugates could inhibit biofilm development more effectively than unconjugated forms of colistin. First, stock solutions of the test compounds were prepared at 1 mg/mL in MH broth and used to prepare a range of antibiotic concentrations (2x MIC - MIC/8) using double dilutions in MH broth across wells of a Greiner glass-bottomed optical 96-well plate (90 µL per well). Sterility (100 µL of MH broth) and growth (90 µL of MH broth plus 10 µL of tested bacterial pathogen) controls were also prepared. Overnight bacterial cultures were grown in TS broth (TSB) and diluted in MH broth to achieve an OD₆₂₅ of 0.5, then 10 µL was added to the wells. Plates were wrapped in parafilm and incubated for 24 h on a rocker (20 rpm) at 37°C. After a 24 h incubation, the supernatant was carefully removed from all wells and 3 µL of live/dead stain (Live/dead Baclight Bacterial Viability kit; prepared by mixing 1 µL of component A and B in 1 mL of PBS) was added. The plate was wrapped in foil and kept in the dark for 15 min. Lastly, 47 µL of PBS was added to all tested wells. The plate was wrapped in parafilm and kept in the dark until it was analysed by confocal laser scanning microscopy (CLSM), n=2.

### Results

OligoG-colistin conjugate (Batch 5, OligoG-A-colistin (1)) markedly disrupted formation of *P. aeruginosa* biofilms and induced bacterial death at 2 × MIC (2 µg/mL) **(****Fig. 9****).** Even at its MIC (1 µg/mL) OligoG-colistin conjugate caused bacterial clumping and a disorganised biofilm structure, with the presence of dead cells. Colistin sulfate, on the other hand, showed only a small effect on *P. aeruginosa* biofilms, with a very slight change in biofilm thickness at the highest concentration, but very little cell death. These results indicate that OligoG-colistin conjugates are significantly more effective that colistin in disrupting a developing biofilm and killing the resident bacteria. Thus they could be considered as antibacterial agents of utility in the combat of biofilm infections

## Claims

1. A polymyxin-alginate oligomer conjugate comprising a polymyxin-class antibiotic connected covalently to at least one alginate oligomer via a direct covalent bond or a covalent molecular linker, wherein said alginate oligomer has 2 to 75 monomer residues, or a pharmaceutically acceptable salt, solvate, hydrate, diastereoisomer, tautomer, or enantiomer thereof.

2. The polymyxin-alginate oligomer conjugate of claim 1, wherein said polymyxin-class antibiotic is
(i) selected from polymyxins A1, A2, B1, B1-I, B2, B3, B4, B5, B6, C, D1, D2, E1, E2, F, K1, K2, M, P1, P2, S and T and functionally equivalent derivatives thereof;
(ii) represented by Formula II 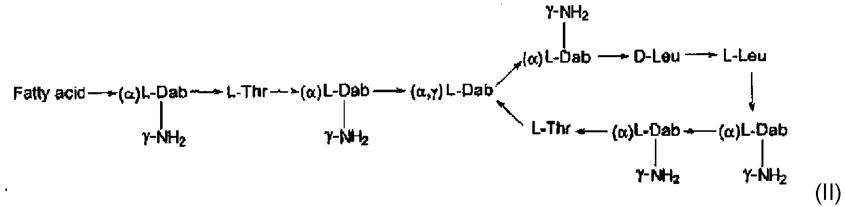 wherein D-Leu is D-leucine; L-Leu is L-leucine; L-Thr is L-threonine and L-Dab is α,γ-diaminobutyric acid and wherein none, or one or more, thereof is replaced by another amino acid residue which may be selected from natural or non-genetically encoded amino acids, preferably wherein said natural or non-genetically encoded amino acid is selected from leucine, threonine, acid, phenylalanine, arginine, histidine, lysine, asparagine, serine, cysteine, homolysine, ornithine, diaminobutyric acid (e.g. α,γ-diaminobutyric acid), diaminopimelic acid, diaminopropionic acid, homoarginine, trimethylysine, trimethylornithine, 4-aminopiperidine-4-carboxylic acid, 4-amino-1-carbamimidoylpiperidine-4-carboxylic acid and 4-guanidinophenylalanine; and/or
(iii) is selected from polymyxin B1, B1-I, B2, E1 or E2.

3. The polymyxin-alginate oligomer conjugate of claim 1 or claim 2, wherein the alginate oligomer has a degree of polymerisation (DP), or a number average degree of polymerisation (DPn) of
(i) 4 to 75, 4 to 50, 4 to 35, 4 to 30, 4 to 25, 4 to 22, 4 to 20, 4 to 18, 4 to 16 or 4 to 14,
(ii) 6 to 50, 6 to 35, 6 to 30, 6 to 25, 6 to 22, 6 to 20, 6 to 18, 6 to 16 or 6 to 14,
(iii) 8 to 50, 8 to 35, 8 to 30, 8 to 25, 10 to 25, 10 to 22, 10 to 20, 10 to 18, or 10 to 15, or
(iv) 20 to 75, 20 to 70, 20 to 65, 20 to 60 , 20 to 55, 20 to 50, 20 to 45, 20 to 40, 20 to 35, 20 to 30 or 20 to 25, or
(v) 30 to 75, 30 to 70, 30 to 65, 30 to 60, 30 to 55, 30 to 50, 30 to 45, 30 to 40 or 30 to 35.

4. The polymyxin-alginate oligomer conjugate of any one of claims 1 to 3, wherein the alginate oligomer has at least 70% G residues, or at least 80%, or at least 85%, or at least 90%, or at least 95% G residues, preferably wherein at least 80% of the G residues are arranged in G-blocks.

5. The polymyxin-alginate oligomer conjugate of any one of claims 1 to 3, wherein the alginate oligomer has at least 70% M residues, or at least 80%, or at least 85%, or at least 90%, or at least 95% M residues, preferably wherein at least 80% of the M residues are arranged in M-blocks.

6. The polymyxin-alginate oligomer conjugate of any one of claims 1 to 5, wherein said direct covalent bond is part of an ester, carbonate ester, orthoester, ketal, hemiketal,, ether, acetal, hemiacteal, peroxy, methylenedioxy, amide, amine, imine, imide, azide, azo, oxime, sulfide, disulfide, sulfinyl, sulfonyl, carbonothioyl, thioester, phosphine or phosphodiester functional group, preferably wherein said direct covalent bond is part of an ester or an amide.

7. The polymyxin-alginate oligomer conjugate of any one of claims 1 to 5, wherein said covalent linker is or comprises molecular groups selected from:
(i) an amino acid or a peptide;
(ii) monosaccharide or an oligosaccharide other than guluronate or mannuronate or polymers formed therefrom;
(iii) a ribonucleotide or a deoxyribonucleotide;
(iv) a straight chain, branched or cyclic, substituted or unsubstituted, alkyl, alkenyl or alkynl group;
(v) an acetyl, succinyl, aconityl (*cis* or *trans*), glutaryl, methylsuccinyl, trimellityl cysteamine, penicillamine, N-(2-mercaptopropionyl)glycine, 2-mercaptopropionic acid, homocysteine, 3-mercaptopropionic acid or deamino-penicillamine group.

8. The polymyxin-alginate oligomer conjugate of any one of claims 1 to 7, wherein said direct covalent bond, a functional group containing said covalent bond or said covalent molecular linker is
(i) acid labile;
(ii) sensitive to reactive oxygen species; and/or
(iii) degraded by an enzyme secreted by a bacterium or an immune cell.

9. The polymyxin-alginate oligomer conjugate of any one of claims 1 to 8, wherein said conjugate consists of
(i) at least one alginate oligomer covalently bonded to a polymyxin-class antibiotic via an ester bond formed from a carboxyl group on the alginate and hydroxyl group on the polymyxin, or
(ii) at least one alginate oligomer covalently bonded to a polymyxin-class antibiotic via an amide bond formed from a carboxyl group on the alginate and an amine group on the polymyxin.

10. The polymyxin-alginate oligomer conjugate of claim 9, wherein the polymyxin-class antibiotic is a colistin (e.g. polymyxin E1 or E2) or a polymyxin B (e.g. polymyxin B1, B1-I or B2).

11. A pharmaceutical composition comprising a polymyxin-alginate oligomer conjugate as defined in any one of claims 1 to 10 and a pharmaceutically acceptable excipient, carrier or diluent.

12. A method for the preparation of a polymyxin-alginate oligomer as defined in any one of claims 1 to 10, said method comprising
(ia) providing an alginate oligomer, wherein said alginate oligomer has 2 to 75 monomer residues, and a polymyxin-class antibiotic and forming a direct covalent bond between two molecular groups thereon; or
(ib) providing an alginate oligomer, wherein said alginate oligomer has 2 -75 monomer residues, a polymyxin-class antibiotic and a covalent molecular linker and forming a direct covalent bond between two molecular groups on the alginate oligomer and the linker molecule and forming a direct covalent bond between two molecular groups on the polymyxin-class antibiotic and the linker molecule; or
(ic) providing an alginate oligomer, wherein said alginate oligomer has 2 -75 monomer residues, and a polymyxin-class antibiotic wherein one or both carry a covalent molecular linker molecule covalently bonded thereto and covalently linking the alginate oligomer to the polymyxin-class antibiotic via at least one of the linker molecules; and optionally
(ii) separating at least a portion of the polymyxin-alginate oligomer conjugate from the reaction mixture.

13. The method of claim 12, said method comprising
(i) providing an aqueous solution of an alginate oligomer having an available carboxyl group, wherein said alginate oligomer has 2 to 75 monomer residues;
(ii) contacting said alginate solution with 1-ethyl-3-[3-dimethylaminopropyl]carbodiimide hydrochloride (EDC) in an amount and under conditions sufficient to activate at least one carboxyl group in the alginate oligomer;
(iii) optionally contacting said carboxyl activated alginate oligomer with sulfo N-hydroxysuccinimide (sulfo-NHS) in an amount and under conditions sufficient to form an amine-reactive sulfo-NHS ester;
(iv) contacting said carboxyl activated alginate oligomer of step (ii) or the amine-reactive sulfo-NHS ester of step (iii) with an polymyxin-class antibiotic having an available primary amine group in an amount and under conditions sufficient to form an amide bond between the alginate oligomer and the polymyxin-class antibiotic; and
(v) separating at least a portion of the polymyxin-alginate oligomer conjugate from the reaction mixture.

14. The method of claim 12, said method comprising
(i) providing a solution of an alginate oligomer having an available carboxyl group, wherein said alginate oligomer has 2 to 75 monomer residues, preferably an organic (e.g. DMF and/or DMSO) solution;
(ii) contacting said alginate solution with dicyclohexylcarbodiimide (DCC) in an amount and under conditions sufficient to form an O-acylisourea intermediate;
(iii) contacting said O-acylisourea intermediate with an polymyxin-class antibiotic having an available hydroxyl group and 4-N,N-dimethylaminopyridine (DMAP) in amounts and under conditions sufficient to form an ester bond between the alginate oligomer and the polymyxin-class antibiotic; and
(iv) separating at least a portion of the polymyxin-alginate oligomer conjugate from the reaction mixture;
wherein steps (ii) and (iii) may be performed simultaneously.

15. A polymyxin-alginate oligomer conjugate as defined in any one of claims 1 to 10 or a pharmaceutical composition as defined in claim 11 for use in the treatment of prevention of a bacterial infection, preferably wherein the bacterial infection is
(i) a systemic infection or an infection of multiple loci within or on the subject;
(ii) a respiratory infection in a subject suffering from an underlying respiratory disorder or condition, preferably selected from CF, COPD/COAD, or asthma;
(iii) a device related infection associated with implantable or prosthetic medical devices;
(iv) in a chronic wound; or
(v) a Gram negative bacterial infection.

## Patentansprüche

1. Polymyxin-Alginat-Oligomer-Konjugat, umfassend ein Antibiotikum der Polymyxin-Klasse, das über eine direkte kovalente Bindung oder einen kovalenten molekularen Linker kovalent an mindestens ein Alginat-Oligomer gebunden ist, wobei das Alginat-Oligomer 2 bis 75 Monomerreste aufweist, oder ein pharmazeutisch akzeptables Salz, Solvat, Hydrat, Diastereoisomer, Tautomer oder Enantiomer davon.

2. Polymyxin-Alginat-Oligomer-Konjugat nach Anspruch 1, wobei das Antibiotikum der Polymyxin-Klasse
(i) aus Polymyxinen A1, A2, B1, B1-I, B2, B3, B4, B5, B6, C, D1, D2, E1, E2, F, K1, K2, M, P1, P2, S und T und funktionell äquivalenten Derivaten davon ausgewählt ist;
(ii) durch Formel II dargestellt ist 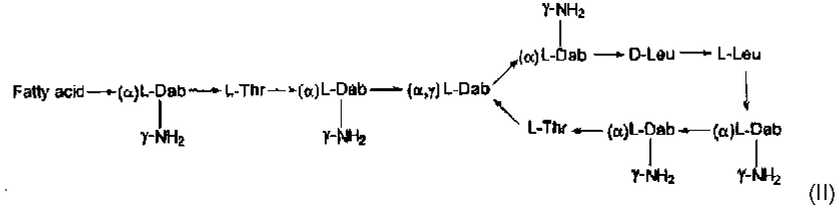 wobei D-Leu D-Leucin ist; L-Leu L-Leucin ist; L-Thr L-Threonin ist und L-Dab α,γ-Diaminobuttersäure ist und wobei keine oder eine oder mehrere davon durch einen anderen Aminosäurerest ersetzt ist, der aus natürlichen oder nicht genetisch kodierten Aminosäuren ausgewählt sein kann, bevorzugt wobei die natürliche oder nicht genetisch kodierte Aminosäure aus Leucin, Threonin, Säure, Phenylalanin, Arginin, Histidin, Lysin, Asparagin, Serin, Cystein, Homolysin, Ornithin, Diaminobuttersäure (z. B. α,γ-Diaminobuttersäure), Diaminopimelinsäure, Diaminopropionsäure, Homoarginin, Trimethylysin, Trimethylornithin, 4-Aminopiperidin-4-Carbonsäure, 4-Amino-1-Carbamimidoylpiperidin-4-Carbonsäure und 4-Guanidinophenylalanin ausgewählt ist; und/oder
(iii) aus Polymyxin B1, B1-I, B2, E1 oder E2 ausgewählt ist.

3. Polymyxin-Alginat-Oligomer-Konjugat nach Anspruch 1 oder Anspruch 2, wobei das Alginat-Oligomer einen Polymerisationsgrad (DP) oder einen zahlenmittleren Polymerisationsgrad (DPn) aufweist von
(i) 4 bis 75, 4 bis 50, 4 bis 35, 4 bis 30, 4 bis 25, 4 bis 22, 4 bis 20, 4 bis 18, 4 bis 16 oder 4 bis 14,
(ii) 6 bis 50, 6 bis 35, 6 bis 30, 6 bis 25, 6 bis 22, 6 bis 20, 6 bis 18, 6 bis 16 oder 6 bis 14,
(iii) 8 bis 50, 8 bis 35, 8 bis 30, 8 bis 25, 10 bis 25, 10 bis 22, 10 bis 20, 10 bis 18 oder 10 bis 15 oder
(iv) 20 bis 75, 20 bis 70, 20 bis 65, 20 bis 60, 20 bis 55, 20 bis 50, 20 bis 45, 20 bis 40, 20 bis 35, 20 bis 30 oder 20 bis 25 oder
(v) 30 bis 75, 30 bis 70, 30 bis 65, 30 bis 60, 30 bis 55, 30 bis 50, 30 bis 45, 30 bis 40 oder 30 bis 35.

4. Polymyxin-Alginat-Oligomer-Konjugat nach einem der Ansprüche 1 bis 3, wobei das Alginat-Oligomer mindestens 70% G-Reste oder mindestens 80% oder mindestens 85% oder mindestens 90% oder mindestens 95% G-Reste aufweist, bevorzugt wobei mindestens 80% der G-Reste in G-Blöcken angeordnet sind.

5. Polymyxin-Alginat-Oligomer-Konjugat nach einem der Ansprüche 1 bis 3, wobei das Alginat-Oligomer mindestens 70% M-Reste oder mindestens 80% oder mindestens 85% oder mindestens 90% oder mindestens 95% M-Reste aufweist, bevorzugt wobei mindestens 80% der M-Reste in M-Blöcken angeordnet sind.

6. Polymyxin-Alginat-Oligomer-Konjugat nach einem der Ansprüche 1 bis 5, wobei die direkte kovalente Bindung Teil einer funktionellen Gruppe der Ester, Carbonatester, Orthoester, Ketale, Hemiketale, Ether, Acetale, Hemiacetale, Peroxide, Methylendioxide, Amide, Amine, Imine, Imide, Azide, Azo-Verbindungen, Oxime, Sulfide, Disulfide, Sulfinylgruppe, Sulfonylgruppe, Carbonothioylgruppe, Thioester, Phosphine oder Phosphodiester ist, vorzugsweise wobei die direkte kovalente Bindung Teil eines Esters oder eines Amids ist.

7. Polymyxin-Alginat-Oligomer-Konjugat nach einem der Ansprüche 1 bis 5, wobei der kovalente Linker molekulare Gruppen ist oder umfasst, die ausgewählt sind aus:
(i) einer Aminosäure oder einem Peptid;
(ii) Monosacchariden oder einer der Oligosaccharide außer Guluronaten oder Mannuronaten oder daraus gebildeten Polymeren;
(iii) einer der Ribonukleotide oder einer der Desoxyribonukleotide;
(iv) einer geradkettigen, verzweigten oder cyclischen, substituierten oder unsubstituierten Alkyl-, Alkenyl- oder Alkinylgruppe;
(v) einer Acetyl-, Succinyl-, Aconityl- (*cis- oder trans*-)*,* Glutaryl-, Methylsuccinyl-, Trimellityl-Cysteamin-, Penicillamin-, N-(2-Mercaptopropionyl)-Glycin-, 2-Mercaptopropionsäure-, Homocystein-, 3-Mercaptopropionsäure- oder Deamino-Penicillamin-Gruppe.

8. Polymyxin-Alginat-Oligomer-Konjugat nach einem der Ansprüche 1 bis 7, wobei die direkte kovalente Bindung, eine funktionelle Gruppe, die die kovalente Bindung enthält, oder der kovalente molekulare Linker
(i) säurelabil;
(ii) empfindlich gegenüber reaktiven Sauerstoffspezies; und/oder
(iii) durch ein Enzym, das von einem Bakterium oder einer Immunzelle abgesondert wird, abgebaut ist.

9. Polymyxin-Alginat-Oligomer-Konjugat nach einem der Ansprüche 1 bis 8, wobei das Konjugat aus
(i) mindestens einem Alginat-Oligomer, das kovalent an ein Antibiotikum der Polymyxin-Klasse über eine Esterbindung gebunden ist, die aus einer Carboxylgruppe auf dem Alginat und einer Hydroxylgruppe auf dem Polymyxin gebildet ist, oder
(ii) mindestens einem Alginat-Oligomer, das kovalent an ein Antibiotikum der Polymyxin-Klasse über eine Amidbindung gebunden ist, die aus einer Carboxylgruppe auf dem Alginat und einer Amingruppe auf dem Polymyxin gebildet ist, besteht.

10. Polymyxin-Alginat-Oligomer-Konjugat nach Anspruch 9, wobei das Antibiotikum der Polymyxin-Klasse ein Colistin (z. B. Polymyxin E1 oder E2) oder ein Polymyxin B (z. B. Polymyxin B1, B1-I oder B2) ist.

11. Pharmazeutische Zusammensetzung, umfassend ein Polymyxin-Alginat-Oligomer-Konjugat wie in einem der Ansprüche 1 bis 10 definiert und einen pharmazeutisch akzeptablen Hilfsstoff oder Träger oder ein pharmazeutisch akzeptables Verdünnungsmittel.

12. Verfahren zur Zubereitung eines Polymyxin-Alginat-Oligomers wie in einem der Ansprüche 1 bis 10 definiert, wobei das Verfahren umfasst
(ia) Bereitstellen eines Alginat-Oligomers, wobei das Alginat-Oligomer 2 bis 75 Monomerreste aufweist, und eines Antibiotikums der Polymyxin-Klasse und Bilden einer direkten kovalenten Bindung zwischen zwei molekularen Gruppen darauf; oder
(ib) Bereitstellen eines Alginat-Oligomers, wobei das Alginat-Oligomer 2 bis 75 Monomerreste, ein Antibiotikum der Polymyxin-Klasse und einen kovalenten molekularen Linker aufweist, und Bilden einer direkten kovalenten Bindung zwischen zwei molekularen Gruppen auf dem Alginat-Oligomer und dem Linker-Molekül und Bilden einer direkten kovalenten Bindung zwischen zwei molekularen Gruppen auf dem Antibiotikum der Polymyxin-Klasse und dem Linker-Molekül; oder
(ic) Bereitstellen eines Alginat-Oligomers, wobei das Alginat-Oligomer 2 bis 75 Monomerreste aufweist, und eines Antibiotikums der Polymyxin-Klasse, wobei eines oder beide ein kovalentes molekulares Linker-Molekül tragen, das kovalent daran gebunden ist und das Alginat-Oligomer über mindestens eines der Linker-Moleküle kovalent an das Antibiotikum der Polymyxin-Klasse bindet; und optional
(ii) Ausscheiden mindestens eines Teils des Polymyxin-Alginat-Oligomer-Konjugats aus dem Reaktionsgemisch.

13. Verfahren nach Anspruch 12, wobei das Verfahren umfasst
(i) Bereitstellen einer wässrigen Lösung eines Alginat-Oligomers mit einer verfügbaren Carboxylgruppe, wobei das Alginat-Oligomer 2 bis 75 Monomerreste aufweist;
(ii) Inkontaktbringen der Alginatlösung mit 1-Ethyl-3-[3-Dimethylaminopropyl]Carbodiimid-Hydrochlorid (EDC) in einer Menge und unter Bedingungen, die ausreichen, um mindestens eine Carboxylgruppe in dem Alginat-Oligomer zu aktivieren;
(iii) optional Inkontaktbringen des carboxylaktivierten Alginat-Oligomers mit Sulfo-N-Hydroxysuccinimid (Sulfo-NHS) in einer Menge und unter Bedingungen, die zur Bildung eines aminreaktiven Sulfo-NHS-Esters ausreichen;
(iv) Inkontaktbringen des carboxylaktivierten Alginat-Oligomers aus Schritt (ii) oder des aminreaktiven Sulfo-NHS-Esters aus Schritt (iii) mit einem Antibiotikum der Polymyxin-Klasse mit einer verfügbaren primären Amingruppe in einer Menge und unter Bedingungen, die zur Bildung einer Amidbindung zwischen dem Alginat-Oligomer und dem Antibiotikum der Polymyxin-Klasse ausreichen; und
(v) Ausscheiden mindestens eines Teils des Polymyxin-Alginat-Oligomer-Konjugats aus dem Reaktionsgemisch.

14. Verfahren nach Anspruch 12, wobei das Verfahren umfasst
(i) Bereitstellen einer Lösung eines Alginat-Oligomers mit einer verfügbaren Carboxylgruppe, wobei das Alginat-Oligomer 2 bis 75 Monomerreste aufweist, bevorzugt einer organischen (z. B. DMF- und/oder DMSO-) Lösung;
(ii) Inkontaktbringen der Alginatlösung mit Dicyclohexylcarbodiimid (DCC) in einer Menge und unter Bedingungen, die zur Bildung eines O-Acylisoharnstoff-Zwischenprodukts ausreichen;
(iii) Inkontaktbringen des O-Acylisoharnstoff-Zwischenprodukts mit einem Antibiotikum der Polymyxin-Klasse mit einer verfügbaren Hydroxylgruppe und 4-N,N-Dimethylaminopyridin (DMAP) in Mengen und unter Bedingungen, die zur Bildung einer Esterbindung zwischen dem Alginat-Oligomer und dem Antibiotikum der Polymyxin-Klasse ausreichen; und
(iv) Ausscheiden mindestens eines Teils des Polymyxin-Alginat-Oligomer-Konjugats aus dem Reaktionsgemisch;
wobei die Schritte (ii) und (iii) gleichzeitig durchgeführt werden können.

15. Polymyxin-Alginat-Oligomer-Konjugat wie in einem der Ansprüche 1 bis 10 definiert oder pharmazeutische Zusammensetzung wie in Anspruch 11 definiert zur Verwendung bei der Behandlung einer Vorbeugung gegen eine bakterielle Infektion, wobei bevorzugt die bakterielle Infektion
(i) eine systemische Infektion oder eine Infektion an mehreren Stellen in dem oder an dem Patienten;
(ii) eine Atemwegsinfektion bei einem Patienten, der an einer zugrundeliegenden Atemwegsstörung oder einem zugrundeliegenden Atemwegszustand, bevorzugt aus CF, COPD/COAD oder Asthma ausgewählt, leidet;
(iii) eine gerätebezogene Infektion im Zusammenhang mit implantierbaren oder prothetischen medizinischen Geräten;
(iv) in einer chronischen Wunde; oder
(v) eine gramnegative bakterielle Infektion ist.

## Revendications

1. Conjugué oligomère d'alginate-polymyxine comprenant un antibiotique de classe polymyxine relié de manière covalente à au moins un oligomère d'alginate par le biais d'une liaison covalente directe ou d'un lieur moléculaire covalent, dans lequel ledit oligomère d'alginate présente 2 à 75 résidus monomères ou un sel pharmaceutiquement acceptable, un solvate, un hydrate, un diastéréoisomère, un tautomère, un énantiomère de celui-ci.

2. Conjugué oligomère d'alginate-polymyxine selon la revendication 1, dans lequel ledit antibiotique de classe polymyxine est
(i) sélectionné parmi les polymyxines A1, A2, B1, B1-I, B2, B3, B4, B5, B6, C, D1, D2, E1, E2, F, K1, K2, M, P1, P2, S et T et des dérivés fonctionnellement équivalents de celles-ci ;
(ii) représenté par la formule II 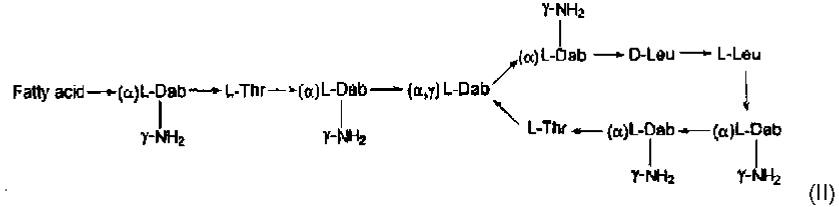 dans lequel D-Leu est la D-leucine ; L-Leu est la L-leucine ; L-Thr est la L-thréonine et L-Dab est l'acide α,γ-diaminobutyrique et dans lequel aucun, ou un ou plusieurs, de celles-ci n'est remplacé par un autre résidu d'acide aminé qui peut être sélectionné parmi des acides aminés naturels ou non génétiquement codés, de préférence dans lequel ledit acide aminé naturel ou non génétiquement codé est sélectionné parmi la leucine, la thréonine, un acide, la phénylalanine, l'arginine, l'histidine, la lysine, l'asparagine, la sérine, la cystéine, l'homolysine, l'ornithine, l'acide diaminobutyrique (par exemple, l'acide α,γ-diaminobutyrique), l'acide diaminopimélique, l'acide diaminopropionique, l'homoarginine, la triméthylysine, la triméthylornithine, l'acide 4-aminopipéridine-4-carboxylique, l'acide 4-amino-1-carbamimidoylpipéridine-4-carboxylique et la 4-guanidinophenylalanine ; et/ou
(iii) est sélectionné parmi la polymyxine B1, B1-I, B2, E1 ou E2.

3. Conjugué oligomère d'alginate-polymyxine selon la revendication 1 ou la revendication 2, dans lequel l'oligomère d'alginate présente un degré de polymérisation (DP) ou un degré moyen en nombre de polymérisation (DPn)
(i) de 4 à 75, 4 à 50, 4 à 35, 4 à 30, 4 à 25, 4 à 22, 4 à 20, 4 à 18, 4 à 16 ou 4 à 14,
(ii) de 6 à 50, 6 à 35, 6 à 30, 6 à 25, 6 à 22, 6 à 20, 6 à 18, 6 à 16 ou 6 à 14,
(iii) de 8 à 50, 8 à 35, 8 à 30, 8 à 25, 10 à 25, 10 à 22, 10 à 20, 10 à 18 ou 10 à 15, ou
(iv) de 20 à 75, 20 à 70, 20 à 65, 20 à 60, 20 à 55, 20 à 50, 20 à 45, 20 à 40, 20 à 35, 20 à 30 ou 20 à 25, ou
(v) de 30 à 75, 30 à 70, 30 à 65, 30 à 60, 30 à 55, 30 à 50, 30 à 45, 30 à 40 ou 30 à 35.

4. Conjugué oligomère d'alginate-polymyxine selon l'une quelconque des revendications 1 à 3, dans lequel l'oligomère d'alginate présente des résidus G d'au moins 70 % ou des résidus G d'au moins 80 % ou d'au moins 85 % ou d'au moins 90 % ou d'au moins 95 %, de préférence dans lequel au moins 80 % des résidus G sont agencés dans des blocs G.

5. Conjugué oligomère d'alginate-polymyxine selon l'une quelconque des revendications 1 à 3, dans lequel l'oligomère d'alginate présente des résidus M d'au moins 70 % ou d'au moins 80 % ou d'au moins 85 % ou d'au moins 90 % ou d'au moins 95 %, de préférence dans lequel au moins 80 % des résidus M sont agencés dans des blocs M.

6. Conjugué oligomère d'alginate-polymyxine selon l'une quelconque des revendications 1 à 5, dans lequel ladite liaison covalente directe fait partie d'un ester, d'un ester de carbonate, d'un orthoester, d'un cétal, d'un hémicétal, d'un éther, d'un acétal, d'un hémiactéal, d'un peroxy, d'un méthylènedioxy, d'un amide, d'une amine, d'une imine, d'un imide, d'un azide, d'un azo, d'une oxime, d'un sulfure, d'un disulfure, d'un sulfinyle, d'un sulfonyle, d'un carbonothioyl, d'un thioester, d'une phosphine ou d'un groupe fonctionnel de phosphodiester, de préférence dans lequel ladite liaison covalente directe fait partie d'un ester ou d'une amide.

7. Conjugué oligomère d'alginate-polymyxine selon l'une quelconque des revendications 1 à 5, dans lequel ledit lieur covalent est, ou comprend, des groupes moléculaires sélectionnés parmi :
(i) un acide aminé ou un peptide ;
(ii) un monosaccharide ou un oligosaccharide autre que le guluronate ou le mannuronate ou des polymères formés à partir de ceux-ci ;
(iii) un ribonucléotide ou un désoxyribonucléotide ;
(iv) un groupe alkyle, alcényle ou alcynyle, à chaîne linéaire, ramifié ou cyclique, substitué ou non substitué ;
(v) un acétyle, un succinyl, le (*cis ou trans*)-aconityle, le glutaryle, le méthylsuccinyle, la cystéamine de trimellityle, la pénicillamine, la N-(2-mercaptopropionyl) glycine, l'acide 2-mercaptopropionique, l'homocystéine, l'acide 3-mercaptopropionique ou le groupe déamino- pénicillamine.

8. Conjugué oligomère d'alginate-polymyxine selon l'une quelconque des revendications 1 à 7, dans lequel ladite liaison covalente directe, un groupe fonctionnel contenant ladite liaison covalente ou ledit lieur moléculaire covalent est
(i) un labile en milieu acide ;
(ii) sensible aux espèces réactives de l'oxygène ; et/ou
(iii) dégradé par une enzyme sécrétée par une bactérie ou une cellule immunitaire.

9. Conjugué oligomère d'alginate-polymyxine selon l'une quelconque des revendications 1 à 8, dans lequel ledit conjugué se compose
(i) d'au moins un oligomère d'alginate lié de manière covalente à un antibiotique de classe polymyxine par le biais d'une liaison ester formée à partir d'un groupe carboxyle sur l'alginate et d'un groupe hydroxyle sur la polymyxine ou
(ii) d'au moins un oligomère d'alginate lié de manière covalente à un antibiotique de classe polymyxine par le biais d'une liaison amide formée à partir d'un groupe carboxyle sur l'alginate et d'un groupe amine sur la polymyxine.

10. Conjugué oligomère d'alginate-polymyxine selon la revendication 9, dans lequel l'antibiotique de classe polymyxine est une colistine (par exemple, la polymyxine E1 ou E2) ou une polymyxine B (par exemple, la polymyxine B1, B1-I ou B2).

11. Composition pharmaceutique comprenant un conjugué oligomère d'alginate-polymyxine tel que défini dans l'une quelconque des revendications 1 à 10 et un excipient, un véhicule ou un diluant pharmaceutiquement acceptable.

12. Procédé pour la préparation d'un oligomère d'alginate-polymyxine tel que défini dans l'une quelconque des revendications 1 à 10, ledit procédé comprenant
(ia) la fourniture d'un oligomère d'alginate, dans lequel ledit oligomère d'alginate présente 2 à 75 résidus monomères et un antibiotique de classe polymyxine et la formation d'une liaison covalente directe entre deux groupes moléculaires sur celui-ci ; ou
(ib) la fourniture d'un oligomère d'alginate, dans lequel ledit oligomère d'alginate présente 2-75 résidus monomères, un antibiotique de classe polymyxine et un lieur moléculaire covalent et la formation d'une liaison covalente directe entre deux groupes moléculaires sur l'oligomère d'alginate et la molécule de liaison et la formation d'une liaison covalente directe entre deux groupes moléculaires sur l'antibiotique de classe polymyxine et la molécule de liaison ; ou
(ic) la fourniture d'un oligomère d'alginate, dans lequel ledit oligomère d'alginate présente 2-75 résidus monomères et un antibiotique de classe polymyxine, dans lequel un ou les deux comportent une molécule de liaison moléculaire covalente liée de manière covalente à ceux-ci et la liaison covalente de l'oligomère d'alginate à l'antibiotique de classe polymyxine par le biais d'au moins l'une des molécules de liaison ; et facultativement
(ii) la séparation d'au moins une partie du conjugué oligomère d'alginate-polymyxine du mélange de réaction.

13. Procédé selon la revendication 12, ledit procédé comprenant
(i) la fourniture d'une solution aqueuse d'un oligomère d'alginate ayant un groupe carboxyle disponible, dans lequel ledit oligomère d'alginate présente 2 à 75 résidus monomères ;
(ii) la mise en contact de ladite solution d'alginate avec l'hydrochlorure de 1-éthyl-3-[3-diméthylaminopropyl] carbodiimide (EDC) en une quantité et dans des conditions suffisantes pour activer au moins un groupe carboxyle dans l'oligomère d'alginate ;
(iii) facultativement la mise en contact dudit oligomère d'alginate activé par un carboxyle avec le sulfo N-hydroxysuccinimide (sulfo-NHS) en une quantité et dans des conditions suffisantes pour former un ester de sulfo-NHS réactif avec les aminés ;
(iv) la mise en contact dudit oligomère d'alginate activé par un carboxyle de l'étape (ii) ou de l'ester de sulfo-NHS réactif avec les amines de l'étape (iii) avec un antibiotique de classe polymyxine ayant un groupe amine primaire disponible en une quantité et dans des conditions suffisantes pour former une liaison amine entre l'oligomère d'alginate et l'antibiotique de classe polymyxine ; et
(v) la séparation d'au moins une partie du conjugué oligomère d'alginate-polymyxine du mélange de réaction.

14. Procédé selon la revendication 12, ledit procédé comprenant
(i) la fourniture d'une solution d'un oligomère d'alginate ayant un groupe carboxyle disponible, dans lequel ledit oligomère d'alginate présente 2 à 75 résidus monomères, de préférence une solution organique (par exemple, DMF et/ou DMSO) ;
(ii) la mise en contact de ladite solution d'alginate avec le dicyclohexylcarbodiimide (DCC) en une quantité et dans des conditions suffisantes pour former un intermédiaire d'O-acylisourée ;
(iii) la mise en contact dudit intermédiaire d'O-acylisourée avec un antibiotique de classe polymyxine ayant un groupe hydroxyle disponible et la 4-N, N-diméthylaminopyridine (DMAP) en une quantité et dans des conditions suffisantes pour former une liaison ester entre l'oligomère d'alginate et l'antibiotique de classe polymyxine ; et
(iv) la séparation d'au moins une partie du conjugué oligomère d'alginate-polymyxine du mélange de réaction ;
dans lequel les étapes (ii) et (iii) peuvent être réalisées simultanément.

15. Conjugué oligomère d'alginate-polymyxine tel que défini dans l'une quelconque des revendications 1 à 10 ou composition pharmaceutique telle que définie dans la revendication 11, destiné à être utilisés dans le traitement de la prévention d'une infection bactérienne, de préférence dans lequel l'infection bactérienne est
(i) une infection systémique ou une infection de multiples loci dans ou sur le sujet ;
(ii) une infection respiratoire chez un sujet souffrant d'un trouble ou d'un état respiratoire sous-jacent, de préférence sélectionné parmi CF, COPD/COAD ou l'asthme ;
(iii) une infection liée à un dispositif qui est associée à des dispositifs médicaux implantables ou prosthétiques ;
(iv) dans une blessure chronique ; ou
(v) une infection bactérienne à Gram négatif.
